# FASCICULE DE BREVET EUROPEEN

(11) **EP 1 520 044 B1**
(45) Date de publication et mention de la délivrance du brevet: **10.12.2008**
(21) Numéro de dépôt: 03755586.9
(22) Date de dépôt: 09.07.2003
(51) Int. Cl.: C12Q 1/68, A61K 8/00

(54) **GENES DU CHROMOSOME 9 IMPLIQUES DANS LA CANITIE PRECOCE**
GENE DES CHROMOSOMS 9 UND IHRE ASSOZIATION ZUR FRÜHZEITIGE HAARERGRAUUNG
CHROMOSOME 9 GENES INVOLVED IN PREMATURE CANITIES

(30) Priorité: 10.07.2002 FR 0208696; 08.04.2003 FR 0304360
(43) Date de publication de la demande: 06.04.2005
(73) Titulaire: L'ORÉAL, 75008 Paris (FR)
(72) Inventeur: DE LACHARRIERE, Olivier, F-75015 Paris (FR); BLOUIN, Jean-Louis, F-74100 Ville-La-Grand (FR); DELOCHE, Claire, F-75015 Paris (FR); ANTONARAKIS, Stylianos, CH-1205 Genève (CH)
(74) Mandataire: Sellin, Carole
(86) Numéro de dépôt international: PCT/FR2003/002154
(87) Numéro de publication internationale: WO 2004/007742

(56) Documents cités:
- WO-A-96/36691
- WO-A-02/097047
- WO-A-20/04007742
- FR-A- 2 842 104
- OETTING W S ET AL: "Molecular basis of albinism: mutations and polymorphisms of pigmentation genes associated with albinism." HUMAN MUTATION. UNITED STATES 1999, vol. 13, no. 2, 1999, pages 99-115, XP008018509 ISSN: 1059-7794
- BARSH G S: "The genetics of pigmentation: from fancy genes to complex traits" TRENDS IN GENETICS, ELSEVIER SCIENCE PUBLISHERS B.V. AMSTERDAM, NL, vol. 12, no. 8, 1 août 1996 (1996-08-01), pages 299-305, XP004037138 ISSN: 0168-9525
- HEMMINKI A ET AL: "Localization of a susceptibility locus for Peutz-Jeghers syndrome to 19p using comparative genomic hybridization and targeted linkage analysis" NATURE GENETICS, NEW YORK, NY, US, vol. 15, no. 1, 15 janvier 1997 (1997-01-15), pages 87-90, XP001105579 ISSN: 1061-4036
- TASSABEHJI M ET AL: "Waardenburg syndrome type 2 caused by mutations in the human microphthalmia (MITF) gene" NATURE GENETICS, NEW YORK, NY, US, vol. 8, no. 3, novembre 1994 (1994-11), pages 251-255, XP000909097 ISSN: 1061-4036

## Description

Annuler ou atténuer autant que faire se peut les effets du vieillissement est une préoccupation qui ne cesse de croître. Dans ce contexte, faire disparaître des cheveux blancs jugés disgracieux grâce à des shampoings traitants colorants est désormais une activité très répandue. Il est clair cependant que, même si cette technique permet effectivement d'annuler les effets du phénomène, elle n'en affecte nullement les causes. De ce fait, cette solution est temporaire et doit être fréquemment renouvelée.

Dans ce contexte, les inventeurs ont choisi d'explorer l'apparition des cheveux blancs, ou **canitie,** sous un angle totalement nouveau, celui de la génétique.

En effet, explorer la canitie de par son aspect génétique permet de mettre en évidence les mécanismes profonds de la dépigmentation. Cela permet aussi d'identifier les gènes qui sont impliqués dans la canitie. Cette identification ouvre la porte à de très nombreuses applications, dans le domaine du soin des cheveux, tant cosmétiques que thérapeutiques ou diagnostiques.

Il est hautement novateur de chercher à connaître les régions génomiques de la canitie par analyse de liaison génétique alors que d'autres études visent plutôt à décrypter la biochimie de la canitie.

Les inventeurs ont choisi de tirer partie de l'hypothèse proposée depuis bien longtemps du caractère héréditaire de la canitie précoce (CP), ou apparition des cheveux blancs **tôt** dans la vie. Le caractère familial du blanchiment précoce des cheveux chez certains est en effet aisément observable.

Le deuxième obstacle de la mise en oeuvre des méthodes de génétique inverse concerne la définition exacte du phénotype. Une parfaite définition du phénotype étudié est en effet nécessaire. Afin de garantir les meilleures chances de succès pour cette identification de gènes, le choix et la composition de l'échantillon utilisé dans la présente invention se sont déroulés selon un protocole rigoureux pour l'attribution du phénotype et la sélection des familles. Le phénotype « canitie précoce » fut attribué aux seuls individus qui présentaient des cheveux blancs avant 25 ans et dont la moitié de la chevelure était grise à 30 ans.

De plus, il est vraisemblable que d'une part la canitie précoce ait une origine multigénique et non monogénique, et que d'autre part, des facteurs environnementaux aient une influence sur le phénotype. Il s'agit de fait de définir un ensemble de causes prédisposant à la canitie précoce plutôt qu'une mutation unique responsable du phénotype. Dans ce contexte, la génétique inverse n'est pas, habituellement, une technique recommandée par les généticiens. Il est donc original de la part des inventeurs d'avoir utilisé cette méthode.

Les résultats de ces travaux ont permis aux inventeurs de définir des zones chromosiques et/ou génomiques comprenant des gènes impliqués avec une forte probabilité dans la canitie. Dans la présente divulgation, les régions des chromosomes ou les sous-parties de ces régions, identifiées par les inventeurs comme comprenant des gènes statistiquement impliqués dans la canitie, seront dénommées indifféremment "régions chromosomiques divulguées" ou "régions génomiques divulguées" ou "zones chromosomiques divulguées" ou "zones génomiques divulguées". Quant aux gènes identifiés dans le cadre de la présente divulgation au sein desdites régions, ils seront dénommés « gènes divulgués ».

L'objet de la présente divulgation concerne d'une part les gènes des régions chromosomiques identifiés et d'autre part l'utilisation de produits dérivés, tels que des produits de transcription ou de traduction, dans les domaines de la cosmétique, de la thérapeutique et du diagnostic.

Pour les domaines de la thérapie et de la cosmétique, la présente divulgation concerne successivement l'utilisation de polynucléotides dérivant d'un gène compris dans une région chromosomique de l'invention, l'utilisation d'agents capables de modifier la fonction attachée à ce gène, l'utilisation des produits d'expression du gène et l'utilisation d'agents capables de modifier la fonction de ces produits d'expression. L'utilisation conjointe ou combinée d'au moins deux des produits précédents peut s'avérer judicieuse, en particulier dans le domaine thérapeutique.

La présente divulgation concerne aussi un procédé pour le diagnostic d'une canitie précoce basé sur les variations alléliques au sein des gènes compris dans les régions chromosomiques de l'invention. En matière de diagnostic, il peut être, de plus, particulièrement pertinent de combiner les renseignements provenant de différents gènes des zones chromosomiques divulguées.

### GLOSSAIRE

Dans le cadre de la présente divulgation, les termes énoncés revêtent la signification suivante :
Par **fragment polynucléotidique,** on entend toute molécule résultant de l'enchaînement linéaire d'au moins deux nucléotides, cette molécule pouvant être monocaténaire, bicaténaire ou tricaténaire. Il peut donc s'agir d'une molécule d'ADN double-brin, d'un ADN simple brin, d'un ARN, d'un duplex ADN simple brin-ARN, d'un triplex ADN-ARN ou de toute autre combinaison. Le fragment polynucléotidique peut être naturel isolé, recombinant ou bien synthétique. Lorsque le fragment polynucléotidique comporte des brins complémentaires, la complémentarité n'est pas nécessairement parfaite, mais l'affinité entre les différents brins est suffisante pour permettre l'établissement de liaison stable de type Watson Crick entre les deux brins.

Bien que l'appariement des bases soient de préférence de type Watson-Crick, d'autres types ne sont pas exclus, tels qu'un appariement de type Hoogsteen ou Hoogsteen inverse.

On considère que la séquence S d'une molécule « **correspond** » à la séquence d'une molécule d'ADN donnée si l'on peut déduire l'enchaînement des bases de S de celui de la molécule d'ADN donnée par l'un des processus suivants
1. par identité, ou bien
2. par identité mais en changeant tout ou partie des Thymine en Uracile, ou bien
3. par complémentarité, ou bien
4. par complémentarité mais en changeant tout ou partie des Thymine en Uracile.

De plus, on considère que deux séquences restent « correspondantes » s'il est introduit globalement moins d'une erreur sur 10 dans l'un des processus précédents (complémentarité ou bien identité, avec ou sans échange T,U), de préférence moins d'une erreur sur 100. Par conséquent, les deux molécules ont aussi nécessairement des longueurs voisines, la variation maximale de longueur étant de 10% d'après le taux d'erreur accepté, elles ont de préférence une différence de longueur inférieure à 1%.

Cette définition ne suppose pas que les deux molécules soient de même nature, en particulier pour ce qui est de leur squelette, il s'agit d'une correspondance entre leur séquence uniquement.

Par exemple, deux séquences d'ADN identiques « correspondent » l'une à l'autre. De même, si ces deux séquences sont substantiellement identiques, c'est-à-dire identiques à plus de 90%, elles correspondent l'une à l'autre. Une séquence d'ARN, issue de la traduction d'une molécule d'ADN quelconque, « correspond » à la séquence de cette molécule d'ADN. De même, une séquence synthétique, par exemple hybride ADN-ARN, peut correspondre à une séquence d'ADN. Il en va de même entre une séquence d'ADN et l'ARN anti-sens ayant pour cible cette séquence.

Sur le même schéma, on considère que la séquence S d'une molécule d'ADN « **correspond** » à la séquence d'une molécule d'ADN donnée si l'on peut déduire la séquence S de celle de la molécule d'ADN donnée par le processus 1 ou 3 uniquement. La même latitude est autorisée concernant la possibilité d'introduction d'erreurs dans ces processus, c'èst-à-dire que l'on considère que deux séquences d'ADN restent « correspondantes » s'il est introduit globalement moins d'une erreur sur 10 dans les processus de complémentarité ou bien d'identité, de préférence moins d'une erreur sur 100.

Les **produits d'expression** d'un fragment d'ADN englobent toutes les molécules traduisant l'information génétique portée par ce fragment. Les ARN correspondant à la transcription du fragment d'ADN, à tous les stades de maturation, sont donc des produits d'expression ; il en est de même pour les polypeptides, à tous les stades de maturation, résultant de la traduction des ARN. Si des clivages interviennent au sein du polypeptide, comme par exemple le clivage de signaux d'adressage, tous les polypeptides résultants sont aussi considérés comme des produits d'expression du fragment d'ADN initial.

Dans le contexte de la divulgation, la « **fonction** » **primaire** d'un fragment d'ADN est de préférence d'être transcrite puis traduite en protéine. La fonction secondaire de l'ADN peut être assimilée à la fonction de la protéine résultant de la traduction de cet ADN. La fonction d'un fragment d'ADN revêt aussi d'autres significations dans la présente invention. En particulier, un fragment d'ADN peut appartenir à une région régulatrice d'un gène, sa fonction est alors, ou bien d'être le site de fixation d'enhancers ou d'inhibiteurs, ou bien d'être le site de fixation de l'ARN polymérase, ou bien d'être un site de reconnaissance pour le positionnement de l'ARN polymérase, ou bien toute autre fonction généralement assimilée à une séquence régulatrice.

D'autres fonctions sont envisageables pour les fragments d'ADN. En particulier, leur simple présence au sein d'un gène peut faciliter les recombinaisons. De même, une fonction selon la divulgation peut être celle des télomères et avoir trait à la dégénérescence. D'autres fonctions particulières attribuées à des fragments d'ADN sont bien connues par les biologistes.

Un **marqueur génétique** est une séquence d'ADN détectable. En génétique humaine, les marqueurs sont des séquences particulières de l'ADN pouvant prendre différentes formes selon les individus. Ce polymorphisme des marqueurs permet de suivre leur transmission dans le cadre des arbres généalogiques.

Parmi les **marqueurs** classiques, on peut identifier deux grandes catégories de marqueurs qui sont les marqueurs microsatellites et les SNPs (Single Nucleotide Polymorphism).

Un **microsatellite** est une séquence d'ADN répétée, constituée d'un motif relativement simple : un di, un tri ou un tétra nucléotide le plus souvent. Le nombre de répétitions change pour un même motif selon les individus et peut varier de quelques unités (une douzaine au minimum pour un dinucléotide), jusqu'à plus d'une centaine. Ces séquences sont dispersées un peu partout dans le génome, de façon presque aléatoire, mais à des endroits identiques d'un individu à un autre. Elles sont très abondantes (environ une tous les 10000 nucléotides = 10 kb) et elles sont très polymorphes. C'est la variation de longueur de la répétition en tandem qui constitue le marqueur. Ces séquences microsatellites sont donc très utilisées comme marqueurs génétiques.

Normalement, il n'y a pas de lien explicite entre un marqueur microsatellite et un gène, sinon une co-localisation. La longueur d'une répétition en tandem n'a pas, selon les connaissances actuelles, et en dehors de quelques rares cas de marqueurs intragéniques associés à certaines pathologies, de lien avec le rôle d'un gène. Dans le cadre de la présente invention, les marqueurs microsatellites sont des outils pour localiser les gènes impliqués dans la canitie précoce. Comme il existe beaucoup moins de polymorphisme dans les gènes que dans les marqueurs, un allèle génique sera représenté par plusieurs allèles d'un même marqueur microsatellite.

Il existe différentes méthodes pour définir la localisation de séquences particulières d'ADN le long des chromosomes. L'unité de mesure physique est le nombre de paires de bases. Toutefois, le **centimorgan** est souvent utilisé, c'est une unité de recombinaison donc une unité de mesure génétique et non physique. Deux séquences particulières d'un même chromosome sont distantes d'un centimorgan si elles recombinent une fois sur cent lors de la méiose. Un centimorgan équivaut approximativement à 10⁶ paires de bases.

Une autre méthode pour localiser des séquences particulières d'ADN le long des chromosomes consiste à définir leur position relativement à des marqueurs jalonnant les chromosomes et dont la position est parfaitement déterminée et connue. Des marqueurs très utilisés sont les marqueurs microsatellites dont il existe des cartographies très complètes. En particulier le GDB « Genome Database » est une banque de données, connue mondialement pour répertorier entre autres les STSs (Sequence tagged sites), bornes spécifiques et uniques de l'ADN dont font partie les microsatellites. Les codes DxSxxxx (par exemple D6S257), servant à identifier ces marqueurs, sont leur numéro d'accession dans GDB. Ces codes sont un moyen d'identification non ambigu et universel car seul GDB attribue ce type de code. Comme on peut trouver de tels marqueurs microsatellites environ tous les 10 kb, il est donc possible de définir la position de toute séquence à 10kb près, en indiquant les marqueurs microsatellites l'encadrant.

Un **SNP** (Single Nucleotide Pholymorphism) est un polymorphisme qui touche une unique base de l'ADN. C'est la forme de polymorphisme la plus répandue dans le génome humain, et elle se caractérise également par une grande stabilité lors de la transmission. La plupart de ces polymorphismes n'ont pas d'implications fonctionnelles. On compte environ 1 SNP sur 100 paires de bases. La connaissance de ces SNPs permet d'établir une cartographie du génome humain et les SNPs servent alors de véritables marqueurs du génome, d'autant plus qu'ils mutent lentement et ont peu de chance de réapparaître de façon récurrente.

Par région chromosomique **entre deux marqueurs**, on entend toute la séquence comprise entre ces deux marqueurs, les bornes étant comprises, donc la séquence des marqueurs y compris.

En **génétique inverse**, les indices permettant de localiser un gène proviennent de la comparaison de la transmission d'un phénotype, supposé induit par un gène muté ou par un allèle donné, avec la transmission de marqueurs connus, au sein d'une même famille. Ces données de co-ségrégation d'un phénotype et d'un marqueur permettent d'établir une analyse de liaison génétique.

La co-transmission d'un phénotype et d'un marqueur suggère que le gène responsable du phénotype et le marqueur sont physiquement proches l'un de l'autre sur le chromosome. La liaison est déterminée par l'analyse du schéma de transmission d'un gène et d'un marqueur dans des familles qui s'y prêtent.

L'**analyse de liaison** repose sur la co-transmission de certaines formes de marqueurs avec la forme défectueuse, ou modifiée d'un gène. Mais c'est une analyse indirecte dans le sens où d'une part, lors d'une première étape, un phénotype est associé à la forme défectueuse ou modifiée du gène. Une erreur dans l'assignation de certains phénotypes fausse l'étude. D'autre part, cette étude est basée sur des statistiques, ces statistiques reposant sur l'analyse d'un échantillon de la population, il s'agit donc d'un sondage. Enfin, il faut noter que lorsqu'il est possible d'associer un allèle particulier du marqueur avec un allèle du gène (en fait un phénotype), cette association n'est a priori valable que pour les échantillons inter-familiaux.

Le résultat des analyses de liaison dépend évidemment du degré de liaison entre le marqueur et le locus de la maladie. Cinq centimorgans (5 cM) est considéré comme un minimum de liaison pour un diagnostic. Une liaison à 5 cM signifie que l'on a 95% de chances d'arriver à une conclusion correcte et seulement 1 chance sur 20 qu'une recombinaison soit survenue entre le marqueur et le locus de la maladie:

Par le terme gène, dans le cadre de la présente invention, on entend non seulement la partie strictement codante, mais également les parties non codantes telles que les introns, et les parties régulatrices, en 5' et 3', les UTR (UnTranslated Region), notamment le ou les promoteurs, « enhancers » etc...associés.

Oetting et King (Human Mutation 13 (2) 99-115, 1999) décrivent six gènes liés à l'albinisme: TYR, OCA2, TYRP1, HPS, CHS1 et OA1 ainsi que des mutations de ces gènes.

Les inventeurs ont identifié deux régions chromosomiques distinctes, appartenant aux chromosomes 6 et 9 et qui sont impliquées dans la canitie précoce. Ces deux régions sont chacune des régions ou zones chromosomiques divulguées. Les inventeurs ont plus particulièrement mis en évidence l'implication de certains gènes appartenant à ces régions chromosomiques, appelés gènes divulgués.

Selon un **premier** aspect, la divulgation concerne les gènes HLAG, NT_007592.445, NT_007592.446, NT_007592.506, NT_007592.507, NT_007592.508, HSPA1B, G8, NEU1 (neuraminidase precursor), NG22, BAT8 (ankyrin repeat-containing protein), HLA-DMB, HLA-DMA, BRD2, HLA-DQA1, HLA-DQA2, NT_007592.588, GRM4 (glutamate receptor, metabotropic 4), RNF23, FLJ22638, NT_007592.459 et NT_007592.457 du chromosome 6 humain identifiés par les inventeurs comme étant impliqués dans la canitie précoce et les utilisations des produits dérivés de ces gènes, tels que des produits de transcription ou d'expression. Ces gènes appartiennent à la première zone chromosomique de l'invention qui est délimitée sur le chromosome 6 par les marqueurs microsatellites D6S1629 et D6S257. Cette zone sera plus particulièrement dénommée « première zone chromosomique divulguée ».

Des gènes préférés parmi les gènes précédemment cités au sein de la première zone chromosomique divulguée sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588. Des gènes tout particulièrement préférés sont les gènes NT_007592.506, NT_007592.507, NT_007592.508.

Selon un **second** aspect, la divulgation concerne les gènes FREQ (frequenin homolog), NT_030046.18, NT_030046.17, GTF3C5 (general transcription factor IIIC, polypeptide 5), CEL (carboxyl ester lipase bile salt-stimulated), CELL (carboxyl ester lipase-like bile salt-stimulated), FS (Forssman synthetase), ABO blood group (transferase A, alpha), BARHL1, DDX31, GTF3C4 et Q96MA6 du chromosome 9 humain identifiés par les inventeurs comme étant impliqués dans la canitie précoce et les utilisations des produits dérivés de ces gènes, tels que des produits de transcription ou d'expression. Ces gènes appartiennent à la seconde zone chromosomique divulguée qui est délimitée sur le chromosome 9 par le marqueur microsatellite D9S290 et la région télomérique (télomère du bras long). Cette zone sera plus particulièrement dénommée « deuxième ou seconde zone chromosomique divulguée ».

Des gènes préférés parmi les gènes précédemment cités au sein de la deuxième zone chromosomique divulguée sont les gènes BARHL1, DDX31, GTF3C4 et Q96MA6. Des gènes tout particulièrement préférés sont les gènes DDX31 et GTF3C4.

Pour les 2 zones chromosomiques identifiées ci-dessus, la présente divulgation couvre des **fragments polynucléotidiques** ayant une longueur minimale de 18 nucléotides, dont la séquence correspond au moins partiellement à l'un des gènes du chromosome 9 humain choisi parmi les gènes FREQ, NT_030046.18, NT_030046.17, GTF3C5, CEL, CELL, FS, ABO, BARHL1, DDX31, GTF3C4 et Q96MA6, ou à l'un des gènes du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. Ces fragments polynucléotidiques se caractérisent de plus fonctionnellement par leur implication dans la canitie, ou bien dans la canitie précoce, et éventuellement dans les deux phénomènes. En effet, les gènes impliqués dans la canitie précoce sont très vraisemblablement également impliqués dans la canitie due au vieillissement.

Selon une possibilité envisagée par la présente divulgation, un fragment impliqué dans la canitie ou la canitie précoce et possédant une séquence répondant aux exigences mentionnées plus haut peut être utilisé en thérapie.

Plus particulièrement, le **premier aspect** de la divulgation concerne les gènes du chromosome 6 humain identifiés par les inventeurs comme étant impliqués dans la canitie précoce. Selon cet aspect, un fragment tel que couvert par la divulgation a une séquence correspondant à tout ou partie d'un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. Ces gènes sont compris dans la première zone chromosomique divulguée délimitée sur le chromosome 6 par les marqueurs microsatellites D6S1629 et D6S257.

De préférence, un fragment selon la divulgation a une séquence correspondant à tout ou partie d'un gène choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588. De manière encore préférée, le gène est choisi parmi les gènes NT_007592.506, NT_007592.507 et NT_007592.508.

Le **second aspect** de la divulgation concerne les gènes du chromosome 9 humain identifiés par les inventeurs comme étant impliqués dans la canitie précoce. Selon cet aspect, un fragment tel que couvert par la divulgation a une séquence correspondant à tout ou partie d'un gène du chromosome 9 humain choisi parmi les gènes FREQ, NT_030046.18, NT_030046.17, GTF3C5, CEL, CELL, FS, ABO, BARLH1, DDX31, GTF3C4 et Q96MA6. Ces gènes sont compris dans la seconde zone chromosomique divulguée délimitée sur le chromosome 9 par le marqueur microsatellite D9S290 et la région télomérique (télomère du bras long).

De préférence, un fragment selon la divulgation a une séquence correspondant à tout ou partie d'un gène choisi parmi les gènes BARHL1, DDX31, GTF3C4 et Q96MA6. De manière encore préférée, le gène est choisi parmi les gènes DDX31 et GTF3C4.

Le fragment polynucléotidique dont il est fait référence dans le cadre de la divulgation correspond à un fragment d'un chromosome. Ce fragment a une longueur minimale de 18 nucléotides, et une longueur maximale qui peut aller jusqu'à la longueur totale du gène en question, ou de plusieurs gènes divulgués qui sont contigus au sein de la région chromosomique. De préférence, le fragment a un nombre de nucléotides supérieur à 18. Une longueur particulièrement préférée est comprise entre 18 et 10000 nucléotides, de préférence entre 30 et 8000 nucléotides.

Selon des variantes préférées, il est fait référence à des fragments dont la longueur est comprise entre 30 et 5000 nucléotides, de préférence entre 50 et 3000 nucléotides, par exemple entre 100 et 2000 nucléotides, ou entre 200 et 1000 nucléotides. La divulgation concerne également l'utilisation en cosmétique ou en thérapie d'un fragment polynucléotidique ou du produit d'expression d'un fragment ou d'un agent modulant la fonction d'un fragment, ou bien d'un agent modulant la fonction du produit d'expression d'un fragment, où le fragment en question correspond à tout ou partie d'un gène du chromosome 9 humain choisi parmi les gènes FREQ, NT_030046.18, NT_030046.17, GTF3C5, CEL, CELL, FS, ABO, BARLH1, DDX31, GTF3C4 et Q96MA6, ou à tout ou partie d'un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457, de l'une des deux zones chromosomiques de l'invention. Selon un cas préféré, le fragment correspond plus particulièrement à une partie d'un exon de l'un de ces gènes.

Dans ce qui suit, seront désignés «produits divulgués », le fragment, le produit d'expression d'un fragment, l'agent modulant la fonction d'un fragment, et l'agent modulant la fonction du produit d'expression d'un fragment polynucléotidique correspondant à tout ou partie de l'un des 22 gènes du chromosome 6 ou l'un des 12 gènes du chromosome 9 identifiés par les inventeurs.

Pour ces gènes identifiés par les inventeurs, la présente divulgation concerne tout d'abord des **utilisations dans le domaine de la cosmétique**. On entend par cosmétique toute application qui ne tend à modifier que l'esthétique et n'a aucune visée thérapeutique.

Pour toutes les utilisations selon la divulgation dans le domaine de la cosmétique, le produit divulgué peut être conditionné sous différentes formes appropriées, seul ou en combinaison avec d'autres agents. En particulier, des formes préférées sont destinées à des applications locales et elles concernent les crèmes, les lotions, les gels, les émulsions, les pommades et les shampoings. D'autres formes sont aussi envisageables pour des utilisations selon la divulgation, en particulier sous forme de pilules pour une administration orale.

Parmi les différentes visées cosmétiques dans le cadre de la présente divulgation, un domaine particulièrement préféré est celui de la pigmentation. La pigmentation peut être celle de la peau ou bien des phanères. Il peut s'agir de la couleur de la pigmentation comme de l'absence de pigmentation ; les problèmes touchant à la qualité et à l'intensité de la pigmentation sont aussi concernés par la présente divulgation.

En particulier, l'objet divulgué se rapporte à l'utilisation d'au moins un produit divulgué pour prévenir et/ou limiter et/ou arrêter le développement de la canitie.

L'objet divulgué se rapporte aussi à l'utilisation d'au moins un produit divulgué pour favoriser la pigmentation naturelle des cheveux et/ou des poils gris.

Un autre objet se rapporte à un procédé de traitement cosmétique de la canitie **caractérisé en ce qu**'on applique sur la zone à traiter une composition comprenant au moins un produit divulgué. La divulgation se rapporte aussi à un procédé de traitement cosmétique destiné à favoriser la pigmentation naturelle des cheveux et/ou des poils gris ou blancs caractérisé en ce qu'on applique sur la zone à traiter une composition comprenant au moins un produit de divulgué.

Les zones à traiter peuvent être, par exemple et sans aucune limitation, le cuir chevelu, les sourcils, la moustache et/ou la barbe.

Plus particulièrement, les procédés de traitement de la canitie et de pigmentation naturelle des cheveux et/ou poils gris ou blancs consistent à appliquer une composition comprenant au moins un produit divulgué.

Les procédés de traitement pour lutter contre la canitie et/ou pour stimuler la pigmentation naturelle des cheveux et/ou des poils gris ou blancs peut par exemple consister à appliquer la composition sur les cheveux et le cuir chevelu, le soir, garder la composition toute la nuit au contact et éventuellement effectuer un shampooing le matin ou laver les cheveux à l'aide de cette composition et à laisser à nouveau en contact quelques minutes avant de rincer. La composition conforme à la divulgation s'est révélée particulièrement intéressante lorsqu'elle est appliquée sous forme de lotion capillaire, éventuellement rincée ou même sous forme d'un shampooing.

Pour ces gènes identifiés par les inventeurs, la présente divulgation concerne ensuite des **utilisations thérapeutiques dans le domaine de la pigmentation.**

Les affections touchant le système de pigmentation, que ce soit celui de la peau ou celui des phanères, peuvent avoir de graves conséquences sur la santé des personnes atteintes. En effet, la pigmentation de la peau joue le rôle de barrière protectrice face aux agressions lumineuses en particulier, les personnes souffrant d'albinisme sont dépourvues de protection faces aux rayons du soleil qui constituent pour eux un danger important. D'autres affections mettant en jeu la pigmentation sont aussi concernées par la présente divulgation.

Dans le cadre des utilisations thérapeutiques et cosmétiques tendant à modifier une caractéristique de la pigmentation, il s'agit de préférence de la pigmentation de la peau. Selon d'autres cas envisagés par la présente divulgation, le type de pigmentation qui doit être modifiée concerne la pigmentation des phanères, en particulier les ongles ou les poils.

Selon un cas particulier préféré, la pigmentation dont on cherche à modifier les caractéristiques est celle du système pileux en général et de la chevelure, moustache et sourcils en particulier. La présente divulgation permet de modifier le phénomène d'arrêt de la pigmentation des cheveux, c'est-à-dire la canitie, en particulier lorsque celle-ci survient de manière prématurée chez une personne et que l'on parle de canitie précoce.

Pour toutes les utilisations en thérapeutique, les produits actifs entrant dans la composition d'un médicament sont de préférence associés à des excipients pharmaceutiquement acceptables. Toutes les voies d'administration considérées comme acceptables peuvent être utilisées dans le cadre de l'invention, en particulier voie intradermique, intraveineuse, musculaire, orale, otique, nasale, optique. La formulation est de préférence adaptée à la voie d'administration choisie.

Les utilisations pour la fabrication d'un médicament selon la divulgation peuvent faire entrer dans leur formulation d'autres principes actifs. De même, l'administration d'un médicament comme défini dans la divulgation peut être combinée avec l'administration d'un autre médicament, que cette administration soit simultanée, séquentielle ou séparée.

De même, les différents produits dont il est fait usage dans le cadre des utilisations en thérapie, peuvent être combinés et entrer dans la composition d'un unique médicament ou bien peuvent servir à la fabrication de différents médicaments. En particulier, s'ils entrent dans la composition de médicaments distincts, ils peuvent être administrés à des fréquences différentes.

Les caractéristiques et les variantes préférées des produits dont il est fait usage dans les **utilisations** selon la divulgation peuvent être identiques dans le cadre des utilisations en cosmétologie et pour des utilisations du même produit dans la fabrication d'un médicament.

Dans les deux cas, l'utilisation de produits selon la divulgation peut nécessiter que le produit soit introduit dans un fluide corporel, ou bien dans des tissus, ou dans des cellules. Pour l'introduction dans des cellules, l'utilisation peut prévoir que le produit soit actif dans le cytoplasme des cellules, ou bien dans le noyau cellulaire.

**La première utilisation,** cosmétique ou thérapeutique, envisagée dans le cadre de la divulgation est l'utilisation d'un fragment polynucléotidique dont la séquence correspond, au moins en partie, à l'un des gènes du chromosome 9 humain choisi parmi les gènes FREQ, NT_030046.18, NT_030046.17, GTF3C5, CEL, CELL, FS, ABO, BARHL1, DDX31, GTF3C4 et Q96MA6, ou à l'un des gènes du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. Pour les utilisations en thérapeutique, le fragment polynucléotidique entre dans la fabrication d'un médicament.

Dans le cadre de cette première utilisation selon la divulgation, les gènes préférés du chromosome 9 sont BARLH1, DDX31, GTF3C4 et Q96MA6, et tout particulièrement les gènes DDX31 et GTF3C4. Les gènes préférés du chromosome 6 sont HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Concernant la nature chimique de ce fragment polynucléotidique, il peut s'agir d'une molécule d'ADN, simple ou double brin, circulaire ou linéaire, d'une molécule d'ARN ou de toute autre molécule envisagée dans la définition de fragment polynucléotidique donnée plus haut.

Concernant son environnement, ce fragment peut être ou faire partie d'un plasmide, d'un génome viral ou d'un autre type de vecteur. Il peut, dans d'autres cas, faire partie du génome d'une cellule, ou bien d'une cellule génétiquement modifiée pour comporter ce fragment dans son génome. Il peut s'agir aussi d'une molécule isolée.

Concernant les régions encadrant ce fragment, il est de préférence sous le contrôle de séquences régulatrices. Si le fragment est inséré dans un vecteur, le dit vecteur comprend de préférence toutes les séquences nécessaires à la transcription et éventuellement la traduction du fragment. Ce fragment peut aussi être entouré par des régions flanquantes permettant une étape de recombinaison homologue avec un autre fragment polynucléotidique, conduisant éventuellement à l'insertion du fragment de l'invention dans l'ADN génomique d'une cellule cible.

Le fragment polynucléotidique tel que décrit peut se trouver sous forme naturelle ou bien être de nature synthétique, ou bien être en partie l'un et en partie l'autre, en particulier s'il s'agit d'une molécule « duplex » constitué de deux brins aux origines différentes. Selon différents cas envisagés, le fragment polynucléotidique peut être isolé, il peut avoir subi une étape de purification. Il peut s'agir aussi d'un fragment recombinant, par exemple synthétisé dans un autre organisme. Selon un exemple préféré, il s'agit d'un fragment d'ADN ayant été amplifié par PCR (Polymerisation Chain Reaction) puis purifié.

Selon d'autres constructions envisagées, la première utilisation fait usage d'un fragment polynucléotidique associé à une sonde. Cette caractéristique peut permettre, entre autres, de suivre la localisation du fragment, du milieu extracellulaire vers la cellule, ou du cytoplasme vers le noyau, ou bien de préciser son interaction avec l'ADN, ou l'ARN ou des protéines. La sonde peut aussi permettre de suivre la dégradation du fragment. La nature de la sonde est de préférence fluorescente, radioactive ou enzymatique. L'homme du métier saura quelle sonde est la mieux adaptée en fonction de la caractéristique qu'il veut pouvoir suivre.

Le fragment polynucléotidique, dont il est fait usage dans le cadre de cette première utilisation selon la divulgation, peut être utilisé dans un test d'hybridation, un séquençage, un microséquençage ou bien un test de détection de mésappariement.

Ce fragment selon la divulgation contient au moins 18 nucléotides successifs, ces 18 nucléotides constituant une séquence qui correspond à tout ou partie d'un des 22 gènes divulgués du chromosome 6 humain ou à tout ou partie d'un des 11 gènes divulgués du chromosome 9 humain, de préférence tout ou partie du gène DDX31 ou GTF3C4. En particulier, un fragment selon la divulgation peut ne contenir que 18 bases complémentaires de 18 bases successives de l'un des gènes précédemment décrits.

Selon un autre cas particulier, le fragment décrit peut être l'ADNc ou l'ARN de l'un des gènes précédemment décrits. Il peut correspondre à un ou plusieurs exons de l'un des gènes, il peut correspondre à une séquence de régulation de l'un des gènes identifiés sur les chromosomes 6 et 9.

Dans le cadre de cette première utilisation, le nombre de fragments polynucléotidiques tels que définis précédemment n'est pas limité et ne se restreint pas nécessairement à un seul.

En particulier, il peut être fait usage de plusieurs fragments polynucléotidiques dont la séquence correspond, au moins en partie, à tout ou partie d'un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. De préférence, les séquences des différents fragments correspondent à des gènes distincts ou à des exons distincts.

Alternativement, il peut être fait usage de plusieurs fragments polynucléotidiques dont la séquence correspond, au moins en partie, à un gène du chromosome 9 humain choisi parmi les gènes FREQ, NT_030046.18, NT_030046.17, GTF3C5, CEL, CELL, FS, ABO, BARLH1, DDX31, GTF3C4 et Q96MA6. De préférence, les séquences des différents fragments correspondent à des gènes distincts ou à des exons distincts.

Cette première utilisation selon la divulgation est de préférence dans le domaine de la cosmétique.

Cette utilisation peut aussi permettre la fabrication d'un médicament pour une action thérapeutique, dans le domaine de la pigmentation.

Selon un cas particulier envisagé, la première utilisation décrite implique une modification génétique, qu'elle soit induite par un fragment nucléotidique tel que décrit ou non.

Dans le cas où un gène responsable de la pigmentation est défectueux car muté, cette première utilisation selon la divulgation permet de restaurer la fonction de ce gène en introduisant un fragment polynucléotidique qui représente une nouvelle copie sauvage du gène endogène défectueux.

Dans le cas où l'activation d'un gène est responsable de la dépigmentation, cette première utilisation selon la divulgation permet d'abolir la fonction de ce gène en introduisant un ARN antisense qui va bloquer la traduction dudit gène.

**Une deuxième utilisation** envisagée par la présente divulgation, dans le domaine de la thérapie et dans celui de la cosmétique, est l'utilisation d'un agent modulant la fonction d'un fragment d'ADN correspondant au moins en partie à l'un des gènes identifiés dans le cadre de la présente l'invention. Pour les utilisations en thérapeutique, l'agent ainsi défini entre dans la fabrication d'un médicament. De préférence, le fragment d'ADN possède au moins 18 nucléotides.

Un tel agent selon la divulgation peut être capable de moduler la fonction d'un fragment d'ADN exogène dont une partie de la séquence correspond à l'un des glènes identifiés par les inventeurs, ou bien il peut être capable de moduler la fonction d'une séquence endogène comprise dans l'un de ces gènes identifiés par divulgation. De préférence, un agent servant pour cette deuxième utilisation selon la divulgation module non seulement la fonction d'un fragment d'ADN exogène tel que défini, mais aussi du fragment d'ADN endogène correspondant.

Le fragment d'ADN dont la fonction est modulée peut correspondre partiellement à l'un des gènes du chromosome 6 choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. Alternativement, le fragment d'ADN dont la fonction est modulée peut correspondre partiellement à l'un des gènes du chromosome 9 choisi parmi les gènes FREQ, NT_030046.18, NT_030046.17, GTF3C5, CEL, CELL, FS, ABO, BARHL1, DDX31, GTF3C4 et Q96MA6. En particulier, il peut s'agir d'un plasmide ayant uniquement une courte séquence correspondante à l'une des régions chromosomiques mentionnées. De préférence, la correspondance des séquences est établie sur au moins 18 nucléotides successifs.

Dans le cadre de cette deuxième utilisation selon la divulgation, les gènes préférés du chromosome 9 sont BARLH1, DDX31, GTF3C4 et Q96MA6, et tout particulièrement les gènes DDX31 et GTF3C4. Les gènes préférés du chromosome 6 sont HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Toutes les remarques qui précèdent, dans la partie définition, sur ce qu'il faut entendre par « fonction d'un fragment d'ADN » sont valables pour envisager toutes les utilisations correspondant à un deuxième utilisation selon la divulgation.

Etant donné la pluralité de fonctions des fragments d'ADN, la modulation de ces fonctions comprend des aspects très différents. Dans le cas particulier où la fonction dudit fragment est d'être transcrite, moduler une telle fonction consiste à favoriser ou inhiber la capacité dudit fragment à être transcrit. Cela peut aussi consister à modifier le site d'initiation et de terminaison de la transcription, ou bien à modifier le taux d'initiation de la transcription. Selon un autre cas; moduler la fonction peut aussi consister à modifier l'épissage de l'ARN, par exemple en modifiant des signaux de reconnaissance de l'ADN responsables la répartition entre introns et exons.

Quand le fragment d'ADN appartient à une séquence régulatrice, modifier sa fonction peut consister à inhiber la fixation des enhancers ou des inhibiteurs. Elle peut consister au contraire à favoriser leur fixation, ou bien à favoriser la fixation d'autres facteurs de transcription. Il en est de même lorsqu'il s'agit de séquences utilisées par l'ARN polymérase.

Dans le cadre de cette utilisation, comme dans le cadre de toutes les autres utilisations selon la divulgation, le nombre de produits, en l'occurrence d'agents modulant la fonction d'un fragment d'ADN correspondant au moins en partie à l'un des gènes identifiés dans le cadre de la présente divulgation, n'est pas limité et peut être supérieur à un.

Cependant, dans le cadre de cette deuxième utilisation, les différents agents dont il est fait usage peuvent avoir en commun de tous moduler la fonction de fragments d'ADN dont la séquence appartient ou correspond à au moins une partie d'un même gène de l'invention. Selon le premier aspect de la divulgation, ce gène est l'un des 22 gènes divulgués du chromosome 6 humain. Selon le deuxième aspect de la présente divulgation, le gène en question est l'un des 12 gènes divulgués du chromosome 9 humain, et de préférence DDX31 ou GTF3C4.

Des agents selon la divulgation sont par exemple des molécules d'ADN simple brin capable de se fixer à des sous-régions définies de l'un des gènes de l'invention, pour former des triples hélices. Dans ces conditions, des agents selon la divulgation abolissent la fonction de la sous-région à laquelle ils s'hybrident.

D'autres agents selon la divulgation sont de préférence des polypeptides capables d'interagir avec des sous-régions définies de l'un des gènes de l'invention. De préférence, des agents selon la divulgation sont des enhancers ou des inhibiteurs se fixant sur des régions régulatrices de l'un des 22 gènes de l'invention du chromosome 6 humain.

Alternativement, des agents selon la divulgation sont des enhancers ou des inhibiteurs se fixant sur des régions régulatrices de l'un des 12 gènes divulgués du chromosome 9 humain, de préférence DDX31 ou GTF3C4.

Une autre catégorie d'agents selon la divulgation concerne des molécules capables d'interagir avec des régions précises de l'ADN pour en changer sa conformation. Une autre catégorie concerne des molécules interagissant avec des inhibiteurs ou des enhancers pour en modifier leur fonction, les inhibiteurs ou enhancers ayant la fonction initiale de modifier l'expression de fragments d'ADN appartenant à l'un des gènes identifiés dans le cadre de la présente divulgation.

Un agent dont il est fait usage selon cette deuxième utilisation de la divulgation peut en particulier moduler la fonction d'un fragment d'ADN correspondant à 18 bases successives de l'un des gènes précédemment décrits.

Cette deuxième utilisation selon la divulgation est de préférence dans le domaine de la cosmétique. Cette utilisation peut aussi permettre la fabrication d'un médicament pour une action thérapeutique, dans le domaine de la pigmentation.

Selon un cas particulier envisagé, la seconde utilisation décrite implique une modification génétique, qu'elle soit induite par un agent modulant la fonction d'un fragment d'ADN tel que décrit ou non.

Dans le cas où l'activation d'un gène est responsable de la dépigmentation, cette seconde utilisation permet d'abolir la fonction de ce gène en introduisant un agent qui va bloquer la traduction dudit gène en se fixant, par exemple, à sa région promotrice.

Dans le cas où l'inactivation d'un gène est responsable de la dépigmentation, cette seconde utilisation permet de restaurer la fonction de ce gène en introduisant un agent qui va activer la transcription dudit gène, par exemple en se fixant à sa région promotrice, ou bien en se fixant à un éventuel inhibiteur qui cessera de ce fait d'inactiver ledit gène.

**Une troisième utilisation** envisagée par la présente divulgation, dans le domaine de la thérapie et dans celui de la cosmétique, est l'utilisation d'un agent modulant la fonction du produit d'expression d'un fragment d'ADN correspondant au moins en partie à l'un des gènes divulgués. Pour les utilisations en thérapeutique, l'agent ainsi défini entre dans la fabrication d'un médicament. De préférence, le fragment d'ADN possède au moins 18 nucléotides.

En particulier, un tel agent module la fonction d'un transcrit issu d'un fragment d'ADN correspondant au moins en partie à l'un des gènes du chromosome 6 choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. Selon un autre cas, un agent module la fonction d'un polypeptide issu de la traduction d'un des transcrits mentionnés. Alternativement, le fragment d'ADN dont la fonction du produit d'expression est modulée, peut correspondre au moins en partie à l'un des gènes du chromosome 9 choisi parmi les gènes FREQ, NT_030046.18, NT_030046.17, GTF3C5, CEL, CELL, FS, ABO, BARHL1, DDX31, GTF3C4 et Q96MA6.

Dans le cadre de cette troisième utilisation, les gènes préférés du chromosome 9 sont BARLH1, DDX31, GTF3C4 et Q96MA6, et tout particulièrement les gènes DDX31 et GTF3C4. Les gènes préférés du chromosome 6 sont HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Un tel agent peut être capable de moduler la fonction du produit d'expression d'un fragment d'ADN exogène dont une partie de la séquence correspond à l'un des gènes de l'invention identifiées par les inventeurs, ou bien il peut être capable de moduler la fonction du produit d'expression d'une séquence endogène comprise dans l'un des gènes de l'invention. De préférence, un agent servant pour cette troisième utilisation module non seulement la fonction d'un produit d'expression d'un fragment d'ADN exogène tel que défini, mais aussi du fragment d'ADN endogène correspondant.

Moduler la fonction d'un polypeptide peut être réalisé de différentes manières. En particulier, il est possible de croître ou décroître son activité, son rendement, sa spécificité, son avidité pour un anticorps, il est possible de modifier son substrat pour une enzyme, son taux de conversion.

Des agents selon la divulgation sont de préférence des molécules d'ARN, dites ARN antisense, qui s'hybrident avec au moins un transcrit issu d'un fragment d'ADN correspondant au moins en partie à l'un des gènes identifiés par les inventeurs sur le chromosome 6, ou bien à l'un des gènes identifiés par les inventeur sur le chromosome 9. D'autres agents remplissant les mêmes rôles peuvent être des molécules d'ADN simple brin, ou bien des molécules hybrides ADN-ARN. Le rôle de ces agents est de préférence de favoriser, empêcher, retarder, accélérer ou introduire des erreurs dans la traduction dudit transcrit.

D'autres agents préférés appartiennent à la classe des polypeptides. En particulier, la divulgation concerne des protéines capables de se fixer sur ledit transcrit et ainsi d'en moduler sa traduction. De tels agents de la classe des polypeptides peuvent être d'origine naturelle ou synthétique (synthétisés chimiquement ou par voie biotechnologique). Notamment il peut s'agir d'anticorps. Comme mentionné plus haut, cette modulation peut se traduire par l'action de favoriser, empêcher, retarder, accélérer ou introduire des erreurs dans la traduction dudit transcrit. En particulier, l'interaction entre le polypeptide et ledit transcrit peut constituer un obstacle à la fixation normale des ribosomes.

Des agents tels que définis dans la présente divulgation peuvent moduler la fonction de la protéine codée par un fragment d'ADN correspondant au moins en partie à l'un des 22 gènes de l'invention du chromosome 6 humain. Ces agents sont ou non de nature protéique. Un agent selon la divulgation peut intervenir à un stade précoce en empêchant le repliement correct de la protéine. Un agent selon la divulgation peut aussi modifier la fonction de ladite protéine en modifiant sa structure tridimentionnelle après le repliement. Il est aussi envisageable que cet agent soit un inhibiteur de la protéine, en particulier un inhibiteur compétitif.

Selon l'autre aspect de la divulgation, des agents tels que définis dans la présente divulgation peuvent moduler la fonction de la protéine codée par un fragment d'ADN correspondant au moins en partie à l'un des 12 gènes divulgués du chromosome 9 humain, de préférence DDX31 ou GTF3C4.

Les agents qui peuvent convenir dans le cadre de la présente divulgation ne sont pas limités à ceux cités précédemment.

Dans le cadre de cette troisième utilisation, le nombre d'agents modifiant la fonction d'un produit d'expression tels que définis précédemment n'est pas limité et ne se restreint pas nécessairement à un seul.

Cependant, dans le cadre de cette troisième utilisation, les différents agents dont il est fait usage peuvent avoir en commun de tous moduler la fonction de produits d'expression de fragments d'ADN dont la séquence appartient ou correspond à au moins une partie d'un même gène de l'invention. Selon le premier aspect de la divulgation, ce gène est choisi parmi les 22 gènes de l'invention du chromosome 6 humain. Selon le deuxième aspect de la présente divulgation, le gène en question est choisi parmi les 12 gènes de l'invention du chromosome 9, de préférence DDX31 ou GTF3C4.

Cette troisième utilisation est de préférence dans le domaine de la cosmétique. Cette utilisation peut aussi permettre la fabrication d'un médicament pour une action thérapeutique, dans le domaine de la pigmentation.

Selon un cas particulier envisagé, la troisième utilisation décrite implique une modification génétique, qu'elle soit induite par un agent modulant la fonction du produit d'expression d'un fragment d'ADN tel que décrit ou non.

Dans le cas où l'activation d'un gène est responsable de la dépigmentation, cette troisième utilisation permet d'abolir la fonction de ce gène en introduisant un ARN antisens qui va bloquer la traduction dudit gène en empêchant le passage ARN-protéine. Une autre situation préférée consiste à choisir pour agent un anticorps capable de se fixer sur la protéine résultant de la traduction dudit gène.

**Une quatrième utilisation** envisagée par la présente divulgation, dans le domaine de la thérapie et dans celui de la cosmétique, est l'utilisation d'un produit d'expression d'un fragment d'ADN correspondant au moins en partie à l'un des gènes identifiés dans le cadre de la présente invention. Pour les utilisations en thérapeutique, l'agent ainsi défini entre dans la fabrication d'un médicament. De préférence, le fragment d'ADN possède au moins 18 nucléotides.

En particulier, un tel produit d'expression est le transcrit ARN, quelque soit le stade de maturation dudit transcrit, issu d'un fragment d'ADN correspondant au moins en partie à l'un des gènes du chromosome 6 choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457, ou bien à l'un des gènes du chromosome 9 choisi parmi les gènes FREQ, NT_030046.18, NT_030046.17, GTF3C5, CEL, CELL, FS, ABO, BARHL1, DDX31, GTF3C4 et Q96MA6. En cas d'épissage, le transcrit peut donc être de taille inférieure au fragment d'ADN dont il est issu. De préférence, si le produit d'expression est une molécule d'ARN, elle comporte au moins 18 nucléotides.

Dans le cadre de cette quatrième utilisation, les gènes préférés du chromosome 9 sont BARLH1, DDX31, GTF3C4 et Q96MA6, et tout particulièrement les gènes DDX31 et GTF3C4. Les gènes préférés du chromosome 6 sont HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Selon un autre cas préféré, un produit d'expression est issu de la traduction d'un des transcrits mentionnés. Un tel produit d'expression peut donc comporter moins de 6 acides aminés si le transcrit dont il est issu a subi des étapes d'épissage. De préférence, un produit d'expression peptidique contient au moins 6 acides aminés.

Le produit d'expression ne provient pas nécessairement des étapes de transcription ou de traduction d'ADN génomique. En particulier, un produit d'expression utilisé selon la divulgation peut être un produit d'expression issu d'ADN exogène dont une partie de la séquence au moins correspond à une partie de l'un des gènes divulgués.

Est aussi envisagée par la présente divulgation l'utilisation d'un agent parfaitement synthétique mais semblable au produit d'expression d'un fragment d'ADN exogène ou endogène correspondant au moins en partie à l'un des gènes divulgués.

Des produits d'expression tels qu'utilisés par la présente divulgation sont de préférence des molécules d'ARN, dites ARN antisense, qui s'hybrident avec au moins un transcrit issu d'un fragment d'ADN correspondant au moins en partie à l'un des gènes identifiés sur le chromosome 6 ou bien à l'un des gènes identifiés sur le chromosome 9. En particulier, pour former des ARN anti-sens ayant pour cible un ARN spécifique, il est envisageable d'utiliser des ARN issus de la transcription de la même séquence d'ADN que la cible mais non pas du brin leader, mais de sa séquence complémentaire portée par l'autre brin. On obtient ainsi des fragments d'ARN complémentaires des fragments cibles normalement synthétisés par la cellule. Le rôle attendu de ces produits d'expression selon l'invention est de préférence de favoriser, empêcher, retarder, accélérer ou introduire des erreurs dans la traduction des transcrits normalement synthétisés par la cellule.

D'autres produits d'expression préférés appartiennent à la classe des polypeptides. En particulier, la divulgation concerne des protéines capables d'introduire un changement dans le fonctionnement de la cellule dans laquelle elles agissent.

Dans le cadre de cette utilisation, le nombre de produits d'expression d'un fragment d'ADN dont la séquence appartient ou correspond au moins en partie à l'un des gènes divulgués, n'est pas limité et peut être supérieur à un.

Cependant, dans le cadre de cette quatrième utilisation, les différents produits dont il est fait usage peuvent avoir en commun d'être tous des produits d'expression de fragments d'ADN dont la séquence appartient ou correspond à au moins une partie d'un même gène divulgué. Selon le premier aspect de la divulgation, ce gène est choisi parmi les 22 gènes divulgués du chromosome 6 humain. Selon le deuxième aspect, le gène en question est choisi parmi les 12 gènes divulgués du chromosome 9, de préférence DDX31 ou GTF3C4.

Cette quatrième utilisation est de préférence dans le domaine de la cosmétique. Cette utilisation peut aussi permettre la fabrication d'un médicament pour une action thérapeutique, dans le domaine de la pigmentation.

Selon un cas particulier envisagé, la quatrième utilisation décrite implique une modification génétique, qu'elle soit induite par un produit d'expression d'un fragment d'ADN tel que décrit ou non.

Dans le cas où l'activation d'un gène est responsable de la dépigmentation, cette quatrième utilisation permet d'abolir la fonction de ce gène en introduisant un ARN anti-sens qui va bloquer la traduction dudit gène en se fixant, au transcrit synthétisé par la cellule.

Dans le cas où l'inactivation d'un gène est responsable de la dépigmentation, cette quatrième utilisation permet de restaurer la fonction de ce gène en introduisant dans la cellule ou bien une molécule d'ARN permettant la synthèse de la protéine codée par le gène ou bien la protéine codée par le gène.

Pour les quatre types d'utilisations décrits précédemment, les utilisations en cosmétique sont de préférence dans le domaine de la pigmentation.

Pour les quatre types d'utilisation décrits précédemment, le produit divulgué pourra être incorporé dans une composition cosmétique ou pharmaceutique. Une telle composition comprend, dans un milieu pharmaceutiquement ou cosmétiquement acceptable, une quantité de produits comprise entre 0,001 et 10 % en poids par volume.

La composition peut être administrée par voie orale ou appliquée sur la peau (sur toute zone cutanée du corps) et/ou le cuir chevelu ou les cheveux.

Par voie orale, la composition peut contenir le ou les produits divulgués en solution dans un liquide alimentaire tel qu'une solution aqueuse ou hydroalcoolique, éventuellement aromatisée. Ils peuvent également être incorporés dans un excipient solide ingérable et se présenter par exemple sous forme de granulés, de pilules, de comprimés ou de dragées. Ils peuvent également être placés en solution dans un liquide alimentaire lui-même éventuellement conditionné dans des capsules ingérables.

Selon le mode d'administration, la composition peut se présenter sous toutes les formes galéniques normalement utilisées, particulièrement en cosmétologie.

Une composition préférée est une composition cosmétique adaptée à une application topique sur le cuir chevelu et/ou la peau.

Pour une application topique, la composition utilisable peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elles peuvent être anhydres ou aqueuses. Elle peut également consister en des préparations solides constituant des savons ou des pains de nettoyage.

Ces compositions sont préparées selon les méthodes usuelles.

La composition peut en particulier être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, de masque.

Lorsque la divulgation consiste en une utilisation à des fins cosmétiques, la composition sera préférentiellement une crème, une lotion capillaire, un shampoing ou un après-shampoing.

Les quantités des différents constituants des compositions utilisables sont celles classiquement utilisées dans les domaines considérés.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

Dans une variante, la composition sera telle que le ou les produits divulgués sont encapsulés dans un enrobage tel que des microsphères, des nanosphères, des oléosomes ou des nanocapsules, l'enrobage sera choisi selon la nature chimique du produit.

A titre d'exemple, les microsphères pourront être préparées selon la méthode décrites dans la demande de brevet EP 0 375 520.

Les nanosphères pourront se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

Les produits divulgués pourront aussi être encapsulés dans des nanocapsules consistant en un enrobage lamellaire obtenu à partir d'un tensio-actif siliconé (voir la demande de brevet EP 0780115), les nanocapsules pourront également être préparées à base de polyesters sulfonique hydrodispersibles (voir la demande de brevet FR 0113337).

Les produits divulgués pourront également être complexés à la surface de globules huileux cationiques, quelque soit leur taille (voir les demandes de brevet EP 1 010 413, EP 1 010 414, EP 1 010 415, EP 1010 416, EP 1 013 338, EP 1 016 453, EP 1 018 363, EP 1 020 219, EP 1 025 898, EP 1 120 101, EP 1 120 102, EP 1 129 684, EP 1 160 005 et EP 1 172 077).

Les produits divulgués peuvent enfin être complexés à la surface de nanocapsules ou nanoparticules pourvues d'un enrobage lamellaire (Voir EP 0 447 318 et EP 0 557 489) et contenant un tensio-actif cationique à la surface (voir les références citées précédemment pour les tensio-actifs cationiques).

En particulier, on préférera une composition telle que l'enrobage du ou des produits de l'invention a un diamètre inférieur ou égal à 10 µm.

De façon connue, la composition peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme huiles ou cires utilisables, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables, on peut citer les alcools inférieurs, notamment l'éthanol et l'isopropanol, le propylène glycol.

Comme gélifiants hydrophiles utilisables, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Les compositions peuvent associer au moins un produit divulgué à d'autres agents actifs. Parmi ces agents actifs, on peut citer à titre d'exemple :
- les agents modulant la différenciation et/ou la prolifération et/ou la pigmentation des cellules de la peau tels que le rétinol et ses esters, la vitamine D et ses dérivés, les oestrogènes tels que l'oestradiol, les modulateurs de l'AMPc tels que les dérivés de POMC, l'adénosine, ou la forskoline et ses dérivés, les prostaglandines et leurs dérivés, la triiodotrionine et ses dérivés ;
- des extraits de végétaux tels que ceux d'Iridacées ou de soja, extraits pouvant alors contenir ou non des isoflavones ;
- des extraits de micro-organismes ;
- les agents anti-radicaux libres tels que l'α-tocophérol ou ses esters, les superoxyde dismutases ou ses mimétiques, certains chélatants de métaux ou l'acide ascorbique et ses esters ;
- les anti-séborrhéiques tels que certains acides aminés soufrés, l'acide 13-cis rétinoïque, l'acétate de cyprotérone ;
- les autres agents de lutte contre les états desquamatifs du cuir chevelu comme le zinc pyrithione, le disulfure de sélénium, le climbazole, l'acide undécylénique, le Kétoconazole, la piroctone olamine (octopirox) ou la ciclopiroctone (ciclopirox) ;
en particulier, il pourra s'agir d'actifs stimulant la repousse et/ou favorisant le ralentissement de la chute des cheveux, on peut plus particulièrement citer à titre non limitatif :
- les esters d'acide nicotinique, dont notamment le nicotinate de tocophérol, le nicotinate de benzyle et les nicotinates d'alkyles en C1-C6 comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4,139,619 et US 4,596,812 ;
- les agents inhibiteurs de lipoxygenase ou inducteur de cyclo-oxydase favorisant la repousse des cheveux comme ceux décrits par la Demanderesse dans la demande de brevet européen EP 0 648 488 ;
- les agents antibactériens tels que les macrolides, les pyranosides et les tétracyclines, et notamment l'Erythromycine ;
- les agents antagonistes de calcium, comme la Cinnarizine, la Nimodipine et la Nifedipine ;
- des hormones, telles que l'estriol ou des analogues, ou la thyroxine et ses sels ;
- des agents antiandrogènes, tels que l'oxendolone, la spironolactone et la flutamide;
- des inhibiteurs stéroïdiens ou non stéroïdiens des 5-α-réductases tels que ceux décrits par la Déinanderesse dans les demandes de brevet européen EP 0 964 852 et EP 1 068 858 ou encore le finastéride ;
- des agonistes des canaux potassiques dépendant de l'ATP tels que la cromakalim et le nicorandil.

Dans une autre possibilité de mise en oeuvre, la présente divulgation concerne des procédés pour le diagnostic d'une prédisposition à la canitie précoce chez un individu.

En effet, la canitie précoce est un phénotype qui a été défini par les inventeurs comme étant, entre autres, caractérisé par l'apparition des premiers cheveux blancs tôt dans la vie, et de préférence vers l'âge de 18 ans. Comme ce phénotype est transmis à la génération suivante, il peut s'avérer important, pour les individus dont un parent ou un proche est atteint, de déterminer, avant l'apparition des symptômes, s'ils seront ou non sujets à cette atteinte. Le procédé de diagnostic divulgué est parfaitement adapté aux individus âgés de moins de 18 ans.

Comme il est très probable que des facteurs environnementaux jouent un rôle dans le phénotype « canitie » comme dans celui « canitie précoce », il s'agit, grâce aux procédés divulgués, d'évaluer les risques de développer un tel phénotype, c'est-à-dire une prédisposition à la canitie précoce.

Un procédé divulgué pour la détermination d'une prédisposition à la canitie précoce comprend une première étape de sélection d'un ou de plusieurs marqueurs dont il va être fait usage dans les étapes suivantes. Par marqueur, on entend une séquence d'ADN dont les différentes variations alléliques sont porteuses d'informations. Un tel marqueur peut être une courte séquence d'un gène dont la mutation est source du phénotype. Il peut s'agir aussi d'un marqueur situé physiquement sur le chromosome dans une région très proche d'un gène impliqué dans la canitie précoce. De préférence, le marqueur est un SNP (Single nucleotide polymorphism).

Selon un premier aspect d'un procédé divulgué, le ou les marqueurs sélectionnés appartiennent à la région du chromosome 6 humain comprenant les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457. De préférence, les marqueurs sont choisis comme appartenant à la séquence de l'un de ces gènes. Des gènes préférés sont les gènes HLAG, NT_007592.445, NT_007592.446, NT_007592.506, NT_007592.507, NT_007592.5.08, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Selon un deuxième aspect d'un procédé divulgué, le ou les marqueurs sélectionnés appartiennent à la région du chromosome 9 humain comprenant les gènes FREQ, NT_030046.18, NT_030046.17, GTF3C5, CEL, CELL, FS, ABO, BARLH1, DDX31, GTF3C4 et Q96MA6. De préférence, les marqueurs sont choisis comme appartenant à la séquence de l'un de ces gènes. Des gènes préférés sont les gènes BARHL1, DDX31, GTF3C4 et Q96MA6 et tout particulièrement les gènes DDX31 et GTF3C4.

L'étape suivante de la mise en oeuvre d'un procédé divulgué consiste, pour le ou les marqueurs choisis, à déterminer les allèles présents dans un échantillon de matériel génétique issu de l'individu qui subit le test diagnostique. Deux allèles différents, portés par les deux versions du chromosome, peuvent être identifiés.

L'échantillon contenant le matériel génétique peut être le sang, une unique goutte étant suffisante pour la mise en oeuvre d'un procédé divulgué. D'autres échantillons de fluides corporels peuvent être utilisés. Il est aussi envisageable d'utiliser quelques cellules issues de l'individu. L'homme de l'art saura déterminer quel échantillon pourra être utilisé dans le cadre de ce test, tout en minimisant le désagrément pour l'individu le subissant. Ce test diagnostic pourra éventuellement être couplé avec d'autres tests génétiques.

Les techniques courantes, bien connues du biologiste moléculaire, peuvent être utilisées pour effectuer la détermination des allèles du ou des marqueurs choisis ; des tests d'hybridation sont en particulier très courants dans ce genre d'étapes.

Divers marqueurs sont potentiellement préférés dans le cadre de la mise en oeuvre du procédé divulgué. En particulier, des marqueurs bi-alléliques peuvent s'avérer particulièrement adéquats si une forme allélique traduit une prédisposition à la canitie précoce alors que l'autre forme allélique reflète au contraire l'absence d'une telle prédisposition. D'autres marqueurs plus courants sont polymorphiques et peuvent être trouvés sous au moins deux formes alléliques et généralement plus de deux.

Parmi les marqueurs qui peuvent être sélectionnés à la première étape du procédé , des marqueurs particulièrement bien connus sont les SNPs. Lors de la sélection du marqueur, il est très important de se baser sur la valeur informative du polymorphisme du marqueur. Une situation particulièrement privilégiée consiste à sélectionner un marqueur dont certains variants alléliques traduisent une prédisposition à la canitie précoce alors que tous les autres variants reflètent au contraire l'absence d'une telle prédisposition. Dans de nombreuses situations, le marqueur ne remplit pas entièrement une telle condition, c'est-à-dire que par exemple certains allèles sont présents préférentiellement mais non exclusivement chez les individus prédisposés à la canitie. Dans ces situations, il peut être très judicieux de sélectionner plusieurs marqueurs, afin d'établir un test diagnostic aussi fiable que possible.

Lorsque les marqueurs sélectionnés sont des SNP, les différents variants alléliques correspondent à la modification d'une base. Une situation particulièrement avantageuse à rechercher lors de la sélection du marqueur, correspond à la situation où certains allèles (modification d'une base) sont caractéristiques d'une prédisposition à la canitie précoce.

Selon le premier aspect d'un procédé divulgué, le ou les marqueurs sélectionnés à la première étape peuvent être choisis parmi les marqueurs SNP suivants : 2734988, 2734967, 1419664, 2517502, rs733539, rs494620, 154973, 206779, 60071, rs763028.

Selon le deuxième aspect d'un procédé divulgué, le ou les marqueurs sélectionnés à la première étape peuvent être choisis parmi les marqueurs SNP suivants : 418620, rs302919, 913705, 932886, 429269 et rs2526008.

Les procédés divulgués ne sont pas limités aux deux étapes décrites et peuvent contenir d'autres étapes antérieures ou postérieures aux deux étapes mentionnées.

En particulier, un procédé divulgué peut comporter l'étape supplémentaire de comparaison de la forme allélique du ou des marqueurs sélectionnés à la forme allélique de ce ou de ces mêmes marqueurs chez d'autres individus. Cette étape de comparaison supplémentaire peut s'avérer nécessaire afin d'établir le diagnostic. Dans ce cas, il peut être utile de faire la comparaison avec la forme du ou des marqueurs chez des individus notoirement atteints de canitie précoce et éventuellement aussi avec la forme du ou des marqueurs chez des individus notoirement exempts d'une telle prédisposition.

Etant donné que la canitie précoce est une atteinte vraisemblablement multigénique, les causes de prédisposition sont nombreuses et peuvent difficilement être toutes envisagées de manière exhaustive. Par contre, au sein d'une même famille dont certains membres ont été précocement atteints de canitie, il est très probable que la cause de la susceptibilité est unique. De ce fait, lors de l'étape de comparaison, mentionnée comme éventuelle troisième étape des procédés de l'invention, une comparaison particulièrement riche en information est la comparaison des allèles du marqueur de l'individu subissant le test aux allèles du même marqueur pour les personnes de sa famille dont le phénotype est connu. Si plusieurs marqueurs ont été sélectionnés, il faut de préférence répéter cette opération pour tous les marqueurs.

La présente divulgation concerne aussi des procédés de criblage de molécules ayant un effet particulier. En particulier, la divulgation concerne un procédé d'identification de molécules capables de moduler la fonction d'un fragment polynucléotidique.

Selon le premier aspect, le fragment polynucléotidique dont on cherche à moduler la fonction comprend au moins 18 nucléotides consécutifs dont la séquence correspond à tout ou partie d'un gène choisi parmi les 22 gènes divulgués du chromosome 6 humain. Des gènes préférés sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Selon le deuxième aspect, le fragment polynucléotidique dont on cherche à moduler la fonction comprend au moins 18 nucléotides consécutifs dont la séquence correspond à tout ou partie d'un gène choisi parmi les 12 gènes divulgués du chromosome 9. Des gènes préférés sont les gènes BARHL1, DDX31, GTF3C4 et Q96MA6, et tout particulièrement les gènes DDX31 et GTF3C4.

Le procédé d'identification de molécules capables de moduler la fonction de l'un ou l'autre de ces fragments comprend une étape de mise en présence de la molécule à tester et du fragment polynucléotidique. Une autre étape du procédé est la détection d'une variation d'un paramètre lié à la fonction dudit fragment, par exemple la détection d'une éventuelle fixation de cette molécule sur le fragment polynucléotidique mise en évidence par une technique de détection de ligands.

La « deuxième utilisation selon la divulgation» est l'utilisation d'un agent modulant la fonction d'un fragment d'ADN correspondant au moins en partie à un des gènes divulgués.

Le procédé de criblage permet d'identifier de tels agents.

Les différentes fonctions que peuvent revêtir un fragment polynucléotidique ont déjà été explicitées. En particulier, ces fonctions dépendent de la nature du fragment polynucléotidique, selon s'il s'agit d'ADN ou d'ARN par exemple. Une modulation de la fonction peut correspondre à une diminution de la capacité à être transcrit, ou traduit, ou bien à un changement dans la capacité à interagir avec d'autres facteurs. En fonction des propriétés dudit fragment utilisé, l'homme de l'art est capable de déterminer quel est le paramètre dont la variation est facile à monitorer.

Le procédé d'identification de la divulgation n'est pas limité aux étapes précédemment décrites, d'autres étapes antérieures ou postérieures peuvent être appliquées.

La présente divulgation couvre aussi les molécules identifiées par le procédé précédemment décrit. En particulier, la présente divulgation couvre les inhibiteurs des fonctions des fragments polynucléotidiques divulgués.

La présente divulgation concerne aussi des procédés de criblage de molécules capables de moduler la fonction du produit d'expression d'un fragment polynucléotidique divulgué.

Selon le premier aspect, le produit d'expression dont on cherche à moduler la fonction est celui d'un fragment d'ADN appartenant et/ou correspondant à tout ou partie d'un gène choisi parmi les 22 gènes divulgués du chromosome 6 humain. Des gènes préférés sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Selon le deuxième aspect, le produit d'expression dont on cherche à moduler la fonction est celui d'un fragment d'ADN appartenant et/ou correspondant à tout ou partie d'un gène choisi parmi les 12 gènes divulgués du chromosome 9. Des gènes préférés sont les gènes BARHL1, DDX31, GTF3C4 et Q96MA6, et tout particulièrement les gènes DDX31 et GTF3C4.

De préférence, le fragment d'ADN comprend au moins 18 nucléotides.

Le procédé d'identification de molécules capables de moduler la fonction du produit d'expression d'un fragment d'ADN tel que décrit comprend une étape de mise en présence de la molécule à tester et du produit d'expression. Une autre étape du procédé est la détection d'une variation d'un paramètre lié à la fonction dudit produit d'expression, par exemple la détection d'une éventuelle fixation de cette molécule sur le produit d'expression mise en évidence par une technique de détection de ligands.

Comme mentionné précédemment, on entend par produit d'expression d'un fragment d'ADN aussi bien les molécules d'ARN issues de la transcription du fragment, à tout stade de maturation, que les polypeptides issus de la traduction, aussi à tout stade de maturation. Pour une molécule d'ARN, différents stades de maturation sont représentés par la présence ou l'absence de coiffe, de queue polyadénylée, par exemple. Par différents stades de maturation d'un polypeptide, on inclut entre autres les polypeptides avant et après le repliement, avant et après clivage des différents signaux d'adressage, avec et sans glycosylation, avec et sans ponts disulfure.

Quant aux fonctions remplies par les produits d'expression des fragments d'ADN, elles sont très nombreuses et elles dépendent de la nature du produit d'expression en question. Des exemples ont déjà été donnés plus haut dans la présente demande.

La « troisième utilisation selon la divulgation » est l'utilisation d'un agent modulant la fonction du produit d'expression d'un fragment d'ADN. Le procédé de criblage permet d'identifier de tels agents. Pour ce qui doit être compris par la locution « moduler la fonction du produit d'expression d'un fragment d'ADN », des exemples ont déjà été donnés pour définir la troisième utilisation selon la divulgation.

En fonction des propriétés dudit produit d'expression utilisé, l'homme de l'art est capable de déterminer quel est le paramètre dont la variation est facile à monitorer.

Le procédé d'identification n'est pas limité aux étapes précédemment décrites, d'autres étapes antérieures ou postérieures peuvent être appliquées.

La présente divulgation couvre aussi les molécules identifiées par le procédé précédemment décrit. En particulier, la présente divulgation couvre les inhibiteurs des fonctions des produits d'expression des fragments polynucléotidiques de l'invention.

La présente divulgation permet aussi de mettre en évidence les mutations des gènes impliqués dans la pigmentation de la peau, des cheveux et des phanères, pour les gènes divulgués.

Une utilisation particulière envisagée par la présente divulgation consiste donc à utiliser les marqueurs SNPs décrits précédemment au sein de chacune des régions comprenant les gènes d'intérêt dans le but de localiser plus finement les mutations de ces gènes impliquées dans la pigmentation, et plus particulièrement ceux impliqués dans l'interruption progressive ou subite de la pigmentation de la peau ou des phanères.

Dans le cadre de ces utilisations, selon le premier aspect, les marqueurs dont il est fait usage pour la détermination des gènes d'intérêt sont choisis parmi les marqueurs SNP : 2734988, 2734967, 1419664, 2517502, rs733539, rs494620, 154973, 206779, 60071, rs763028.

Selon le deuxième aspect, les marqueurs dont il est fait usage pour la détermination des gènes d'intérêt sont choisis parmi les marqueurs SNP 418620, rs302919, 913705, 932886, 429269 et rs2526008.

La présente divulgation repose sur l'identification par les inventeurs de gènes, sur les chromosomes 6 et 9 humains, impliqués dans le phénomène de pigmentation ou de dépigmentation. Cette base génétique leur a permis d'envisager les utilisations en thérapie et en cosmétique décrites précédemment, ainsi que les procédés de diagnostic illustrés précédemment.

Mais comme déjà mentionné, les inventeurs soupçonnent que de nombreux gènes sont impliqués dans les phénomènes de pigmentation, et dans ceux liés à la régulation et la cessation de cette pigmentation. Notamment, il est envisagé que les gènes divulgués sur le chromosome 9 sont principalement responsables de la canitie, alors que les gènes divulgués sur le chromosome 6 sont concernés par la précocité de la canitie. De ce fait, un part importante de la présente divulgation consiste à combiner les résultats obtenus pour les gènes divulgués, pour en tirer le plus d'informations complémentaires.

En particulier, une **première utilisation de combinaison** dans le domaine de la cosmétique et de la thérapie, fait de préférence usage d'au moins deux fragments polynucléotidiques dont la séquence de chacun correspond, au moins en partie, à celle d'un gène choisi parmi les 22 gènes divulgués du chromosome 6 humain ou bien à celle d'un gène choisi parmi les 12 gènes divulgués du chromosome 9.

Des gènes préférés sur le chromosome 6 sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARHL1, DDX31, GTF3C4 et Q96MA6, et tout particulièrement les gènes DDX31 et GTF3C4.

De préférence, parmi les différents fragments polynucléotidiques dont il est fait usage, au moins deux ont des séquences correspondant à deux gènes distincts. Il est aussi envisageable que pour un même gène, les différents fragments dont il est fait usage aient des natures chimiques différentes, par exemple de l'ADN pour le premier fragment et de l'ARN pour le second. Il est aussi envisageable que différents fragments aient une séquence correspondant au même gène, mais avec les faibles variations permises par la définition de « séquences correspondances », c'est-à-dire au plus un nucléotide différent sur 10, de préférence 1 sur 100.

Les fragments contiennent au moins 18 nucléotides successifs, ces 18 nucléotides formant la séquence qui doit correspondre au moins partiellement à l'un des gènes divulgués.

Toutes les utilisations préférées, la nature chimique des fragments, leur environnement, ont déjà été explicités en détails dans la partie décrivant la première utilisation.

Lorsqu'il est fait usage d'au moins deux fragments polynucléotidiques, ces deux fragments sont de préférence portés par des molécules distinctes. Il est aussi envisageable que ces deux fragments fassent partie par exemple d'un même vecteur. Selon un cas préféré, les différents fragments sont de même nature chimique, par exemple de l'ADN pour tous les fragments.

Pour les utilisations en thérapeutique, les fragments polynucléotidiques entrent dans la fabrication d'un médicament.

Une **deuxième utilisation de combinaison** envisagée, dans le domaine de la thérapie et dans celui de la cosmétique, est l'utilisation d'une combinaison d'au moins deux agents modulant chacun la fonction d'un fragment d'ADN choisi parmi les fragments appartenant et/ou correspondant à tout ou partie d'un des 34 gènes de l'invention sur les chromosome 6 et 9 humains.

Des gènes préférés sur le chromosome 6 sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARHL1, DDX31, GTF3C4 et Q96MA6, et tout particulièrement les gènes DDX31 et GTF3C4.

De préférence, parmi les différents agents dont il est fait usage, au moins deux modulent la fonction de fragments d'ADN correspondant à deux gènes distincts. Il est aussi envisageable que pour un même gène, les différents agents dont il est fait usage modulent des fonctions différentes d'un même fragment d'ADN.

Les fragments d'ADN dont la fonction est modulée contiennent de préférence au moins 18 nucléotides successifs, ces 18 nucléotides formant la séquence qui doit correspondre au moins partiellement à l'un des gènes identifiés dans le cadre de la présente divulgation.

Toutes les utilisations préférées de tels agents ont déjà été explicitées en détails dans la partie décrivant la deuxième utilisation.

Pour les utilisations en thérapeutique, les agents entrent dans la fabrication d'un médicament.

Une **troisième utilisation de combinaison** envisagée, dans le domaine de la thérapie et dans celui de la cosmétique, est l'utilisation d'une combinaison d'au moins deux agents modulant chacun la fonction du produit d'expression d'un fragment d'ADN choisi parmi les fragments appartenant et/ou correspondant à tout ou partie d'un des 34 gènes divulgués sur les chromosomes 6 et 9 humains.

Des gènes préférés sur le chromosome 6 sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARBA1, DDX31, GTF3C4 et Q96MA6, et tout particulièrement les gènes DDX31 et GTF3C4.

De préférence, parmi les différents agents dont il est fait usage, au moins deux modulent la fonction du produit d'expression de fragments d'ADN correspondant à deux gènes distincts. Il est aussi envisageable que pour un même gène, les différents agents dont il est fait usage modulent des fonctions différentes d'un même produit d'expression d'un fragment d'ADN ou bien modulent différents produits d'expression d'un fragment d'ADN, par exemple les ARN à différents stades de maturation, ou bien les ARN issus de différents épissages.

Les fragments d'ADN dont.la fonction du produit d'expression est modulée contiennent de préférence au moins 18 nucléotides successifs, ces 18 nucléotides formant la séquence qui doit correspondre au moins partiellement à l'un des gènes divulgués.

Toutes les utilisations préférées de tels agents ont déjà été explicitées en détails dans la partie décrivant la troisième utilisation.

Pour les utilisations en thérapeutique, les agents entrent dans la fabrication d'un médicament.

Une **quatrième utilisation de combinaison** envisagée, dans le domaine de la thérapie et dans celui de la cosmétique, est l'utilisation d'une combinaison d'au moins deux produits d'expression de fragments d'ADN choisis parmi les fragments appartenant et/ou correspondant à tout ou partie d'un des 34 gènes divulgués sur les chromosomes 6 et 9 humains.

Des gènes préférés sur le chromosome 6 sont les gènes HLAG, NT_007592.445 NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARHL1, DDX31, GTF3C4 et Q96MA6, et tout particulièrement les gènes DDX31 et GTF3C4.

De préférence, parmi les différents produits d'expression dont il est fait usage, au moins deux sont issus de fragments d'ADN correspondant à deux gènes distincts. Il est aussi envisageable que pour un même gène, les différents produits d'expression dont il est fait usage soient issus d'un même fragment d'ADN, il peut s'agir par exemple des ARN à différents stages de maturation, ou bien des ARN issus de différents épissages. Les mêmes possibilités sont offertes pour les polypeptides issus de la traduction des ARN.

Les fragments d'ADN dont les produits d'expression sont utilisés contiennent de préférence au moins 18 nucléotides successifs, ces 18 nucléotides formant la séquence qui doit correspondre au moins partiellement à l'un des gènes divulgués.

Toutes les utilisation préférées de tels produits d'expression ont déjà été explicitées en détails dans la partie décrivant la quatrième utilisation.

Pour les utilisations en thérapeutique, les produits d'expression entrent dans la fabrication d'un médicament.

Pour les quatre types d'utilisations de combinaison décrits précédemment, les utilisations en cosmétique sont de préférence dans le domaine de la pigmentation.

Parmi les nombreuses combinaisons envisagées, une combinaison très intéressante comprend au moins un fragment polynucléotidique correspondant à tout ou partie d'un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457 pour la première utilisation de combinaison.

Pour la deuxième utilisation de combinaison, parmi les agents dont il est fait usage, de préférence un au moins de ces agents module la fonction d'un fragment d'ADN correspondant ou bien appartenant à un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457.

Pour la troisième utilisation de combinaison, parmi les agents dont il est fait usage, de préférence un au moins de ces agents module la fonction du produit d'expression d'un fragment d'ADN correspondant ou bien appartenant à un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457.

Pour la quatrième utilisation de combinaison, parmi les produits d'expression dont il est fait usage, de préférence, un au moins de ces produits d'expression est issu d'un fragment d'ADN correspondant ou bien appartenant à un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457.

Dans toutes les utilisations de combinaison précédemment décrites, en cosmétique ou en thérapie, il est fait usage de plusieurs fragments ou de plusieurs produits d'expression de fragment ou de plusieurs agents modulant la fonction de fragments, ou bien de plusieurs agents modulant la fonction du produit d'expression de fragments, où le fragment en question correspond à tout ou partie de l'un des gènes divulgués. De préférence, l'un au moins des fragments dont il est question correspond à un gène du chromosome 6 humain choisi parmi les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2, NT_007592.588, GRM4, RNF23, FLJ22638, NT_007592.459 et NT_007592.457.

De préférence, l'un au moins des fragments dont il est question dans les utilisations de combinaison correspond à un gène du chromosome 9 humain choisi parmi les gènes FREQ, NT_030046.18, NT_030046.17, GTF3C5, CEL, CELL, FS, ABO, BARLH1, DDX31, GTF3C4 et Q96MA6.

Pour les quatre types d'utilisation de combinaison décrits précédemment, les combinaisons divulguées pourront être incorporées dans une composition cosmétique ou pharmaceutique telle que décrite plus haut.

Afin de tirer profit de la combinaison de ces gènes sur les deux régions chromosomiques, la présente divulgation concerne aussi des procédés de combinaison. Ces procédés sont mis en oeuvre pour la détermination d'une éventuelle prédisposition à la canitie précoce.

Un procédé de combinaison pour la détermination d'une prédisposition à la canitie précoce comprend une première étape de sélection d'au moins deux marqueurs dont il va être fait usage dans les étapes suivantes. Les marqueurs sélectionnés sont choisis parmi les marqueurs appartenant aux régions des chromosomes 6 et 9 humains comprenant les 34 gènes de l'invention. De préférence, les marqueurs sélectionnés appartiennent à la séquence de l'un des gènes mentionnés sur les chromosomes 6 et 9.

Des gènes préférés sur le chromosome 6 sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARLH1, DDX31, GTF3C4 et Q96MA6, et tout particulièrement les gènes DDX31 et GTF3C4.

De préférence, parmi les marqueurs sélectionnés, deux au moins n'appartiennent pas au même gène. Selon un cas particulier, les marqueurs, au nombre de deux au minimum, sont choisis parmi la liste suivante de marqueurs SNP : 2734988, 2734967, 1419664, 2517502, rs733539, rs494620, 154973, 206779, 60071, rs763028, 418620, rs302919, 913705, 932886, 429269 et rs2526008.

L'étape suivante de la mise en oeuvre d'un procédé selon la divulgation consiste, pour les marqueurs choisis, à déterminer les allèles présents dans un échantillon de matériel génétique issu de l'individu qui subit le test diagnostique. Deux allèles différents, portés par les deux versions du chromosome, peuvent être identifiés.

Les modalités de mise en oeuvre de ces procédés ont déjà été explicitées dans la partie consacrée aux procédés de diagnostic.

Les procédés de combinaison ne sont pas limités à l'utilisation de deux marqueurs, ils ne sont pas limités non plus aux deux étapes décrites et peuvent contenir d'autres étapes antérieures ou postérieures aux deux étapes mentionnées.

En particulier, un procédé de combinaison peut comporter l'étape supplémentaire de comparaison de la forme allélique des marqueurs sélectionnés à la forme allélique de ces mêmes marqueurs chez d'autres individus. Cette étape de comparaison supplémentaire peut s'avérer nécessaire afin d'établir le diagnostic. Dans ce cas, il peut être utile de faire la comparaison avec la forme des marqueurs chez des individus notoirement atteints de canitie précoce et éventuellement aussi avec la forme des marqueurs chez des individus notoirement exempts d'une telle prédisposition.

Comme pour les procédés de diagnostic déjà mentionnés, une situation particulièrement intéressante consiste à comparer les allèles des marqueurs sélectionnés aux allèles de ces mêmes marqueurs chez d'autres individus de la même famille que l'individu à diagnostiquer.

De plus, dans une autre demande de brevet, déposée le même jour par le même demandeur, les inventeurs ont aussi mis en évidence, par une méthode similaire, d'autres régions génomiques, sur les chromosomes 3, 5 et 11 humains, impliqués aussi dans le phénomène de pigmentation ou de dépigmentation (voir également les exemples 1 et 3 de la présente demande). Ces régions sont délimitées sur le chromosome 3 par les marqueurs microsatellites D3S1277 et D3S1285, sur le chromosome 5 par les marqueurs microsatellites D5S2115 et D5S422 et sur le chromosome 11 par les marqueurs microsatellites D11S898 et D11S925. Plus particulièrement, les inventeurs ont identifié les gènes de ces régions statistiquement impliqués dans la canitie précoce, chez certains sujets, il s'agit des gènes KIAA1042, CCK, CACNA1D, ARHGEF3 et AL133097 du chromosome3, des gènes KLHL3, HNRPA0, CDC25C, EGR1, C5orf6, C5orf7, LOC51308, ETF1, HSPA9B, PCDHA1 à PCDHA13, CSF1R, RPL7, PDGFRB, TCOF1, AL133039, CD74, RPS14, NDST1, G3BP, GLRA1, C5orf3, MFAP3, GALNT10 et FLJ117151 du chromosome 5 et des gènes GUCY1A2, CUL5, ACAT1, NPAT, ATM, AF035326, AF035327, AF035328, BC029536, FLJ20535, DRD2, ENS303941, IGSF4, LOC51092, BC010946, TAGLN, PCSK7 et ENS300650 du chromosome 11. Cette base génétique a permis aux inventeurs d'envisager des utilisations en thérapie et en cosmétique similaires à celles décrites précédemment, ainsi que des procédés de diagnostic similaires à ceux illustrés précédemment.

Dans le cadre de la présente divulgation, il est est donc avantageux de tirer profit de ces résultats en combinant des produits élaborés en tenant compte des séquences impliquées dans la canitité sur les chromosomes 6 et 9, et des produits élaborés en tenant compte des séquences impliquées dans la canitie sur les chromosomes 3, 5 et 11.

Une combinaison particulièrement préférée dans le cadre de la présenté divulgation est la combinaison de produits élaborés en tenant compte des séquences d'intérêt sur le chromosome 9 et de produits élaborés en tenant compte de séquences d'intérêt sur les chormosomes 6,11, 5 et 3.

En particulier, une **première utilisation** dans le domaine de la cosmétique et de la thérapie, fait de préférence usage d'au moins deux fragments polynucléotidiques dont la séquence du premier correspond, au moins en partie, à celle du gène DDX31 ou GTF3C4 du chromosome 9 et la séquence d'au moins un autre correspond, au moins en partie à l'un des gènes suivants: KIAA1042, CCK, CACNA1D, ARHGEF3 et AL133097 du chromosome 3, KLHL3, HNRPA0, CDC25C, EGR1, C5orf6, C5orf7, LOC51308, ETF1, HSPA9B, PCDHA1 à PCDHA13, CSF1R, RPL7, PDGFRB, TCOF1, AL133039, CD74, RPS14, NDST1, G3BP, GLRA1, C5orf3, MFAP3, GALNT10 et FLJ117151 du chromosome 5 et GUCY1A2, CUL5, ACAT1, NPAT, ATM, AF035326, AF035327, AF035328, BC029536, FLJ20535, DRD2, ENS303941, IGSF4, LOC51092, BC010946, TAGLN, PCSK7 et ENS300650 du chromosome 11.

Une **deuxième utilisation** envisagée, dans le domaine de la thérapie et dans celui de la cosmétique, est l'utilisation d'une combinaison d'au moins deux agents modulant chacun la fonction d'un fragment d'ADN, le premier fragment appartenant et/ou correspondant à tout ou partie du gène DDX31 ou GTF3C4 du chromosome 9, au moins un autre fragment appartenant et/ou correspondant à tout ou partie à l'un des gènes suivants : KIAA1042, CCK, CACNA1D, ARHGEF3 et AL133097 du chromosome 3, KLHL3, HNRPA0, CDC25C, EGR1, C5orf6, C5orf7, LOC51308, ETF1, HSPA9B, PCDHA1 à PCDHA13, CSF1R, RPL7, PDGFRB, TCOF1, AL133039, CD74, RPS14, NDST1, G3BP, GLRA1, C5orf3, MFAP3, GALNT10 et FLJ117151 du chromosome 5 et GUCY1A2, CUL5, ACAT1, NPAT, ATM, AF035326, AF035327, AF035328, BC029536, FLJ20535, DRD2, ENS303941, IGSF4, LOC51092, BC010946, TAGLN, PCSK7 et ENS300650 du chromosome 11.

Une **troisième utilisation** envisagée, dans le domaine de la thérapie et dans celui de la cosmétique, est l'utilisation d'une combinaison d'au moins deux agents modulant chacun la fonction du produit d'expression d'un fragment d'ADN, le premier fragment appartenant et/ou correspondant à tout ou partie du gène DDX31 ou GTF3C4 du chromosome 9, au moins un autre fragment appartenant et/ou correspondant à tout ou partie à l'un des gènes suivants : KIAA1042, CCK, CACNA1D, ARHGEF3 et AL133097 du chromosome 3, KLHL3, HNRPA0, CDC25C, EGR1, C5orf6, C5orf7, LOC51308, ETF1, HSPA9B, PCDHA1 à PCDHA13, CSF1R, RPL7, PDGFRB, TCOF1, AL133039, CD74, RPS14, NDST1, G3BP, GLRA1, C5orf3, MFAP3, GALNT10 et FLJ117151 du chromosome 5 et GUCY1A2, CUL5, ACAT1, NPAT, ATM, AF035326, AF035327, AF035328, BC029536, FLJ20535, DRD2, ENS303941, IGSF4, LOC51092, BC010946, TAGLN, PCSK7 et ENS300650 du chromosome 11.

Une **quatrième utilisation** envisagée, dans le domaine de la thérapie et dans celui de la cosmétique, est l'utilisation d'une combinaison d'au moins deux produits d'expression de fragments d'ADN, le premier fragment appartenant et/ou correspondant à tout ou partie du gène DDX31 ou GTF3C4 du chromosome 9, au moins un autre fragment appartenant et/ou correspondant à tout ou partie à l'un des gènes suivants : KIAA1042, CCK, CACNA1D, ARHGEF3 et AL133097 du chromosome 3, KLHL3, HNRPA0, CDC25C, EGR1, C5orf6, C5orf7, LOC51308, ETF1, HSPA9B, PCDHA1 à PCDHA13, CSF1R, RPL7, PDGFRB, TCOF1, AL133039, CD74, RPS14, NDST1, G3BP, GLRA1, C5orf3, MFAP3, GALNT10 et FLJ117151 du chromosome 5 et GUCY1A2, CUL5, ACAT1, NPAT, ATM, AF035326, AF035327, AF035328, BC029536, FLJ20535, DRD2, ENS303941, IGSF4, LOC51092, BC010946, TAGLN, PCSK7 et ENS300650 du chromosome 11.

Enfin, la présente divulgation concerne un kit comprenant une combinaison d'au moins deux fragments polynucléotidiques choisis parmi ceux comprenant au moins 18 nucléotides consécutifs dont la séquence correspond à tout ou partie d'un des 34 gènes de l'invention sur les chromosomes 6 et 9 humains.

Des gènes préférés sur le chromosome 6 sont les gènes HLAG, NT_007592.445, NT_007592.446, NT_007592.506, NT_007592.507, NT_007592.508, HSPA1B, G8, NEU1, NG22, BAT8, HLA-DMB, HLA-DMA, BRD2, HLA-DQA1, HLA-DQA2 et NT_007592.588, et tout particulièrement les gènes NT_007592.506, NT_007592.507 et NT_007592.508.

Des gènes préférés sur le chromosome 9 sont les gènes BARLH1, DDX31, GTF3C4 et Q96MA6, et tout particulièrement les gènes DDX31 et GTF3C4.

Les fragments ont de préférence une longueur comprise entre 15 et 5000 nucléotides, de préférence entre 30 et 5000, de préférence entre 40 et 3000 nucléotides.

### Légende des figures

- **Figure 1 :**: Composition des familles analysées pour liaison avec la région-candidate
- **Figure 2 :**: Région candidate pour la CP sur le chromosome 6 (figure 2A) et sur le chromosome 3 (figure 2B): Localisation chromosomique et distribution des marqueurs.
- **Figure 3 :**: Représentation graphique des scores NPL obtenus pour l'analyse de liaison non-paramétrique multipoints génome global sur les familles CP pour les chromosomes 6, 9 (figure 3A), 3, 5 (figure 3B) et 11 (figure 3C).
Abscisse = position sur la carte génétique (0 = pter)
Ordonnée = score NPL
- **Figure 4 :**: Représentation schématique des loci CP identifiés sur les chromosomes 6 et 9 par l'étude génome global. La distance entre marqueurs est indiquée en cM.
**Figure 4A** **:** Chromosome 6, locus6p21-p12
**Figure 4B** **:** Chromosome 9, locus 9q34
**Figure 4C** **:** Chromosome 11, locus 11q14-q22
**Figure 4D** **:** Chromosome 5, locus 5q31-q32
**Figure 4E** **:** Chromosome 3, locus 3p14.1-p12.3
- **Figure 5 :**: Lod scores simulés pour les 29 familles sélectionnées.
Dans l'ordre, les colonnes affichent le Lod score moyen, la déviation standard, le Lod score minimum, le Lod score maximum et le groupe (A-E) dans lequel la famille se place selon son score.

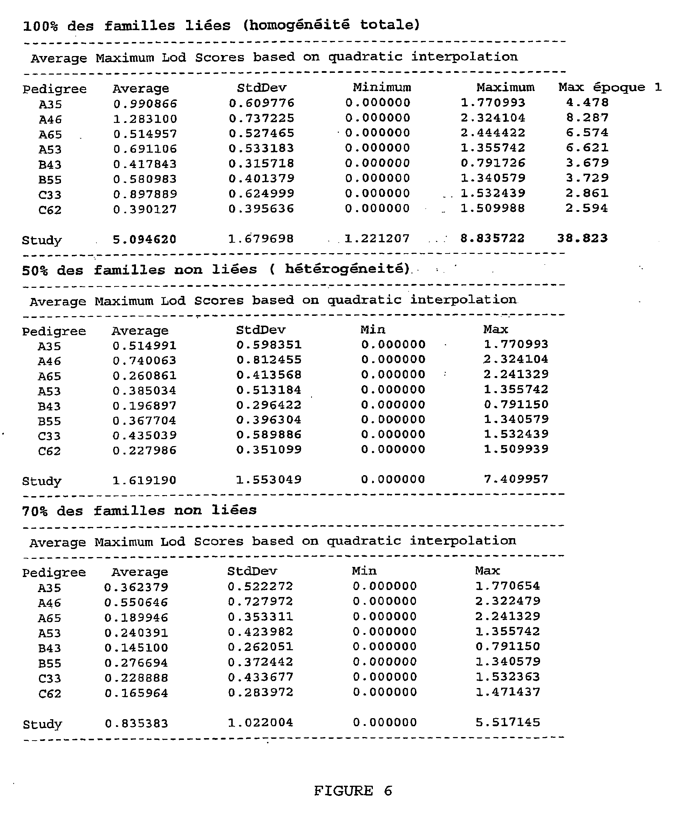

- **Figure 6 :**: Lod scores potentiels par famille en fonction du taux d'hétérogénéité génétique de la CP.

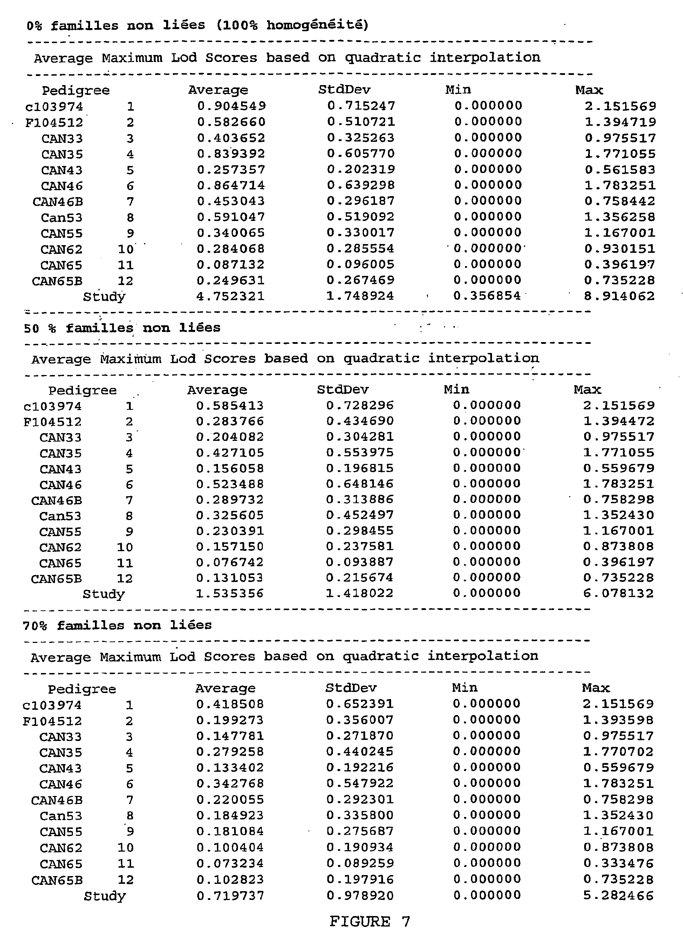

- **Figure 7 :**: Lod scores détaillé par famille en fonction du taux d'hétérogénéité génétique de la CP : nouvelle simulation après collection des nouvelles familles.
- **Figure 8 :**: Nouvelle simulation sur les familles finalisées pour la recherche des régions chromosomiques candidates. Lod scores potentiels en fonction du taux d'hétérogénéité.
Les résultats sont exprimés par famille.

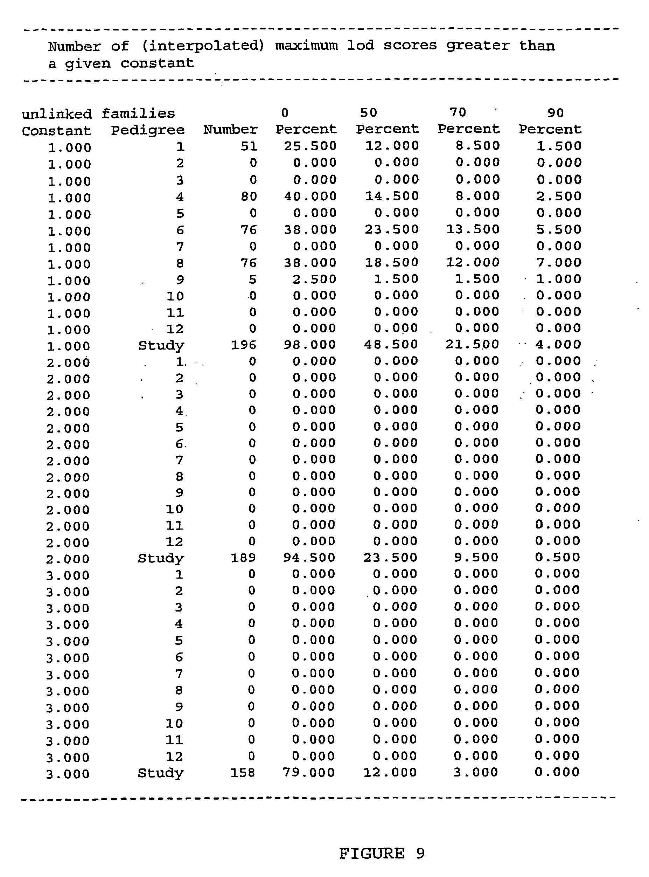

- **Figure 9 :**: Probabilité (en %) d'atteindre ou de dépasser un Lod score de 1, 2 ou 3 pour chaque taux d'hétérogénéité.
Les résultats sont exprimés par famille.
- **Figure 10 :**: Comparaison de la composition des familles entre l'analyse région-candidate et l'analyse Génome-global.
- **Figure 11 :**: Lod scores potentiels en fonction du taux d'hétérogénéité de la CP.
Les résultats sont exprimés par famille.
- **Figure 12 :**: Probabilité (en %) d'atteindre ou de dépasser un Lod score de 1,2 ou 3 pour chaque taux d'hétérogénéité.
Les résultats sont exprimés par famille.
- **Figure 13 :**: Schéma récapitulatif des différentes étapes de l'analyse des régions A et B à l'aide de la technologie basée sur les SNPs.
- **Figure 14 :**: Composition des 4 pools. Les pools AI et AII sont composés d'individus atteints de canitie précoce. Les deux pools contrôles BI et BII sont composés d'individus « croisés » pour origine et âge avec les individus atteints de canitie précoce.
- **Figure 15 :**: Graphe indiquant la significativité des 288 SNPs testés sur les pools pour la régions A. L'abscisse représente les SNPs, numérotés de 1 à 288 le long de la région A (du télomère p vers le télomère q), chaque SNP étant séparé de ses voisins par une région de 30kb en moyenne. L'ordonnée représente 1/p, p étant la significativité statistique. Cependant, les valeurs 1/p supérieure à 500 (c'est-à-dire p<0,02) ont été maximalisées à 500.
- **Figure 16 :**: Graphe indiquant la significativité des 171 SNPs testés sur les pools pour la régions B. L'abscisse représente les SNPs, numérotés de 1 à 171 le long de la région B (du télomère p vers le télomère q), chaque SNP étant séparé de ses voisins par une région de 30kb en moyenne. L'ordonnée représente 1/p, p étant la significativité statistique. Cependant, les valeurs 1/p supérieure à 500 (c'est-à-dire p<0,02) ont été maximalisées à 500.
- **Figure 17 :**: Tableau répertoriant les 43 SNP retenus pour le génotypage individuel. La première colonne indique leur numéro (numéro attribué à l'étape précédente de 1 à 288 le long de la région A, du télomère p vers le télomère q). La seconde colonne indique le nom du SNP. Les colonnes suivantes indiquent les valeurs des différentes comparaisons A-B (AI-BI ; AII-BII; AI-BII) avec la valeur p associée. La mention « M » signifie que la valeur de la significativité « p » est inférieure à 0,05. La dernière colonne précise le gène éventuellement chevauché par ledit SNP.

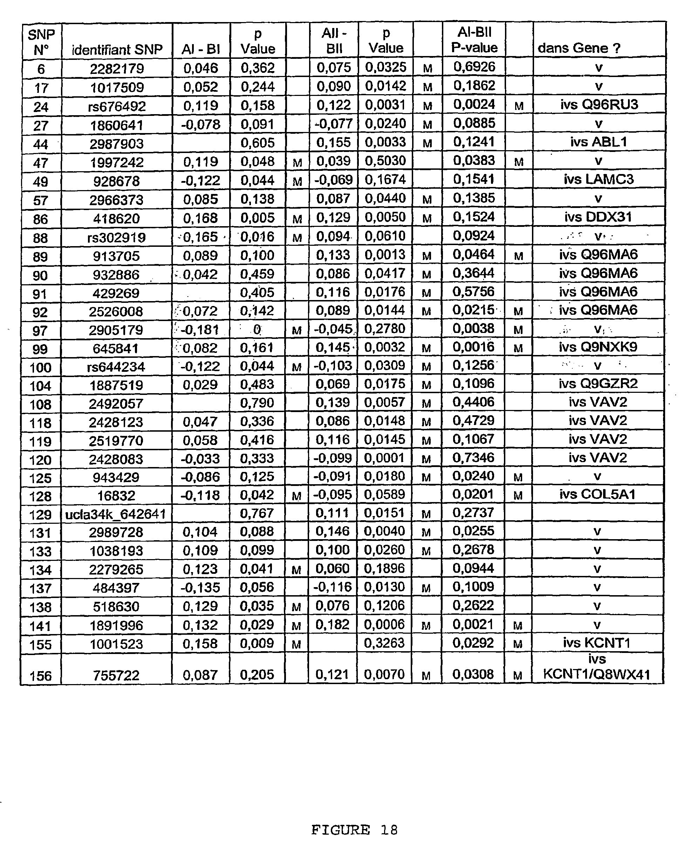

- **Figure 18 :**: Tableau répertoriant les 33 SNP retenus pour le génotypage individuel. Les colonnes contiennent le même type d'informations que pour la figure 17.
- **Figure 19 :**: Tableau répertoriant les 43 SNP retenus pour le génotypage individuel. La première colonne indique la position sur le chromosome, la seconde leur identifiant, la colonne suivante leur numéro (numéro attribué à l'étape précédente de 1 à 288 le long de la région A, du télomère p vers le télomère q). Les colonnes suivantes indiquent s'il s'agit de SNPs présents au sein d'un cluster ou de doubles spots.
- **Figure 20 :**: Tableau répertoriant les 33 SNP retenus pour le génotypage individuel. Les colonnes contiennent le même type d'informations que pour la figure 19.
- **Figure 21 :**: Représentation schématique des résultats de déséquilibre de liaison sur la région A. La significativité des associations entre SNPs pris deux à deux est représentée par un code de couleur.
- **Figure 22 :**: Représentation schématique des résultats de déséquilibre de liaison sur la région B. La significativité des associations entre SNPs pris deux à deux est représentée par un code de couleur.
- **Figure 23 :**: Graphique représentant la comparaison des fréquences alléliques/génotypiques pour chaque SNP de la région A dans les groupes 'canitie précoce' et contrôle, mettant en lumière les associations SNPs/phénotypes.
Les gènes concernés sont indiqués en abscisse avec les SNPs.

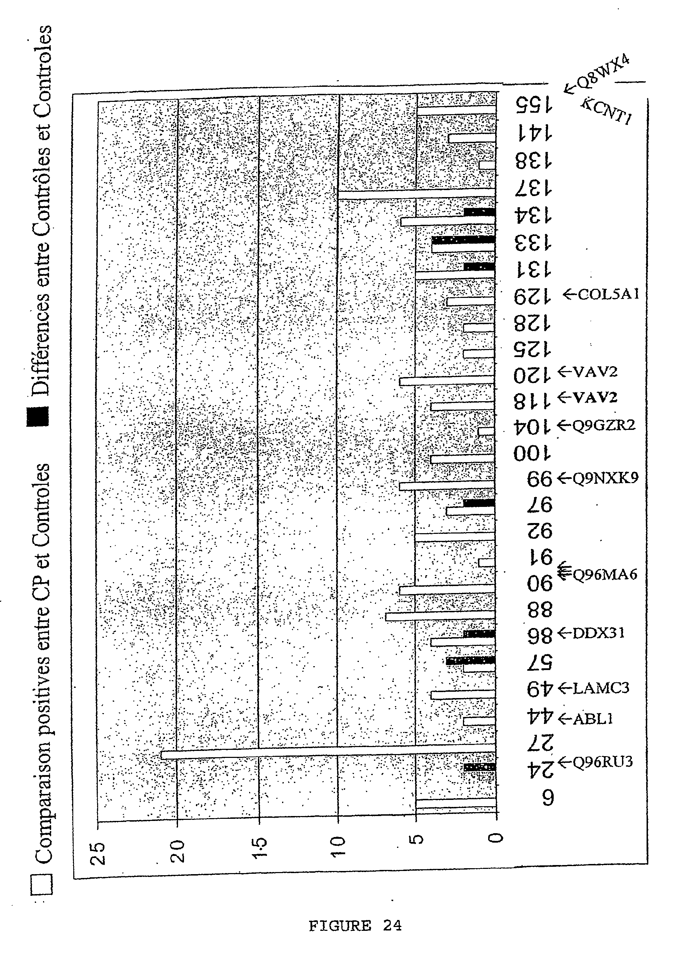

- **Figure 24 :**: Graphique représentant la comparaison des fréquences alléliques/génotypiques pour chaque SNP de la région B dans les groupes 'canitie précoce' et contrôle, mettant en lumière les associations SNPs/phénotypes.
Les gènes concernés sont indiqués en abscisse avec les SNPs.

### EXEMPLES

### EXEMPLE 1

### Résumé des travaux effectués

Afin de localiser le ou les gènes de la canitie précoce (CP), il a été réalisé un programme d'analyse de ségrégations (liaison génétique) chez des familles où ce trait se transmet à travers les générations. Au terme d'une série de présélections sur la base de la puissance statistique de l'échantillon et de la confirmation des phénotypes, douze familles sont retenues pour participer à une étude de liaison et l'ADN fut préparé à partir d'un échantillon de sang périphérique de chacun des individus informatifs (présentant et ne présentant pas le trait). L'étude a été réalisée selon deux approches principales, analyse ciblée sur une région-candidate et étude génome-global sur les vingt-deux chromosomes autosomiques et le chromosome X.

De l'ensemble des analyses réalisées en fixant ou ne fixant pas des paramètres pour la transmission du trait de CP, deux loci potentiels se dégagent sur les chromosomes 6 et 9. Le locus sur le chromosome 6p21-p12 entre les marqueurs D6S1629 et D6S1280, dans la région des gènes du complexe majeur d'histocompatibilité (MHC, HLA) présente de robustes évidences pour contenir un gène de prédisposition et un autre locus (chromosome 9q31-q32) montre également des signes suggestifs de liaison à la CP.

Trois autres loci (chromosomes 11q14-q22, 5q31-q32, 3p14.1-p12.3) montrent également des signes suggestifs de liaison à la CP.

Cette étude, et en particulier la discordance entre les scores obtenus pour les analyses paramétrique/non-paramétrique, suggère que la canitie précoce ne serait pas provoquée par un petit nombre de gènes à effet majeur, mais serait plutôt gouvernée par un système multifactoriel incluant l'action de plusieurs gènes de prédisposition.

### INTRODUCTION

Le caractère héréditaire de la canitie précoce (CP), ou apparition des cheveux blancs tôt dans la vie, est une hypothèse proposée depuis bien longtemps, du fait du caractère familial du blanchiment précoce des cheveux chez certains.

Pour explorer la canitie de par son aspect génétique, il a été réalisé une étude de ségrégation de l'ADN chez des familles dont la canitie apparaît très tôt dans la vie. Afin de garantir les meilleures chances de succès pour cette chasse aux gènes, la composition de l'échantillon pour l'étude s'est déroulée selon un protocole rigoureux pour l'attribution du phénotype et la sélection des familles. Le phénotype CP fut attribué aux seuls individus qui présentaient des cheveux blancs avant 25 ans et dont la moitié de la chevelure était grise à 30 ans. Les familles furent retenues pour l'étude sur la base de leurs performances statistiques dans l'analyse de ségrégation.

La partie de l'étude réalisée est décrite selon quatre époques principales:
A- Epoque 1: Détermination du potentiel de l'étude. Un première sélection des familles les plus informatives est réalisée par une simulation d'analyse de liaison.
B- Epoque 2: Confirmation médicale des phénotypes et collection des échantillons sanguins chez les familles présélectionnées. Cette campagne de vérification aboutit à une nouvelle liste de familles candidates pour l'étude. Une nouvelle simulation de liaison permet d'estimer le potentiel de l'échantillon corrigé.
C- Epoque 3: Analyse de liaison génétique avec les régions chromosomiques candidates pour la CP. Première phase d'analyse des ADNs pour les régions chromosomiques qui pourraient contenir les gènes de la CP.
D- Epoque 4: Analyse de liaison génétique globale de la CP sur tout le génome humain. Analyse de ségrégations familiales sur l'ADN des 22 chromosomes autosomiques et le chromosome X afin de détecter les régions qui se lient au trait CP.

Les résultats obtenus à chaque époque sont présentés sous forme de tableaux et de figures de manière synthétique dans les tableaux récapitulatifs ou de manière profonde dans les tableaux détaillés.

### RESULTATS

### A- Epoque 1: Choix des familles avec l'aide de la simulation d'analyse de liaison

Au terme d'une tentative de sélection des familles avec une canitie précoce selon des critères d'informativité pour une localisation génique, 29 familles ont été soumises à une simulation d'analyse de liaison génétique. Sur la base de la disponibilité de tous les individus, il s'avérait que ce projet possédait un potentiel de succès très encourageant car dix-neuf pedigrees (soit 255 individus) furent alors retenus. Cette conclusion n'étant valable que si les phénotypes sont confirmés et si la majorité des sujets acceptent de participer à l'étude. Parmi cette sélection se trouvent sept familles très informatives qui individuellement pourraient atteindre ou dépasser un Lod score Z=4.00 (soit plus que la limite inférieure de significativité du Lod score qui est de Z=3.00). Afin de rassembler un maximum de chance de succès, il était très important que le diagnostic de CP soit attribué avec rigueur.

Le résultat de cette étude permet, selon la robustesse de l'évaluation clinique, de qualifier les familles qui sont ensuite collectées pour l'étude génétique.

### 1. Critères pour l'attribution du phénotype CP

Vingt-neuf familles parmi les 65, ont été retenues, sur des critères de structure (nombre d'individus total, atteints, disponibles), pour être analysées en simulation.

Lors du processus de simulation:
a) un logiciel génère une série de réplicats du fichier code en assignant des génotypes simulés. Le fichier obtenu pour chaque famille explore plusieurs combinaisons alléliques (génotypes) chez chacun des individus.
b) un logiciel vient ensuite analyser chacun des réplicats pour estimer les Lod scores (Z) possibles de analyse de liaison génétique chez chaque famille. Les résultats, sous forme de Lod scores minimum, moyen et maximum permettent ainsi d'évaluer le potentiel de chaque famille dans ce type d'étude.

Evidemment, ces estimations ne restent valables que dans le cas où chaque individu s'est vu attribuer un phénotype correct. En cas d'incertitude, le phénotype doit être indiqué comme inconnu; il n'est alors pas pris en compte dans l'étude et donc ne nécessite pas d'être prélevé. Le Lod score diminue (dans des proportions variables) chaque fois qu'un individu est enlevé pour phénotype incertain.

Les arbres généalogiques ont été redessinés à l'aide d'un logiciel de gestion de pedigrees, qui construit également les fichiers codés (fichiers preplink) pour l'analyse de liaison génétique. En plus des codes indiquant pour chaque individu, les liens de parenté, le sexe, le phénotype ainsi que le génotype qui sera incrémenté par le logiciel de simulation SLINK, un code de disponibilité (cd) **(tableau 1)** est attribué. Il pondère ainsi, par un code de 0 à 3, le caractère informatif de chaque individu dans l'étude.

Le phénotype de chaque individu fut assigné d'après les informations figurant dans les pedigrees et les tableaux de description du rapport Genormax. Toutefois, pour certains individus, le phénotype a été modifié d'après les critères indiqués dans le **tableau 2.** Les individus, non-présents sur les pedigrees initiaux (identifiés par un numéro seulement) ont été portés phénotypiquement inconnus, et ont été considérés indisponibles (cd=3).

### a. Codes de disponibilité

Lors de la simulation, n'ont été pris en compte que les individus pour lesquels **(tableau 1):**
- il sera possible, d'après l'étude Genormax, de prélever du sang en vue de préparer l'ADN pour les étude de génétique (âge, domicile en France métropolitaine/outremer/étranger, consentement à priori);
- le phénotype de canitie précoce aura été clairement défini **(tableau 2).**

**Tableau 1: Définition des codes de disponibilité**

| code de disponibilité (cd) | ADN | phénotype |
|---|---|---|
| 0 | indisponible | connu |
| 1 | disponible | inconnu |
| 2 | disponible | connu |
| 3 | indisponible | inconnu |

**Tableau 2: Définition des phénotypes**

| | < 25ans | 25 ans | >25ans |
|---|---|---|---|
| pas de cheveux blancs | 0 | 0 | 1 |
| quelques cheveux blancs (qb) (- de 50%) | 2 | 0 | 0 |

### b. Assignation du phénotype selon l'âge

Afin d'écarter les risques d'erreurs, les critères suivants ont été définis pour l'assignation du phénotype en fonction de l'âge chez les individus de moins de 30 ans. Toutefois, lors de l'examen clinique final, il est souhaitable que la définition du phénotype soit plus quantitative.

### c. Autres paramètres

Après examen de la variation du Lod score maximum chez la famille Can65 (test 100, 200, 300, 500 réplications), le nombre de réplications (générations des combinaisons alléliques) a finalement été fixé à 200.

La fréquence du trait a été fixée à 1%. Le nombre d'allèles possibles pour le génotype est fixé à 6 avec une fréquence équivalente pour chacun.

### 2. Résultats

### a- Classification des familles selon leurs scores

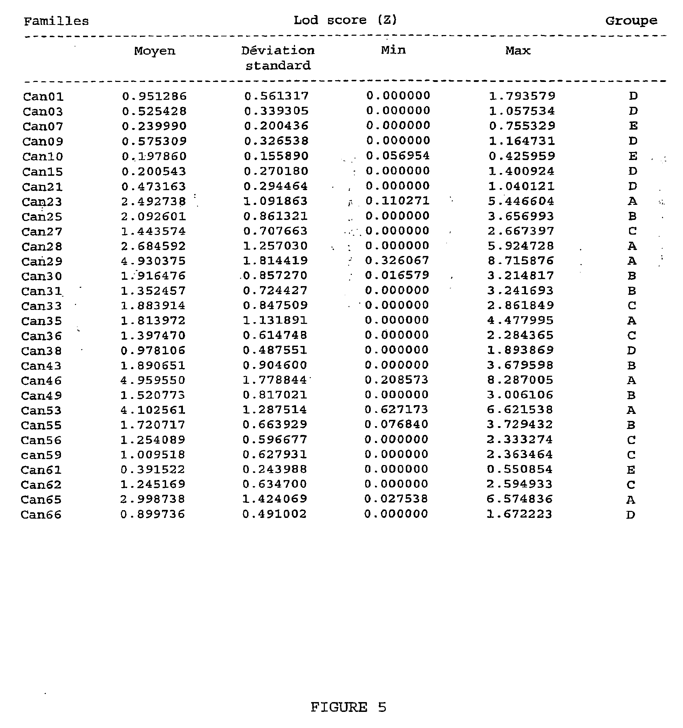

Le **tableau 3** donne une indication du potentiel des familles GENORMAX en fonction du Lod score maximum (Zmax) atteint (groupe A-E). La **figure 5** rapporte le Lod score simulé pour chaque famille.

**Tableau 3: Statistiques familles/Lod scores maximum. Les résultats détaillés figurent dans le tableau de la figure 5.**

| Lod Score Maximum (LMx) | nombre de familles | groupe |
|---|---|---|
| Zmax ≥ ou = 4 | 7 | A |
| 3≤Zmax<4 | 6 | B |
| 2≤Zmax<3 | 6 | C |
| 1≤Zmax<2 | 7 | D |
| Zmax<1 | 3 | E |

Le Lod score maximum ne peut être atteint que lorsqu'un marqueur de l'ADN est informatif à 100% dans une famille. Le plus souvent, même avec le type de marqueurs utilisés (les marqueurs les plus informatifs dans les régions: chromosomiques à visiter), le Lod score n'atteindra pas sa valeur maximum.

En analyse de liaison génétique, pour être significatif, le Lod score doit atteindre ou excéder la valeur de 3 (résultat à 1000/1).

### b- Effet d'un diagnostic incorrect sur les résultats d'analyse de liaison

L'effet de l'attribution d'un diagnostic incorrect a été testé sur les résultats d'analyse (en cas de liaison à un locus) par une simulation sur la famille Can46 en variant le phénotype de 1,2 ou 3 individus **(tableau 4).**

**Tableau 4: Lod score obtenus pour une série de distances du marqueur au locus du trait (Lod score maximum en gras).**

| 1- selon le diagnostic actuel(lod score maximum) | | | | | | | | |
|---|---|---|---|---|---|---|---|---|
| distance | 0.0 | 0.01 | 0.05 | 0.1 | 0.2 | 0.3 | 0.4 | |
| Lod score | 2.28 | 2.24 | 2.08 | 1.88 | 1.44 | 0.95 | 0.43 | a |

| 2- 3 individus incorrectement diagnostiqués (A2, R10, R19) | | | | | | | | |
|---|---|---|---|---|---|---|---|---|
| distance | 0.0 | 0.01 | 0.05 | 0.1 | 0.2 | 0.3 | 0.4 | |
| Lod score | -3.89 | -1.75 | -0.90 | -0.50 | -0.14 | -0.01 | 0.01 | a |

| 3- 2 individus incorrectement diagnostiqués(A2, R19) | | | | | | | | |
|---|---|---|---|---|---|---|---|---|
| distance | 0.0 | 0.01 | 0.05 | 0.1 | 0.2 | 0.3 | 0.4 | |
| Lod score | -2.85 | -0.75 | -0.05 | 0.22 | 0.36 | 0.30 | 0.17 | a |

| 4- 1 individu incorrectement diagnostiqué(R19) | | | | | | | | |
|---|---|---|---|---|---|---|---|---|
| distance | 0.0 | 0.01 | 0.05 | 0.1 | 0.2 | 0.3 | 0.4 | |
| Lod score | 1.24 | 1.24 | 1.23 | 1.16 | 0.94 | 0.63 | 0.27 | a |

### 3. Discussion, conclusion et décisions

Cette étude de simulation permet de placer les 29 familles présélectionnées en 5 groupes selon le Lod score maximum potentiel. Bien qu'une liaison soit significative dès que la valeur de Lod score de Z=3.00 est atteinte, les inventeurs ont préféré distinguer 2 groupes lorsque ce critère est vérifié car le Lod score simulé maximum est très rarement atteint. Ainsi la probabilité que le Lod score réel pour des familles se plaçant dans le groupe B (3≤Zmax<4) est assez faible.

Les familles du groupe A seront informatives dans une analyse de liaison génétique pour la localisation du/des gènes de la canitie précoce. Pour cela, aucune incertitude ne doit subsister quant au phénotype. Dans le doute, il est conseillé d'exclure des individus, voire des familles de l'étude.

Toutefois, chez certaines de ces familles, la proportion élevée d'individus atteints/non-atteints (parfois tous les enfants atteints) doit inciter à une extrême méfiance sur le caractère génétique à 100% du trait. Bien entendu, il n'est pas exclu que dans certaines familles, le gène CP ait été transmis simultanément par les branches paternelle et maternelle de la première génération. Dans ce cas, les petits enfants seraient tous atteints (Can28, 43, 53...). Afin de pouvoir considérer positivement ces quelques familles, il est hautement souhaitable de vérifier cette hypothèse.

Les familles du groupe B sont elles-mêmes très intéressantes car elles permettent d'étoffer l'échantillon, même si individuellement elles ne pourront accéder à un Lod score significatif dans la plupart des cas. En groupe, elles peuvent cependant permettre de consolider le Lod score, spécialement s'il s'avère que le trait était génétiquement hétérogène (légèrement).

Les familles du groupe C, peuvent être également utilisées dans des études de réplications des résultats de liaison génétique.

Les familles des groupes D et E (Zmax<2) sont peu informatives pour une analyse de liaison génétique.

Il semble, sous réserve d'une caractérisation clinique robuste, que les familles des groupes A, B et C sont appropriées pour une étude d'analyse de liaison génétique et qu'elles doivent être collectées (individus avec cd=2). En effet, les analyses de liaison génétique réalisées sur des échantillons insuffisamment caractérisés sont vouées à l'échec ou à un "pâle" résultat (locus imprécis). Etant donné, que dans de telles analyses, certains paramètres ne peuvent être sous total contrôle (en particulier l'informativité des génotypes), et que l'hétérogénéité génétique toujours possible rend la tâche plus délicate (car elle réduit la puissance de l'analyse sur l'ensemble des familles), il semble donc indispensable de rassembler dès le départ tous les atouts qui peuvent être gérés. Dans cette première étape, les inventeurs ont donc fortement recommandé que le phénotype de chaque individu avec un code de disponibilité approprié (cd=2) soit soigneusement vérifié avant collection (phénotype confirmé, ou exclusion de l'échantillon/de la famille).

### B- Epoque 2: Collection des échantillons, confirmation des phénotypes et nouvelles estimations du potentiel de l'étude

Sur la base des résultats de l'époque 1, les 19 familles (groupes A, B, et C) retenues pour former une banque dans laquelle seront prélevés les pedigrees pour les analyses de liaison génétique, furent contactées pour confirmation du diagnostic CP et collection d'une série d'individus atteints et non atteints.

Cette campagne de vérification médicale des phénotypes a permis de confirmer un grand nombre des diagnostiques CP, mais pas tous comme prévu. Le refus de quelques individus clés (atteints de CP) de participer au projet, le décès de certains ainsi que réajustement d'une partie des phénotypes a conduit à ne pas retenir un certain nombre de familles et réduit le potentiel d'informativité de plusieurs autres familles.

### 1- Ré-estimation du potentiel de l'étude après confirmation des phénotypes

Afin de ré-estimer le potentiel de l'étude après la vérification des phénotypes, les inventeurs ont simulé une analyse de liaison sur les 8 familles qui pouvaient encore être informatives. Le **tableau 5** présente les résultats obtenus pour l'ensemble des 8 familles dans 3 situations d'hétérogénéité génétique (0%, soit toutes les familles liées au même locus, 50% ou seulement la moitié des familles liées au locus, 70% ou seulement environ un tiers des familles liées).

**Tableau 5: Lod scores potentiels en fonction du taux d'hétérogénéité génétique de la CP**

| Les résultats détaillés par familles figurent dans le tableau de la **figure 6****.** | | | | | |
|---|---|---|---|---|---|
| Taux d' Hétérogénéité | Moyen | StdDev | Minimun | Maximum | Max époque 1 |
| 0% | 5.094620 | 1.679698 | 1.221207 | 8.835722 | 38.823 |
| 50% | 1.619190 | 1.553049 | 0.000000 | 7.409957 | - |
| 70% | 0.835383 | 1.022004 | 0.000000 | 5.517145 | - |

### 2- Conclusion

Dans un effort continuel pour l'optimisation de l'échantillon pour les analyses de liaison, 4 familles supplémentaires ont été identifiées **(tableau 6;** 2 familles -can65B et can46B- sont des branches collatérales des familles can65 et can46) et les phénotypes de nouveau vérifiés.

Après une nouvelle simulation **(tableau 7),** il s'avère que le Lod score maximum général de liaison est à peine supérieur (Z=8.91) si les 12 familles retenues (phénotypes strictement définis) sont liées au même locus. L'augmentation attendue du Lod score par l'apport de nouvelles familles est cependant réduite par la correction et le durcissement des phénotypes.

**Tableau 6: Liste finale des familles**

| | |
|---|---|
| 1 | c103974 |
| 2 | F104512 |
| 3 | CAN33 |
| 4 | CAN35 |
| 5 | CAN43 |
| 6 | CAN46 |
| 7 | CAN46B |
| 8 | Can53 |
| 9 | CAN55 |
| 10 | CAN62 |
| 11 | CAN65 |
| 12 | CAN65B |

**Tableau 7: Nouvelle simulation après collection des nouvelles familles.**

| Les Lod scores potentiels sont exprimés en fonction du taux d'hétérogénéité génétique Les résultats détaillés figurent dans le tableau de **la** **figure 7****.** | | | | |
|---|---|---|---|---|
| Taux d' Hétérogénéité | Moyen | StdDev | Minimun | Maximum |
| 0% | 4.752321 | 1.748924 | 0.356854 | 8.914062 |
| 50% | 1.535356 | 1.418022 | 0.000000 | 6.078132 |
| 70% | 0.719737 | 0.978920 | 0.000000 | 5.282466 |

La puissance de l'échantillon peut être également observée sous l'angle du nombre de réplications qui atteignent ou dépassent les Lod scores Z=1.0, Z=2.0, Z=3.0 respectivement et qui donne une approximation de la chance de trouver une liaison significative **(tableau 8).**

**Tableau 8: Probabilités (en %) d'atteindre ou de dépasser un Lod score de 1, 2 ou 3 pour chaque taux d'hétérogénéité**

| | Taux d' hétérogénéité | 0% | 50% | 70% |
|---|---|---|---|---|
| Lod score | 1.000 | 99.500 | 57.000 | 27.000 |
| | 2.000 | 95.500 | 31.000 | 8.500 |
| | 3.000 | 84.500 | 14.000 | 3.500 |

### C- Epoque 3: Analyse de liaison génétique de la CP avec les régions candidates

### 1- Hypothèse

La région 6p21 est dite « région-candidate » (RC) car elle est associée à la canitie précoce par le biais d'affections auto-immunes (maladie de Biermer, maladie de Basedow, Thyroïdites, Myasthénie) auxquelles le trait est associé.

La région 3p14.1-p12.3 est également dite « région-candidate » (RC) car elle est associée à la canitie précoce par le biais de la maladie de Klein Waardenburg (type IIA) à laquelle le trait est associé.

### 2- Composition finale des familles

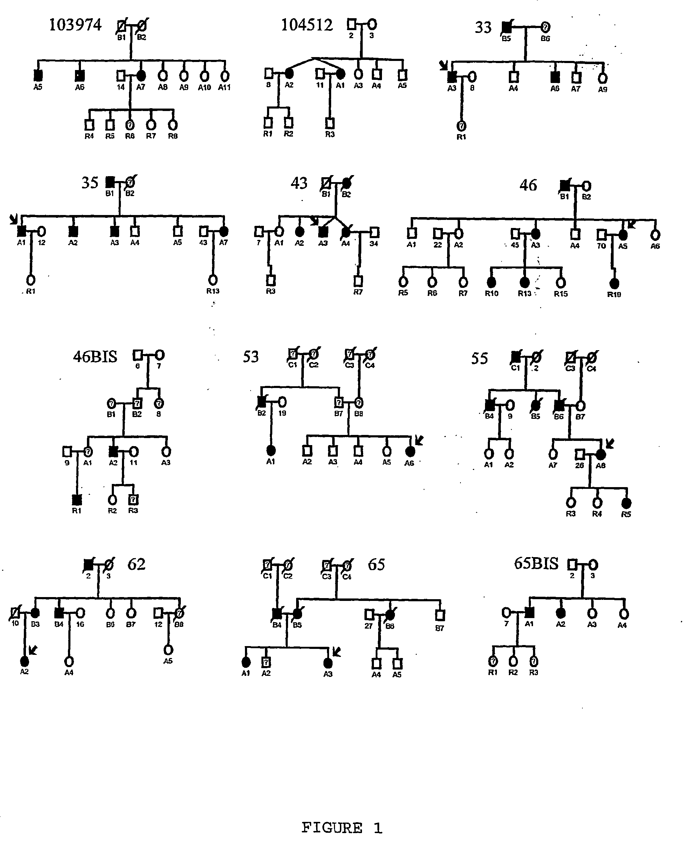

Dans un souci d'optimisation technique, un échantillon de 92 individus atteints et non-atteints parmi les 12 familles est retenu **(voir** **figure 1****)**. Cette sélection est formée en fonction de l'informativité potentielle de chaque individu et confirmée par une nouvelle simulation de liaison. L'ajout de quelques individus conduit à une légère augmentation du Lod score potentiel (de 8.91 à 9.04, selon homogénéité complète, **tableau 9)** et donc de la puissance de l'échantillon **(tableau 10).**

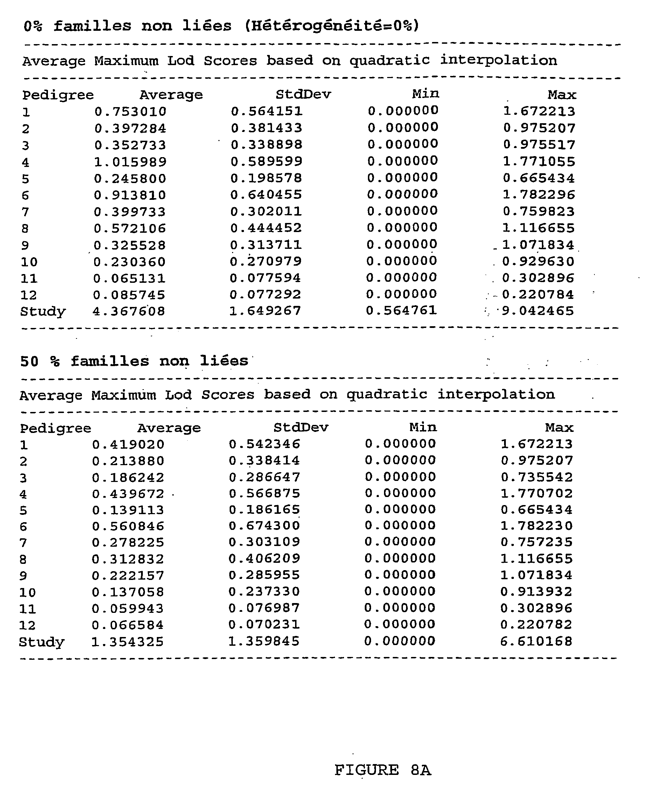

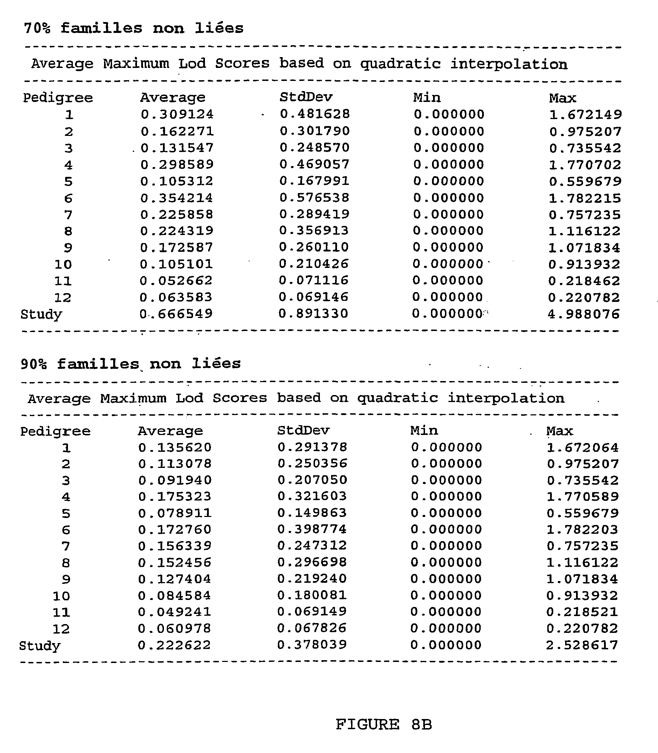

**Tableau 9 Nouvelle simulation sur les familles finalisées pour la recherche sur les régions chromosomiques candidates. Lod scores potentiels en fonction du taux d'hétérogénéité génétique. Les résultats détaillés figurent dans le tableau de la figure 8.**

| Taux d' hétérogénéité | Moyen | StdDev | Minimun | Maximum |
|---|---|---|---|---|
| 0% | 4.367608 | 1.649267 | 0.564761 | 9.042465 |
| 50% | 1.354325 | 1.359845 | 0.000000 | 6.610168 |
| 70% | 0.666549 | 0.891330 | 0.000000 | 4.988076 |
| 90% | 0.222622 | 0.378039 | 0.000000 | 2.528617 |

**Tableau 10: Probabilités (en %) d'atteindre ou de dépasser Lod score de 1, 2 ou 3 pour chaque taux d'hétérogénéité.**

| Les résultats détaillés figurent dans le tableau de la **figure 9****.** | | | | |
|---|---|---|---|---|
| Taux d' hétérogénéité | 0% | 50% | 70% | 90% |
| Lod score | | | | |
| 1.000 | 98.000 | 48.500 | 21.500 | 4.000 |
| 2.000 | 94.500 | 23.500 | 9.500 | 0.500 |
| 3.000 | 79.000 | 12.000 | 3.000 | 0.000 |

### 3- Marqueurs microsatellites utilisés

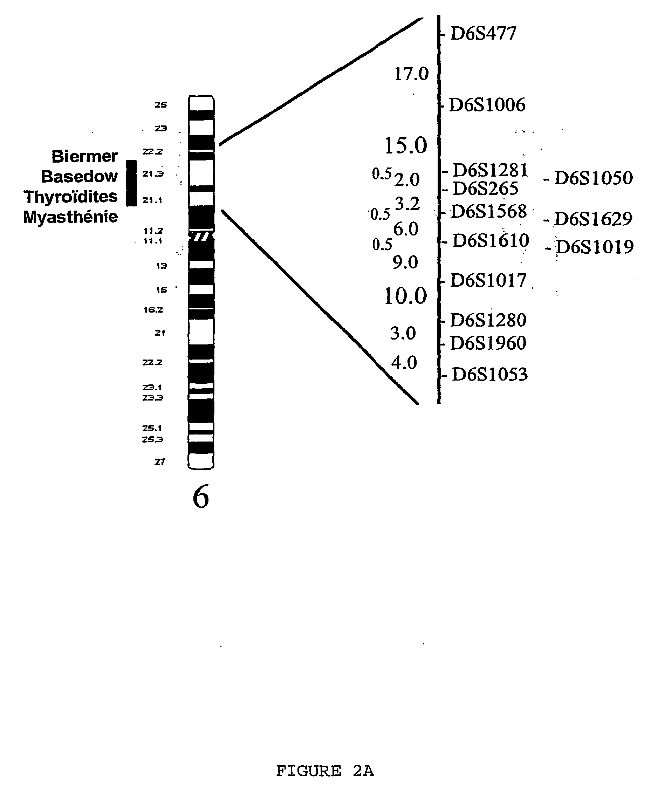

La distribution des marqueurs sur la région candidate du chromosome 6 est présentée dans la **figure 2A****.**

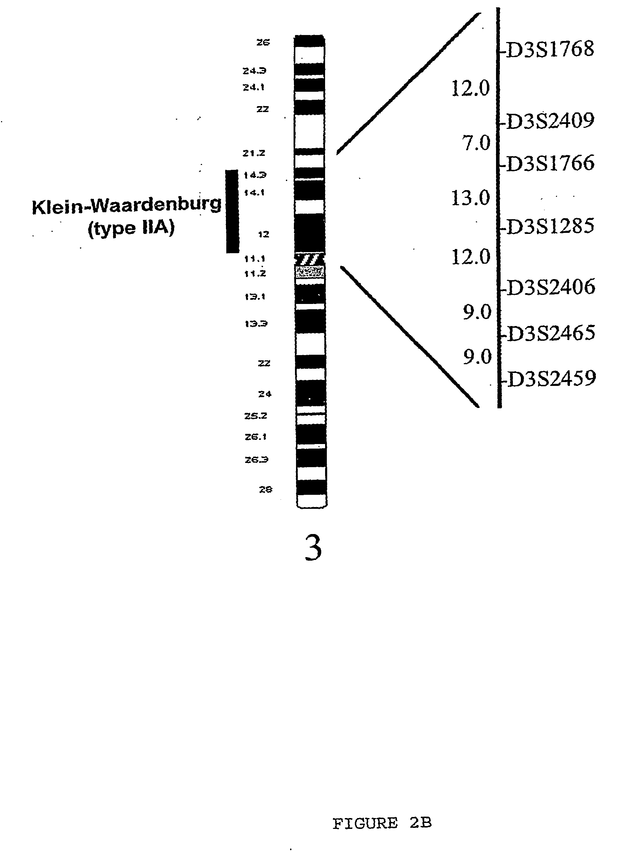

La distribution des marqueurs sur la région candidate du chromosome 3 est présentée dans la **figure 2B**

### 4- Analyses de liaison

Plusieurs types d'analyses de liaison ont été réalisés afin d'augmenter la probabilité d'observer une liaison existante entre ces régions chromosomiques et la CP.

Pour cette analyse, les deux approches suivantes ont été réalisées:
- 2-points (analyse itérative entre le trait et les marqueurs pris un-à-un);
- multipoints (analyse pour chaque chromosome selon une carte de marqueurs placés en fonction de leurs distances respectives).

1. Analyses avec paramètres définis, paramétriques (PL): Mode de transmission (dominant), fréquence du trait (1%), équifréquence des allèles de marqueurs testés et pénétrance (90 % hétérozygotes mutants- 100% homozygotes mutants). Méthode 2-points et multipoints.
2. Analyse indépendante du mode de transmission du trait, non-paramétrique (NPL):
   Analyse de déviation de la proportion d'allèles partagés pour chaque paire d'individus atteints (dans chaque famille par identité par descendance) par rapport à une transmission au hasard. Dans l'analyse multipoints, toutes les paires d'atteints (all-pairs, score Z-all=log₁₀ de p-values, et p-values) ont été considérées. Dans la méthode 2-points, ce sont les paires de germains qui ont été étudiées (affected sib-pair, p-values).

### 5- Résultants

Les résultats sont exposés dans le **tableau 11.**

**Tableau 11: Résultat de l'analyse de liaison sur les régions-candidates**

| Quand seule une position est indiquée, et non pas un nom de marqueur, cela signifie que la position est intermédiaire entre 2 marqueurs. | | | | | | | | |
|---|---|---|---|---|---|---|---|---|
| | | **Global** | | | | | | |
| | | **2-points** | | | **multipoint** | | | |
| **Chromosome** | **Région** | **Lod** | | **Marqueur/ position cM** | **Lod** | **Marqueur/ position cM** | **NPL** | **Marqueur/ position cM** |
| 3 | 3p14.1-p12.3 | 1.03 @ 0.2 | | D3S1285/90 | 1,55 | 82 | 2,58 | D3S2409/70 |
| 6 | 6p21 | 1.12 @ 0.1 | | D6S1017/54 | 1,28 | 58 | 3,56 | D6S1017/54 |
| | | | | | | | | |

| | | **Familles positives** | | | | | | |
|---|---|---|---|---|---|---|---|---|
| | | | **2-points** | | **multipoint** | | | |
| **Chromo -some** | **Région** | **ID** | **Lod** | **marqueur** | **Lod** | **position en cM/ marqueur** | **NPL** | **position en cM/ marqueur** |
| 3 | 3p14.1-p12.3 | 53 | 1,54 | D3S1285 | 1,61 | D3S1285 | 1,58 | D3S1285 |
| 6 | 6p21 | 35 | 1,67 | D6S1629 | 0,93 | 40/D6S1629 | | |

### 6- Discussion et conclusion

Cette étude distingue un locus prédominant de prédisposition à la canitie précoce sur le **chromosome 6p21-p12** dans la région des gènes HLA (autour de **D6S1017;** scores maximum **NPL= 3.52, p=0.000514, HLod:=2.01**). La région de liaison (avec un degré de confiance de 99%, NPL>2.50) se situe entre les position 41 et 67 sur la carte des marqueurs sélectionnés. L'analyse non-paramétrique fournit une série de valeurs significatives entre les positions 47 cM et 58 cM **(entre D6S1019 et D6S1280).** Le Lod score bien que de rang suggestif supporte également la localisation d'un gène important sur le chromosome 6p.

Ces résultants documentent positivement les présomptions d'association qui portent sur la région du chromosome 6p21 vis-à-vis de la canitie précoce.

Il est à noter qu'une famille (CAN35) montre une liaison relativement élevée.

Le résultat de la liaison sur le chromosome 6p21 dans la région du HLA est très encourageant si l'on se souvient que la littérature fournit peu d'exemples d'analyses non-paramétriques aussi informatives. Il fournit un point de départ prometteur vers l'identification des gènes de susceptibilité à la Canitie Précoce, à l'aide des stratégies d'associations de polymorphismes.

Cette étude distingue également un locus fortement suggéré de prédisposition à la canitie précoce sur le **chromosome 3p14-p12** (vers les marqueurs **D3S2409 et D3S1766;** multipoints **NPL= 2.58** à la position 12 et **HLod:=1.55** à la position 24).

Ce résultat documente positivement les présomptions d'association qui portent sur la région chromosomique 3p14-P12 vis-à-vis de la canitie précoce.

Il est à noter qu'une famille (CAN53) montre une liaison relativement élevée pour cette région chromosomique.

### D- Epoque 4: Analyse de liaison génétique globale de la CP avec le génome.

L'analyse globale du génome permet de faire une visite de tous les chromosomes (un sondage global) afin de trouver des régions impliquées et éventuellement un locus majeur qui gouvernerait la canitie précoce. Cette grande analyse permet également d'estimer le taux d'hétérogénéité génétique de la CP.

### 1- Contenu des familles

Ce sont les mêmes familles que celles qui ont été étudiées dans la phase régions-candidates (époque 3), mais avec cependant un ajustement de certaines dans leur contenu (voir **tableau 12).** Certains membres peu informatifs ont été remplacés par d'autres, plus récemment recrutés, ou dont le diagnostic a été précisé plus tardivement.

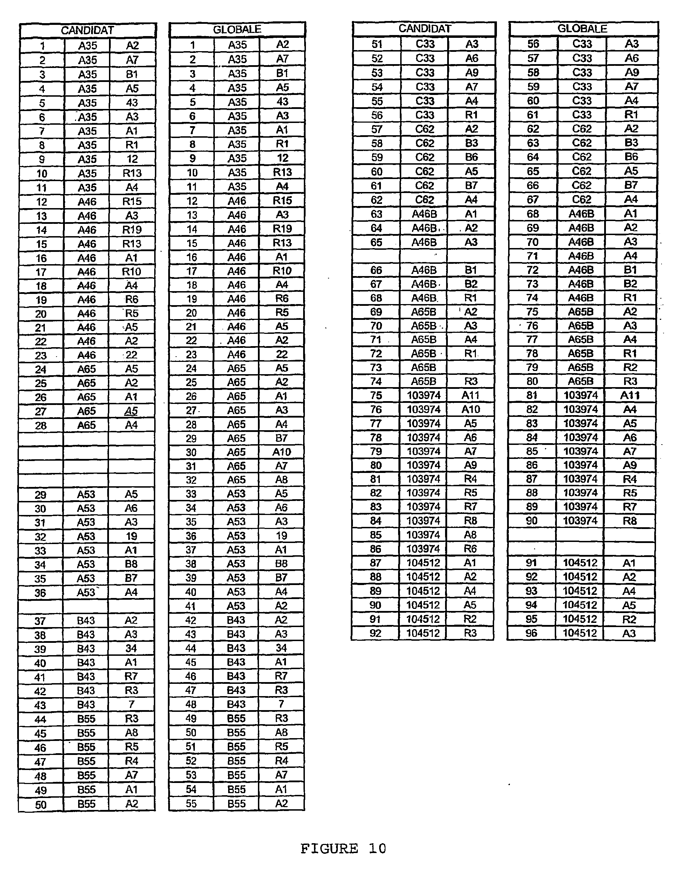

**Tableau 12: Comparaison du nombre d'individus étudiés dans chaque familles entre l'analyse régions-candidates et l'analyse génome-global. (détail composition des familles, tableau de la figure 10)**

| familles | Région Candidate | Génome Global |
|---|---|---|
| A35 | 11 | 11 |
| A46 | 12 | 12 |
| A65 | 5 | 9 |
| A53 | 8 | 9 |
| B43 | 7 | 7 |
| B55 | 7 | 7 |
| C33 | 6 | 6 |
| C62 | 6 | 6 |
| A46B | 6 | 7 |
| A65B | 6 | 6 |
| 103974 | 12 | 10 |
| 104512 | 6 | 6 |
| Total individus | 92 | 96 |

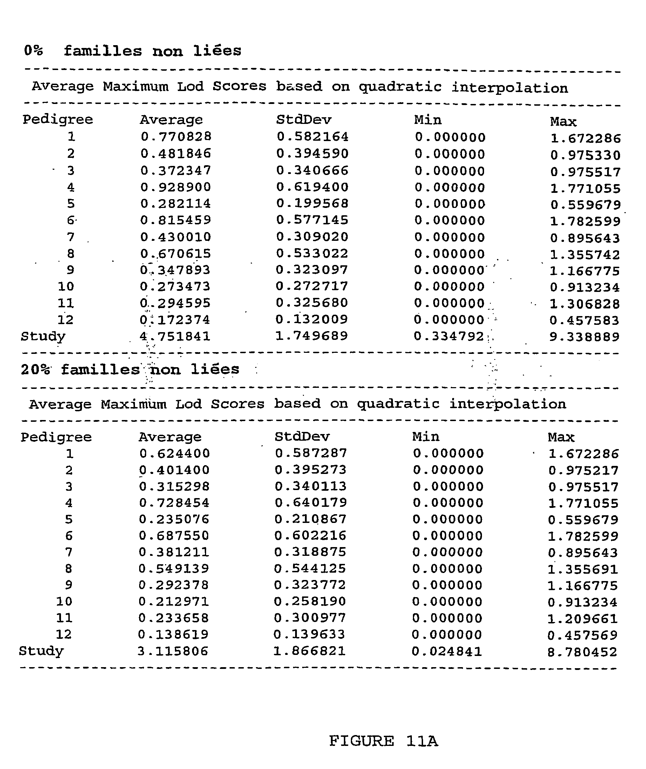

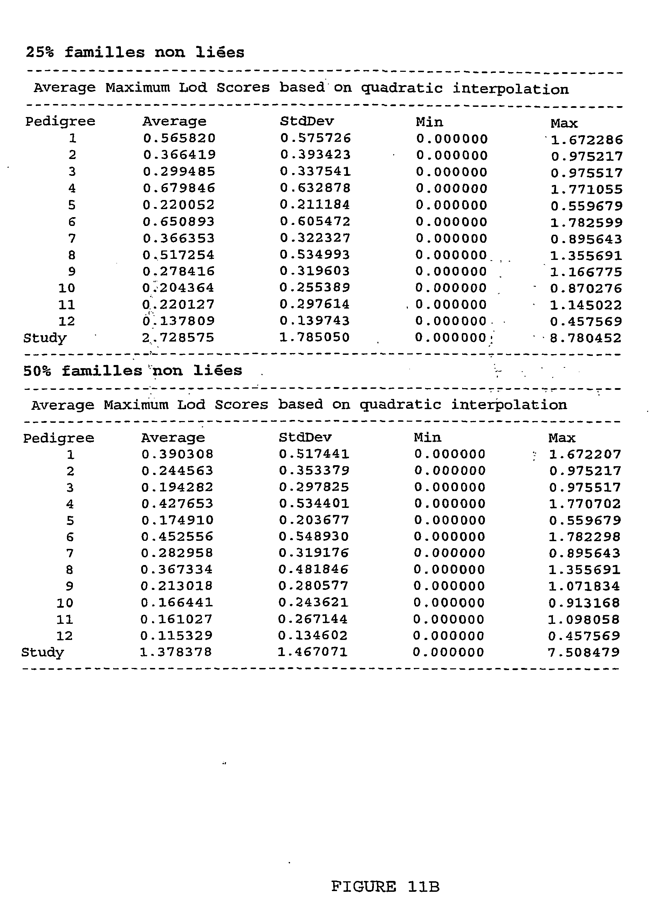

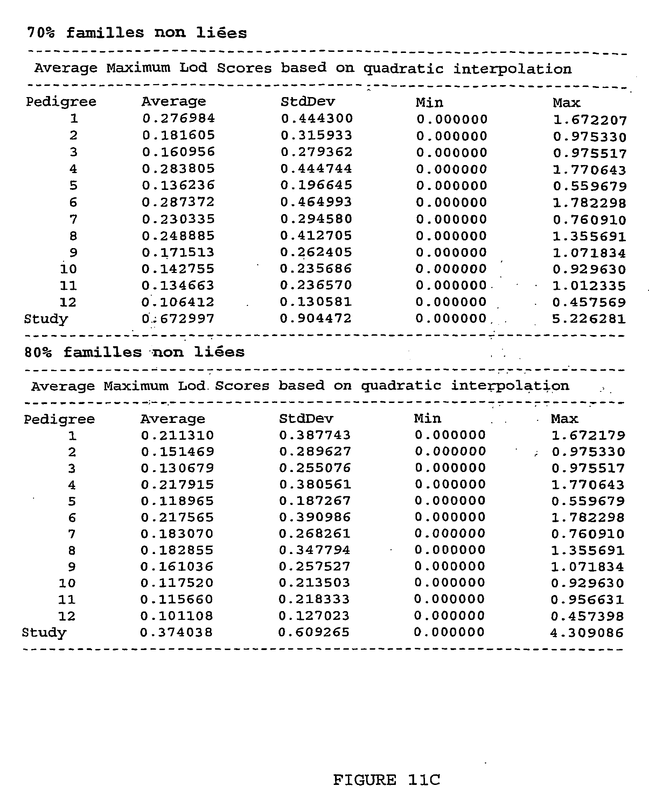

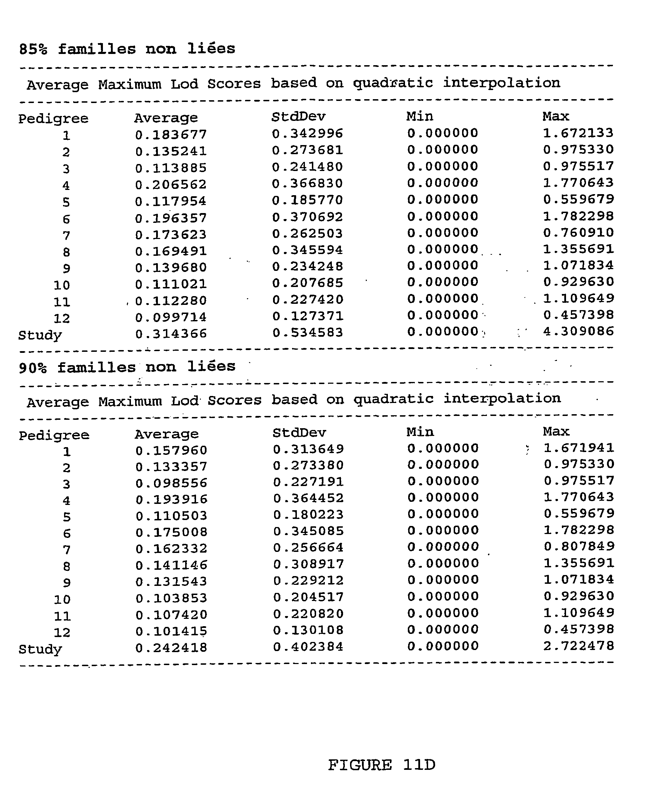

Afin de confirmer le bénéfice de l'évolution de l'échantillon, une nouvelle simulation d'analyse de liaison a été réalisée pour différentes situations d'hétérogénéité génétique **(tableau 13** et tableaux des **figures 11A****,** **11B****,** **11C** **et** **11D****).** Les Lods scores exprimés montrent une évolution favorable. Ainsi, considérant lé Lod score moyen possible, il serait possible d'obtenir un résultat significatif (Z≥3.0) pour une hétérogénéité atteignant 20% (soit 1/5 des familles non liées au locus). En fait, ce résultat est très conservateur et il serait possible d'atteindre la significativité avec un échantillon nettement plus hétérogène (soit 50-70% avec l'utilisation de marqueurs microsatellites montrant une hétérozygosité moyenne de 0.7).

**Tableau 13: Lod scores potentiels en fonction du taux d'hétérogénéité génétique de la CP**

| Les résultats détaillés figurent dans les tableaux de la **figure 11****.** | | | | |
|---|---|---|---|---|
| Taux d' hétérogénéité | Moyen | StdDev | Minimun | Maximum |
| 0% | 4.751841 | 1.749689 | 0.334792 | 9.338889 |
| 20% | 3.115806 | 1.866821 | 0.024841 | 8.780452 |
| 50% | 1.378378 | 1.467071 | 0.000000 | 7.508479 |
| 70% | 0.672997 | 0.904472 | 0.000000 | 5.226281 |
| 90% | 0.242418 | 0.402384 | 0.000000 | 2.722478 |

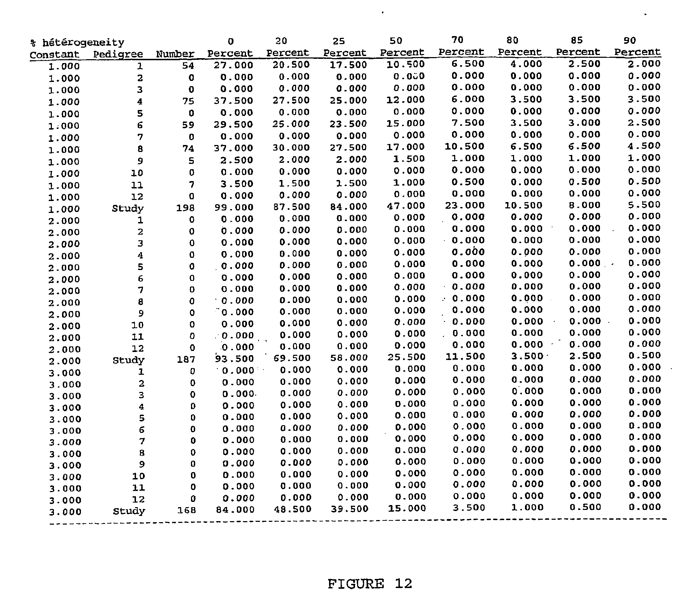

La puissance de l'échantillon peut être également observée sous l'angle du nombre de réplications (de génotypes) qui atteignent ou dépassent les Lod scores Z=1.0, Z=2.0, Z=3.0 respectifs. La probabilité de trouver une liaison significative **(tableau 14** et **figure 12****)** avec 4/5 des familles liées au même locus est de 50%. Ce résultat est basé sur un lod score moyen qui est conservateur.

**Tableau 14: Probabilités (en %) d'atteindre ou de dépasser Lod score de 1, 2 ou 3 pour chaque taux d'hétérogénéité**

| Les résultats détaillés figurent dans les tableaux de la **figure 12****.** | | | | | |
|---|---|---|---|---|---|
| Taux d' hétérogénéité | 0% | 20% | 50% | 70% | 90% |
| 1.000 | 99.000 | 87.500 | 47.000 | 23.000 | 5.500 |
| 2.000 | 93.500 | 69.500 | 25.500 | 11.500 | 0.500 |
| 3.000 | 84.000 | 48.500 | 15.000 | 3.500 | 0.000 |

Les analyses peuvent donc indiquer des liaisons significatives si l'hétérogénéité de l'échantillon ne dépasse pas 20 % (soit seulement 1/5 des familles ne se liant pas à un locus majeur unique), mais il est encore possible d'identifier une liaison dans le cas où la moitié des familles ne sont pas liées à ce locus.

### 2- Marqueurs de l'ADN

L'ADN de 96 individus appartenant aux 12 familles sélectionnées a été génotypé pour 400 marqueurs polymorphes distribués sur les 22 autosomes et le chromosome X (tableau 15) selon un intervalle inter-marqueurs moyen de 9.2 cM (densité). Ce sont des microsatellites de l'ADN, qui sont composés de répétitions en tandem de type dinucléotidique (CA)ₙ de la collection du Généthon (Evry, France).

**Tableau 15: Nombre de marqueurs analysés pour chaque chromosome durant le genome wide-scan.**

| **Chromosome** | **Nb de marqueurs** |
|---|---|
| 1 | 31 |
| 2 | 30 |
| 3 | 23 |
| 4 | 22 |
| 5 | 22 |
| 6 | 20 |
| 7 | 22 |
| 8 | 14 |
| 9 | 20 |
| 10 | 20 |
| 11 | 18 |
| 12 | 19 |
| 13 | 14 |
| 14 | 14 |
| 15 | 14 |
| 16 | 13 |
| 17 | 15 |
| 18 | 14 |
| 19 | 12 |
| 20 | 13 |
| 21 | 5 |
| 22 | 7 |
| X | 18 |
| **total** | **400** |

Le taux d'hétérozygosité moyen observé est de 0.70, et la taille moyenne de l'intervalle inter-marqueurs se situe à 9.2 cM.

### 3- Analyses de liaison

Pour cette approche globale, les inventeurs ont réalisé plusieurs types d'analyses afin d'optimiser leurs performances pour trouver une liaison entre une région du génome et la CP. L'analyse paramétrique, plus performante sur le plan de la puissance, tient compte du mode de transmission du trait et est la plus adaptée dans le cas des traits monogéniques. L'analyse non-paramétrique qui permet d'identifier une liaison même si le mode de transmission supposé est erroné, est aussi plus robuste dans le cas des traits multigéniques.

Pour chacune de ces analyses, les inventeurs ont utilisé les méthodes déjà évoquées, la méthode 2-points (analyse itérative entre le trait et chaque marqueur) et la méthode multipoints (analyse globale sur chaque chromosome selon une carte de marqueurs).
*a- Analyses avec paramètres définis, paramétriques (PL)*
   • Mode de transmission (dominant),
   • Fréquence du trait (1%),
   • Equi-fréquences alléliques de tous les marqueurs,
   • Pénétrances définies (90 % hétérozygotes mutants- 100% homozygotes mutants),
   • Méthodes 2-points et multipoints.
   • Les Scores de probabilité de liaison sont exprimés en:
      - Lod score Z (échantillon homogène);
      - Lod score ZH (échantillon hétérogène) et taux d'hétérogénéité α (proportion de familles qui ne sont pas liées à ce locus).
*b- Analyse indépendante du mode de transmission du trait, non-paramétrique (NPL),*

Il s'agit d'une analyse de la déviation de la proportion d'allèles partagés par paires d'individus atteints par rapport à une transmission des allèles au hasard (identité par descendance). Les inventeurs ont considéré toutes les paires d'individus atteints pour l'analyse multipoints, et les paires de germains pour l'analyse 2-points.

Les scores de probabilité de liaison sont exprimés en
- NPL ou Z-all (Log10 de valeur p) pour la méthode multipoints sur toutes les paires d'individus atteints;
- valeur "p" pour la méthode 2-points sur les paires de germains atteints.

### 4- Résultats

### a- résultats détaillés

Pour le chromosome 6 en particulier, les 20 marqueurs appartenant à la batterie du génome global (GG) ont d'abord été analysés, puis l'analyse a été répétée en additionnant les 13 marqueurs utilisés pour l'analyse région-candidate (RC) aux 20 marqueurs du génome global.

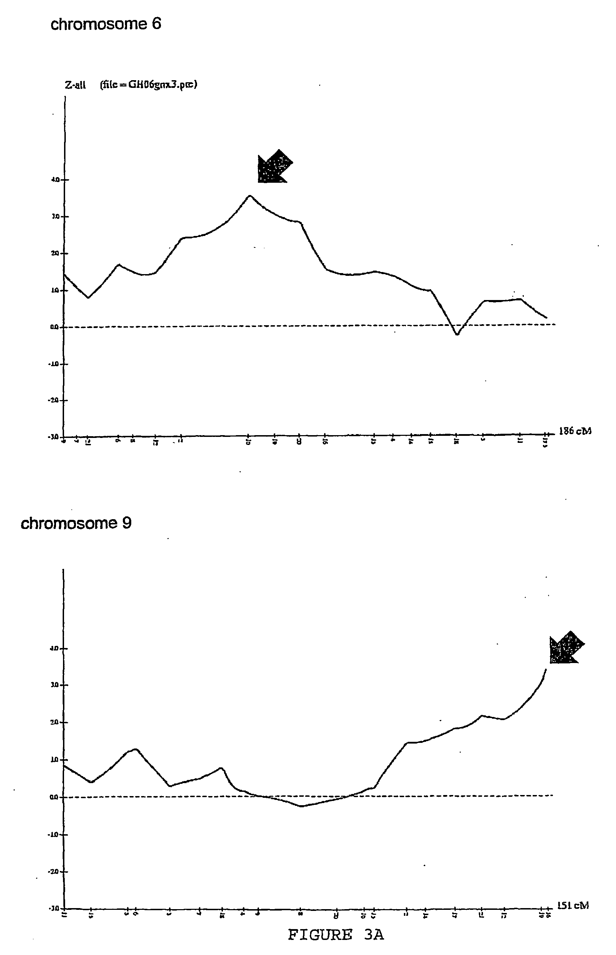

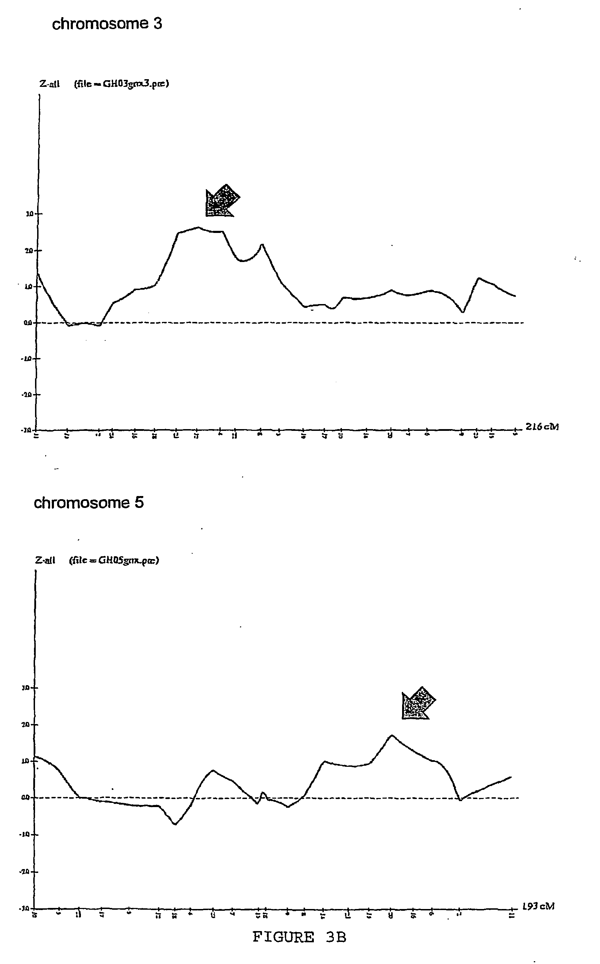

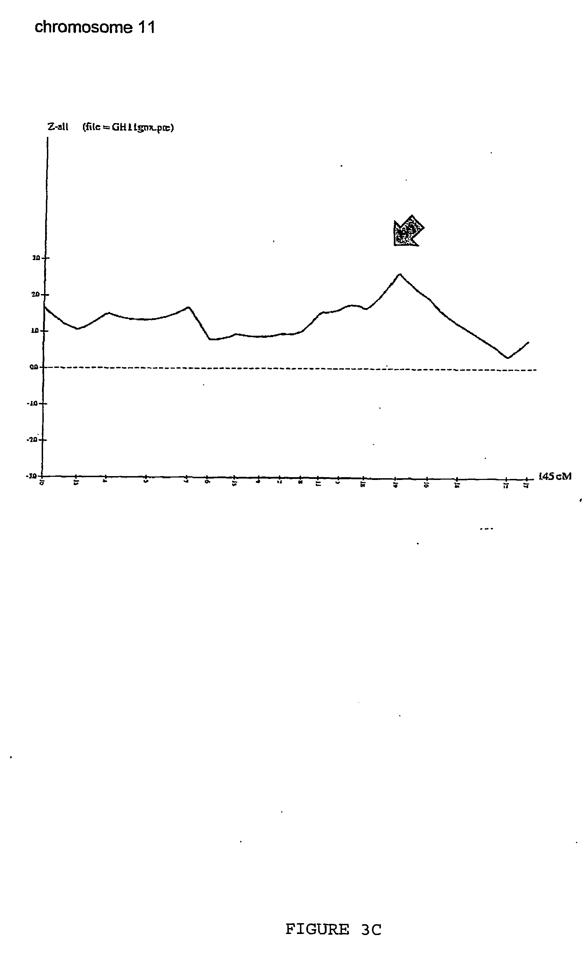

La **figure 3** rapporte les scores NPL obtenus pour l'analyse de liaison non-paramétrique sur les chromosomes 6, 9, 3, 5 et 11.

Le **tableau 16** rapporte les meilleurs résultats retenus pour chaque type d'analyse (PL, NPL) discutés ci-dessous:

**Tableau 16 : Meilleurs résultats retenus pour chaque type d'analyse (PL, NPL) sur les marqueurs utilisés pour l'analyse GG sauf * (* 33 marqueurs RC+GG).**

| ***Chromosome*** | ***Position*/*pter*** | ***Score*** | ***2-point (2P) ou Multipoint (MP)*** |
|---|---|---|---|
| **Non-paramétrique** | | | |
| ***npl >3.0*** | | ***Z-all*** | |
| 6 | 57 | 3,59 | MP* |
| 9 | 151 | 3,37 | MP |

| ***npl* >2.5** | | ***Z-all*** | |
|---|---|---|---|
| 3 | 72 | 2,62 | MP |
| 11 | 106 | 2,61 | MP |

| ***npl* >*2.0*** | | ***Z-all*** | |
|---|---|---|---|
| 9 | 131.00 - | 2,13 | MP |
| 3 | 72 | 2,12 | MP |

| **p<1x10-4** | | **p** | |
|---|---|---|---|
| 6 | 154,1 | 0,000012 | 2P |
| | | | |

| ***Chromosome*** | ***Position*/*pter*** | ***Score*** | ***2-point (2P) ou Multipoint (MP)*** |
|---|---|---|---|
| **Paramétrique** | | | |
| ***Lod* >*2.0*** | | ***Lod*** | |
| 5 | 164,2 | 2,007 | 2P |

| ***Lod* >*1.5*** | | ***Lod*** | |
|---|---|---|---|
| 11 | 106 | 1,5288 | MP |

| ***Lod* >*1.0*** | | ***Lod*** | |
|---|---|---|---|
| 5 | 168 | 1,1118 | MP |
| 6 | 61 | 1,4294 | MP* |
| 6 | 89,7 | 1,458 | 2P |

### i En terme de PL:

a- Un Lod score (2P) ZH=2.007 a été relevé sur le bras long du chromosome 5 (position 164.2 cM à partir du télomère supérieur) soit en position 3/4 sur la bande 5q31-q32.
b- Lod scores intermédiaires (1.5<ZH<2.0)
   - chromosome 11q14-q22, position 106 (MP-ZH=1.53)
c- Lod score intéressants (1.0<ZH<1.5)
   - chromosome 5q31-q32, position 168 (MP-ZH=1.11)
   - chromosome 6p21-p12, position 61 (MP-ZH=1.43)
   - chromosome 6q13-q14, position 90 (2P-ZH=1.46)

### ii En terme de NPL:

Il a été distingué ici les scores log₁₀ pour l'étude de déviation du partage allélique, identité par descendance (IBD), pour toutes les paires d'atteints (scores étude multipoints Z-all) ainsi que les valeurs p pour les paires de germains atteints (affected sib-pairs, scores étude 2-points, p-values).

Les meilleurs scores sont abordés selon leur rang par rapport à la limite de significativité:
- Z-all>3, 2.5<Z-all<3,2.0<Z-all<2.5
- p<10⁻⁵ et 10⁻⁵<p<10⁻⁴

*a- Scores Z-all>3.0*
   Sur le chromosome 6p21-p12, le score atteint en utilisant les marqueurs génome-global maximise à un Z-all=3.52 à la position 71 (entre les marqueurs D6S1610 et D6S257). Toutefois, la précision du locus est probablement affectée par la grande distance entre ces 2 marqueurs de la batterie génome-global qui est plus nettement plus importante que l'intervalle moyen observé (26.11 cM).
   Compte-tenu de la taille de cet intervalle, une analyse complémentaire a été réalisée sur l'ensemble des 33 marqueurs utilisés lors des analyses GG et RC. Cette analyse permet de retrouver un score plus élevé (Z-all=3,59) pour la position 57cM (voir **tableau 16).**
   Le second meilleur score est atteint sur le chromosome 9q31-q32, position 151 (Z-all=3.37)
*b- Scores 2.5<Z-all<3.0*
   - chromosome 3p14-p13, position 72 (Z-all=2.62)
   - chromosome 11q21-q22, position 106 (Z-all=2.61)
*c- Scores 2.0<Z-all<2.5*
   - chromosome 9q31-q32, position 131 (Z-all=2.13)
   - chromosome 3q21, position 101 (Z-all=2.15)
   ainsi que les p-values <10⁻⁵ et 10⁻⁴
*d. p*<*1x10⁻⁴*
- chromosome 6q31.3-q33, position 154 (p=0.000012)

### iii Les loci qui sont identifiés simultanément par PL et NPL:

| | PL | NPL |
|---|---|---|
| 6p21-p12, position 57-61 | 1.42 | 3.59 |
| 11q14-q22, position 106 | 1.52 | 2.6 |

### 5- Discussion et conclusion

Les 2 scores significatifs ou à la bordure de la significativité selon que l'on considère le trait comme étant monogénique ou multifactoriel (Lander et Kruglyak 1995) ont été observés pour les chromosomes 6p21-p12 (NPL MP Z-all=3.59) et 9q31-q32 (NPL MP Z-all=3.37).

Parmi ces 2 loci, le plus robuste est celui du 6p21-p12 qui est renforcé par un MP-PL Lod score, qui bien que moyen, maximise à ZH=1.42.

Un autre locus apparaît également assez intéressant, il est situé sur le chromosome 11q14-q22 car les scores PL et NPL maximisent à la même position 106 (Z-all 2.61, PL 1.52). Le score NPL est d'ailleurs dans la fourchette de suggestivité dans un cas de monogénisme ou dans un cas de multigénisme (suggestivité: monogénisme 2<Z-all<3; multigénisme 2.2<Z-all<3.6).

Enfin le locus 5q31-q32 avec le meilleur PL lod score (2P) (ZH=2.00) se situe également dans des valeurs de suggestivité (en monogénisme).

Il faut ajouter une série de valeur p pour les analyses de germains atteints qui sont également dans la fourchette de suggestivité (chromosomes 6q31.3-q33). Ce sont des loci à considérer aussi.

### D- Discussion et conclusion générales

Au terme des différentes époques d'analyses, plusieurs régions chromosomiques sont identifiées ou suggérées.

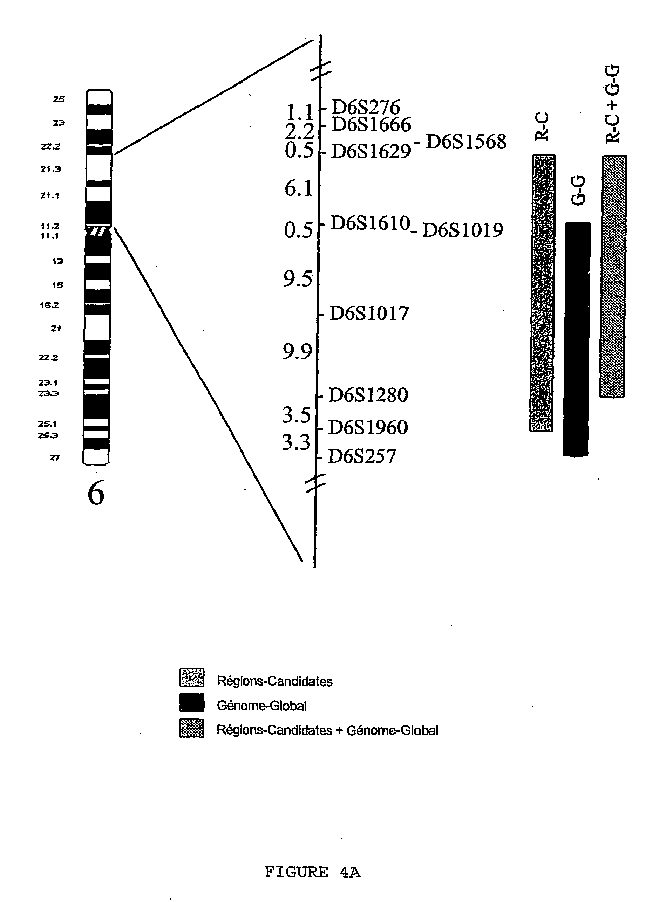

C'est la région 6p21-p12 (**voir** **Figure 4A**) qui a recueilli le meilleur consensus pour une liaison génétique au trait CP. La discordance entre analyses paramétriques et non-paramétriques génère quelques incertitudes quant à l'importance du rôle du ou de ces gènes dans la pathophysiologie de la canitie.

Le second locus est sur le chromosome 9q31-q32 avec un score NPL presque significatif (Z-all=3.37).

### a Chromosome 6p21-p12:

Les limites de la région désignée par l'analyse de liaison, se situent sur la position 41 (limite supérieure, Z-all=2.02) et la position 95 (limite inférieure, Z-all=2.09) soit 54 cM. Il est possible de réduire cette région à une longueur de 15cM en considérant des bornes 65 cM et 80 cM (entre D6S1610 et D6S257 respectivement ; analyse 20 marqueurs) ou 50 cM et 73 cM (entre D6S1629 et D6S1280 respectivement ; analyse 33 marqueurs).

La région identifiée contient les gènes du complexe majeur histocompatibilité (MHC, HLA). Il ne faut cependant pas exclure qu'un gène indépendant du HLA puisse être impliqué dans la gouverne, ou la susceptibilité au trait CP.

### b Chromosome 9q34

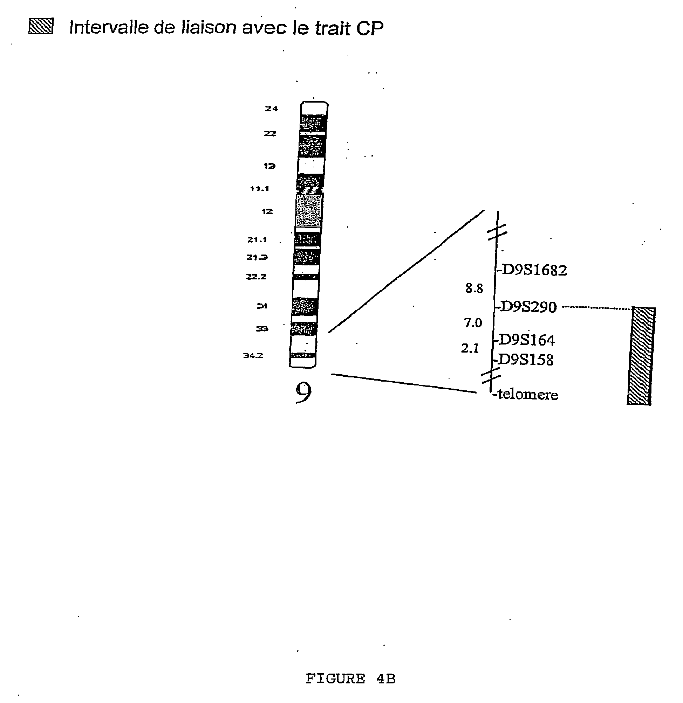

Les inventeurs placent la limite proximale de cette région à partir de la position qui recueille un score Z-all=2.5 sur le marqueur D9S290; la limite distale se plaçant sur le télomère du bras long du chromosome 9 (vers le marqueur D9S158). Cette région s'étend sur une longueur de 10 cM (**figure 4B**).

### c Chromosome 11q14-q22

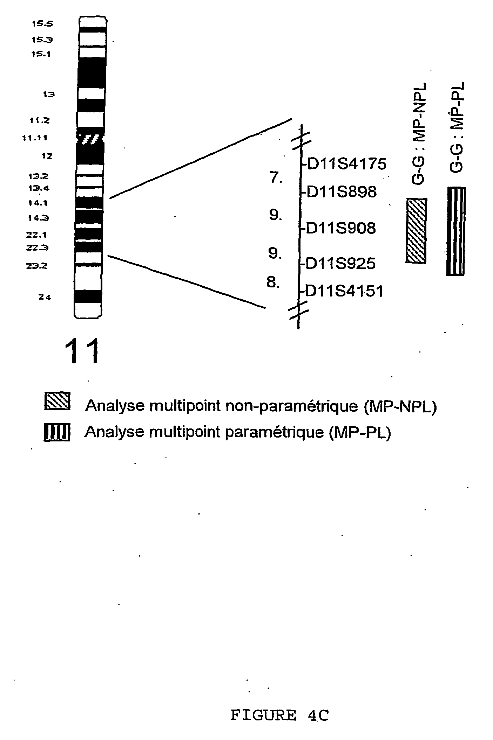

Les inventeurs identifient une région entre les positions 100 et 115 (entre D11S898 et D11S925) (**figure 4C**).

### d Chromosome 5q31-q32

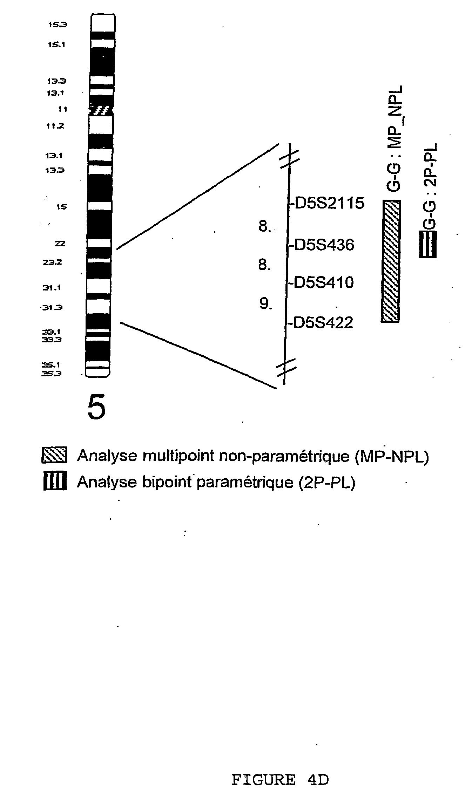

Cette région recueille le meilleur résultat PL et bipoint de ces analyses qui se situe à une distance de recombinaison (theta) 0.14 (environ 14 cM) du marqueur D5S422. En se plaçant à cette distance de D5S422 vers le haut de la carte (position 149), on arrive dans le voisinage du locus qui rassemble le meilleur score NPL multipoints du chromosome 5 (Z-all=1.70, vers marqueur D5S436). Un certain consensus apparaît pour ce locus également (**figure 4D**).

### e Chromosome 3p14.1-p12.3

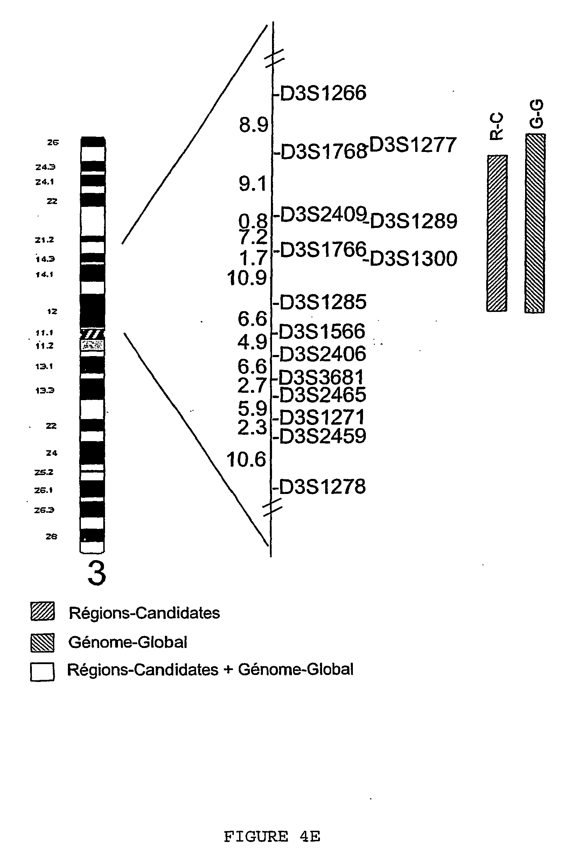

Il s'agit d'une région de presque 30 cM entre les positions 60 et 87 (entre D3S1277 et D3S1285 (**figure 4E**).

### EXEMPLE 2 : Analyse des régions d'intérêt avec des SNP 'Single Nucleotide Polymorphism'

A la suite des travaux présentés dans l'exemple 1, les inventeurs ont poursuivi l'analyse des régions des chromosomes 6 et 9 à l'aide la technologie basée sur les SNP, afin de mettre en évidence les gènes impliqués dans la canitie précoce.

Les SNP (single nucleotide polymorphism) représentent une forme de polymorphisme particulièrement répandue dans le génome humain et très stable. On évalue le nombre de SNP à environ 0,8 SNP par 1000 nucléotides (séquences codantes et non codantes confondues), ce qui permet d'établir une véritable cartographie du génome humain à l'aide des SNP. Les SNP sont souvent classés en différentes catégories, notamment selon qu'ils sont dans une région codante ou non, dans une région régulatrice ou dans une autre région non codante du génome, que le polymorphisme modifie l'acide aminé codé ou non, etc...

A l'issue du programme « Human Genome Project », les SNP sont désormais mieux connus et référencés, ainsi que leur position dans le génome (GDB).

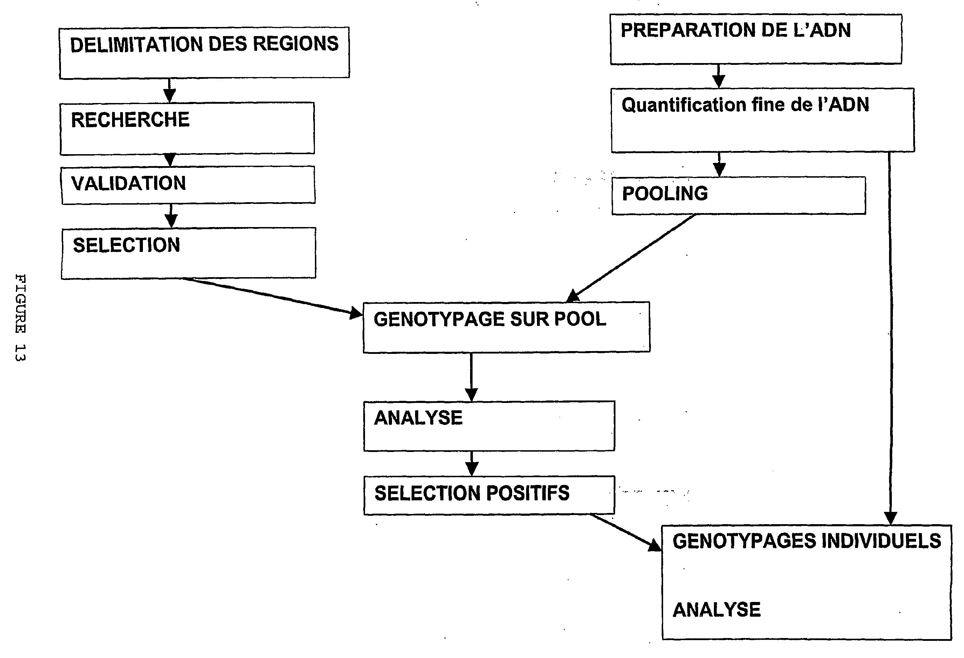

Différentes méthodes ont été mises au point pour mettre en évidence ces polymorphismes entre différents individus, souvent basées sur les méthodes utilisées pour détecter des mutations ponctuelles (RFLP-PCR, hybridation avec des oligomères spécifiques des allèles, mini-séquençage, séquençage direct...).
Dans le cadre de la présente demande, les inventeurs ont utilisé la technologie MALDI-TOF (matrix-assisted laser desorption/ionization time-of-flight mass spectrometry) pour détecter les différents allèles des SNPs candidats. De plus amples détails sur cette technologie sont connus de l'homme du métier et sont décrits dans diverses publications (Stoerker J, et al, Nat Biotechnol 2000 Nov;18(11):1213-6 et Tang K et al, Proc. Natl. Acad. Sci. USA 1999 Aug, 96, 10016-20).
Dans un premier temps, les inventeurs ont défini très précisément les régions des chromosomes 6 et 9 à analyser avec les SNPs. Dans une seconde étape, 1500 SNPs appartenant aux régions précédentes ont été pré-sélectionnés sur certains critères (SNPs candidats in silico) et 697 ont été retenus suite à une étape de validation expérimentale. Dans une étape suivante, les inventeurs ont assemblé les ADN des différents individus atteints de canitie précoce et des individus 'contrôles' en différents groupes, puis effectué le génotypage de ces différents groupes grâce à 450 SNPs choisis parmi les 697. A l'issue de ce génotypage, les résultats ont permis de définir 76 SNPs pour effectuer ensuite un génotypage individuel (et non plus sur des groupes). Les différentes étapes sont décrites plus amplement dans les sections suivantes et représentées schématiquement dans la figure 13.

### 1- Définition des régions à analyser par les SNP

Dans un premier temps, les inventeurs ont défini plus précisément les régions d'intérêt sur les chromosomes 6 et 9, à partir des résultats obtenus grâce à l'analyse avec les marqueurs microsatellites (voir les travaux décrits dans l'exemple 1) sur les 12 familles sélectionnées (voir tableau 6).
La région sur le chromosome 6, dénommée région A, est définie par sa position chromosomique ainsi que par trois autres types de coordonnées pour une précision et une sécurité optimale dans la définition de cette région pour les étapes ultérieures. Il en est de même pour la région du chromosome 9, dénommée région B.
- Région A :: position chromosomique : 6p22-6p12.3
Entre le gène HLA-F et le marqueur microsatellite D6S1651
Entre le SNP rs2075682 et le SNP rs1973934
Entre les positions 39'625'529 bp* et 50'602'544 bp*
- Région B :: position chromosomique 9q34.13-9q34.3 (qter)
Entre le marqueur D9S290 et le télomère 9q
Entre le SNP rs2096071 et le SNP rs1378955
Entre les positions 123'405'258 bp* et 133'021'490 bp*.
* La position de la séquence (en terme de paires de bases bp) est exprimée en fonction de la version de la banque de données sur le génome humain mise à jour en décembre 2001 (soit NCBI Build28).

### 2- Recherche des SNP candidats (in silico) et validation (expérimental).

A partir des régions A et B telles que définies plus haut, une seconde étape a consisté à déterminer une collection de SNPs appartenant à ces régions, de façon à obtenir une carte de marqueurs des deux régions. Ces marqueurs ont également été définis de telle façon qu'ils couvrent les 21Mbp (longueur totale des deux régions) d'une façon homogène et équidistante. La distance entre les différents SNPs a été fixée en moyenne à 30 kb. Cette opération a été réalisée par la sélection de 1500 SNPs répondant à ces critères (SNPs candidats in silico).
Parmi les 1500 ainsi présélectionnés lors de la première étape, 1379 SNPs se sont avérés opérationnels. Par opérationnel, on entend amplifiables à l'aide des réactifs définis de manière usuelle. Les 1379 SNPs sélectionnés ont été analysés sur 92 individus contrôles (individus du Centre d'Etude du Polymorphisme Humain) afin de valider la présence d'au moins deux allèles pour chacun des SNP (validation du polymorphisme).
A l'issue de cette sélection expérimentale, seuls les SNPs présentant une fréquence allélique de l'allèle le plus rare d'au moins 10%, ont été retenus. Par cette méthode, 697 SNPs ont été validés, 465 sur la région A et 232 sur la région B.

### 3- Assemblage des ADN (Pooling)

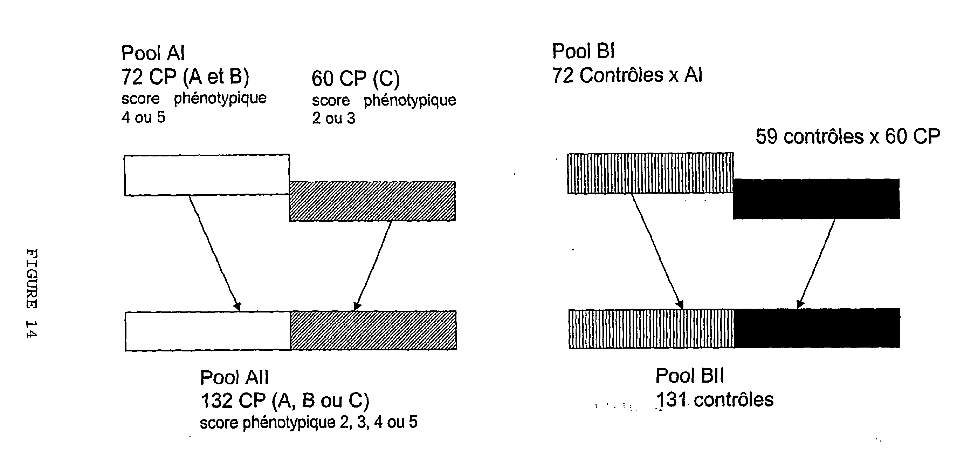

Afin d'augmenter la capacité de génotypage, une stratégie de pooling a été effectuée sur les différents ADN. La puissance de cette méthode est rapportée dans différentes publications (notamment Werner et al, Hum Mutat 2002 Jul;20(1):57-64, Bansal et al, Proc Natl Acad Sci U S A 2002 Dec 24;99(26):16871-4).
Pour effectuer ce pooling, les ADN des différents individus avec le trait 'canitie précoce' (CP) et celui des individus contrôles ont été assemblés. Cet assemblage a été réalisé de façon à ce que chacun des ADN soit représenté de manière équimolaire, afin de garantir qu'aucun individu n'ait d'influence prépondérante sur le résultat par rapport à un autre. A cette fin, la concentration exacte de chacun des ADN a été mesurée par la méthode « Picogreen » dans les différents échantillons des individus.
Des groupes ont été constitués en tenant compte d'un « score phénotypique d'intensité de canitie » qui a été attribué pour chaque individu de la façon suivante.
Dans un premier temps, deux sortes de critères ont été définis, les critères primaires auxquels sont affectés des valeurs de score de 2, et les critères secondaires auxquels sont affectés des valeurs de score de 1.
Il y a 2 critères primaires (valeur de score=2 pour chacun d'eux) qui sont : (i) premiers cheveux blancs avant 18 ans ; (ii) chevelure poivre et sel claire à 30 ans:
Il y a 3 critères secondaires (valeur de score=1 pour chacun d'eux) qui sont : (i) premiers cheveux blancs avant 25 ans ; (ii) Chevelure poivre et sel foncée à 30 ans , (iii) notion familiale de canitie précoce.
En additionant pour chaque individu les scores obtenus avec de chacun des critères diagnostics, on peut définir pour chaque individu un score d'intensité du phénotype de canitie précoce.
Il a ainsi été possible de définir plusieurs groupes différents selon le score phénotypique. Parmi les individus atteints, 72 individus dont lé score est supérieur ou égal à 4 ou 5 et 132 individus dont le score phénotypique est supérieur ou égal à 2.
Groupe AI : ce groupe est constitué par l'ADN des 72 individus CP dont le score phénotypique est de 4 ou 5.
Groupe AII : ce groupe est constitué par l'ADN des 132 individus CP, dont le score phénotypique est de 2, 3, 4 ou 5.
Groupes BI et BII : ces groupes sont constitués par l'ADN des individus contrôles dont l'origine géographique est proche de celle des individus CP. Pour ces individus contrôles, les critères de sélection ont été : (i) un âge supérieur à 40 ans, (ii) l'absence de signe de canitie chez l'individu contrôle, (iii) l'absence de notion familiale de canitie. Les critères d'appariement avec un individu du groupe AI ou AII sont une origine géographique identique, un même sexe et une couleur des cheveux identique à 18 ans.
Ainsi de cette façon outre l'appariement atteint versus non atteint par le trait phénotypique (CP), chaque individu CP du groupe AI est représenté par un individu contrôle dans le groupe BI dont le lieu d'origine géographique est proche ou identique. Il en va de même pour chaque individu du groupe AII.
La constitution des différents groupes est représentée schématiquement sur la figure 14. L'utilisation de ces méthodes rigoureuses de diagnostic clinique des sujets atteints et des sujets contrôles donnent une garantie de fiabilité sur la qualité des données phénotypiques. Par ailleurs, la rigueur de l'appariement selon les règles fixées par les inventuers est la garantie de la pertinence des analyses statistiques comparant les données génomiques issues de ces individus qu'ils soient regroupés en pools ou comparés individuellement.

### 4- Sélection des SNPs validés pour le génotypage sur les ADN groupés.

Parmi les 697 SNPs validés à l'étape 2, 450 ont été choisis lors d'une nouvelle étape de sélection. Cette nouvelle sélection est basée sur l'intervalle entre les SNPs, fixé en moyenne entre 30 et 50 kb.
Les 450 SNPs retenus se partagent ainsi :
Région A : 283 SNPs
Région B : 167 SNPs.
Les différents SNPs utilisés pour les étapes successives sont répertoriés dans les tableaux suivants. Ces tableaux comportent également 9 SNPs additionnels, qui ont été rajoutés à une étape suivante pour compléter la liste. Ces 9SNPs additionnels sont les SNPs n°19, 38, 103, 105 et 287 pour la région A et les SNPs n°86, 97, 131 et 137 pour la région B.
Les 288 SNPs de la région A et les 171 SNPs de la régions B ont été numérotés par ordre de croissant le long (télomère p vers télomère q) des régions A et B qu'ils couvrent de manière quasi-équidistante et homogène.

| Région A : | | | | | | | |
|---|---|---|---|---|---|---|---|
| **SNP N°** | **Identifiant** | **SNP N°** | **Identifiant** | **SNP N°** | **Identifiant** | **SNP N°** | **Identifiant** |
| 1 | rs1610602 | 73 | rs1061808 | 145 | rs847852 | 217 | 326799 |
| 2 | rs1737071 | 74 | rs1044506 | 146 | rs847846 | 218 | 1384546 |
| 3 | rs1737006 | 75 | rs367398 | 147 | rs707967 | 219 | 477011 |
| 4 | rs1736936 | 76 | rs397379 | 148 | rs1886243 | 220 | 227813 |
| 5 | 2734988 | 77 | rs482194 | 149 | rs942373 | 221 | 609699 |
| 6 | 2734967 | 78 | rs505274 | 150 | rs1888822 | 222 | 1338908 |
| 7 | 2524033 | 79 | rs1265758 | 151 | 2071920 | 223 | 1557143 |
| 8 | ucla34k_818417 | 80 | 1555115 | 152 | 2267663 | 224 | 1293467 |
| 9 | rs426483 | 81 | rs743862 | 153 | rs1888823 | 225 | rs542444 |
| 10 | rs259926 | 82 | rs983561 | 154 | 2267664 | 226 | 626965 |
| 11 | rs259919 | 83 | rs1548306 | 155 | rs2038067 | 227 | 857318 |
| 12 | rs1264708 | 84 | 2308818 | 156 | 2894401 | 228 | 636845 |
| 13 | 1015465 | 85 | rs1987948 | 157 | rs1016146 | 229 | 1329711 |
| 14 | rs757259 | 86 | 608766 | 158 | rs2064319 | 230 | 1329714 |
| 15 | rs1029237 | 87 | 60071 | 159 | 2395634 | 231 | 2396380 |
| 16 | rs971570 | 88 | rs763024 | 160 | rs1360780 | 232 | 1285007 |
| 17 | rs962899 | 89 | rs763028 | 161 | 2766532 | 233 | 1748235 |
| 18 | rs1045251 | 90 | 2857152 | 162 | 2766557 | 234 | 1284958 |
| 19 | rs261943 | 91 | 241455 | 163 | rs1998894 | 235 | 2763135 |
| 20 | rs1264585 | 92 | 2621426 | 164 | rs879668 | 236 | 2024786 |
| 21 | 984802 | 93 | rs241405 | 165 | rs1049649 | 237 | 1321081 |
| 22 | 1245219 | 94 | 241398 | 166 | 2395639 | 238 | rs1321076 |
| 23 | rs1264562 | 95 | 154973 | 167 | 2250151 | 239 | 1343799 |
| 24 | rs1264513 | 96 | 206779 | 168 | rs651158 | 240 | 1934328 |
| 25 | rs1059510 | 97 | 2856817 | 169 | rs969659 | 241 | 1928533 |
| 26 | rs1362119 | 98 | rs1883414 | 170 | rs743923 | 242 | 713270 |
| 27 | rs1110465 | 99 | rs721393 | 171 | rs851016 | 243 | 1449648 |
| 28 | rs1264432 | 100 | rs1799908 | 172 | rs851007 | 244 | 1449642 |
| 29 | rs1264420 | 101 | rs439205 | 173 | rs743926 | 245 | 1375696 |
| 30 | 1076829 | 102 | rs213194 | 174 | 2859129 | 246 | 756081 |
| 31 | 1075496 | 103 | rs462618 | 175 | 2245972 | 247 | 3088356 |
| 32 | 51457 | 104 | rs213201 | 176 | 1029312 | 248 | 2095771 |
| 33 | 2535323 | 105 | rs462093 | 177 | rs1541316 | 249 | 1555214 |
| 34 | rs1264377 | 106 | rs1014779 | 178 | rs933234 | 250 | 1338471 |
| 35 | rs1264347 | 107 | rs211467 | 179 | rs763021 | 251 | 2179994 |
| 36 | 2535326 | 108 | rs211457 | 180 | 2071794 | 252 | 1555215 |
| 37 | rs1264326 | 109 | 381847 | 181 | rs941816 | 253 | rs1204296 |
| 38 | rs1419693 | 110 | rs1755047 | 182 | rs664370 | 254 | 995564 |
| 39 | rs1264300 | 111 | 769051 | 183 | rs1061632 | 255 | ucla34k_299503 |
| 40 | 2532921 | 112 | rs396516 | 184 | rs605684 | 256 | 2799353 |
| 41 | rs1634713 | 113 | rs210145 | 185 | rs648125 | 257 | 1442224 |
| 42 | 1419664 | 114 | rs494835 | 186 | rs1406945 | 258 | 1899405 |
| 43 | 2517502 | 115 | rs1570760 | 187 | rs720170 | 259 | 2396635 |
| 44 | 2535291 | 116 | rs943470 | 188 | 2395656 | 260 | 2277121 |
| 45 | 2284177 | 117 | rs498114 | 189 | rs236470 | 261 | 1867015 |
| 46 | rs1265111 | 118 | rs652049 | 190 | rs236430 | 262 | 953887 |
| 47 | rs1265181 | 119 | rs943475 | 191 | rs236402 | 263 | 1527707 |
| 48 | rs1639108 | 120 | 942496 | 192 | rs236375 | 264 | 871728 |
| 49 | 1793891 | 121 | 2104362 | 193 | 625474 | 265 | 1881030 |
| 50 | rs1819788 | 122 | rs1853656 | 194 | 449840 | 266 | 1234181 |
| 51 | rs1005248 | 123 | 747889 | 195 | rs8472 | 267 | 699945 |
| 52 | rs1620583 | 124 | 2499740 | 196 | 707542 | 268 | 2206927 |
| 53 | 2596429 | 125 | 2495975 | 197 | 831477 | 269 | 952884 |
| 54 | 2507977 | 126 | 902197 | 198 | 2567280 | 270 | 2216464 |
| 55 | 2516446 | 127 | rs733457 | 199 | 2734977 | 271 | 1421372 |
| 56 | 2523675 | 128 | rs2029461 | 200 | 445117 | 272 | 1862008 |
| 57 | rs1065076 | 129 | 1776888 | 201 | 1565356 | 273 | 1410820 |
| 58 | 2857605 | 130 | 1759627 | 202 | 2252937 | 274 | 1410825 |
| 59 | 239157 | 131 | rs206942 | 203 | 2596464 | 275 | ucla34k_810022 |
| 60 | 2736176 | 132 | rs206930 | 204 | 2442750 | 276 | 220669 |
| 61 | rs805303 | 133 | rs206919 | 205 | 1041523 | 277 | 220711 |
| 62 | rs805282 | 134 | 2395560 | 206 | 1041524 | 278 | 2021916 |
| 63 | rs805293 | 135 | rs205284 | 207 | 693955 | 279 | 1932033 |
| 64 | rs707939 | 136 | 2744971 | 208 | 483536 | 280 | 1226490 |
| 65 | rs743399 | 137 | 2814986 | 209 | ucla34k_654528 | 281 | 2171937 |
| 66 | rs733539 | 138 | rs2064253 | 210 | 2396240 | 282 | 819511 |
| 67 | rs494620 | 139 | 2814951 | 211 | 2221224 | 283 | 937054 |
| 68 | rs644045 | 140 | rs912716 | 212 | 1331293 | 284 | 926774 |
| 69 | ucla34k_328681 | 141 | rs1555773 | 213 | 545455 | 285 | 1986278 |
| 70 | 1265899 | 142 | 2395607 | 214 | 911983 | 286 | 2207224 |
| 71 | rs1150755 | 143 | 2296362 | 215 | 2025230 | 287 | 2281458 |
| 72 | 204999 | 144 | rs1051115 | 216 | 1322651 | 288 | 993612 |

| Région B : | | | | | | | |
|---|---|---|---|---|---|---|---|
| **SNP N°** | **Identifiant** | **SNP N°** | **Identifiant** | **SNP N°** | **Identifiant** | **SNP N°** | **Identifiant** |
| 1 | 2096071 | 44 | 2987903 | 87 | 787469 | 130 | 2989736 |
| 2 | 2282394 | 45 | 2314027 | 88 | rs302919 | 131 | 2989728 |
| 3 | 2805103 | 46 | 1544012 | 89 | 913705 | 132 | 3012797 |
| 4 | 1331336 | 47 | 1997242 | 90 | 932886 | 133 | 1038193 |
| 5 | 1533967 | 48 | 928677 | 91 | 429269 | 134 | 2279265 |
| 6 | 2282179 | 49 | 928678 | 92 | 2526008 | 135 | 964138 |
| 7 | 2011978 | 50 | 2315073 | 93 | 2072058 | 136 | 515078 |
| 8 | 955910 | 51 | 933093 | 94 | rs739441 | 137 | 484397 |
| 9 | 1147360 | 52 | 2315076 | 95 | 2905078 | 138 | 518630 |
| 10 | rs940373 | 53 | 2315078 | 96 | 64967 | 139 | 752835 |
| 11 | 2498905 | 54 | 981759 | 97 | 2905179 | 140 | 1778993 |
| 12 | 2542248 | 55 | 2483469 | 98 | rs649168 | 141 | 1891996 |
| 13 | 1220653 | 56 | 2478858 | 99 | 645841 | 142 | 1106256 |
| 14 | 1867099 | 57 | 2966373 | 100 | rs644234 | 143 | 2382867 |
| 15 | ucla34k_454177 | 58 | 540621 | 101 | 532861 | 144 | 2065385 |
| 16 | 2241271 | 59 | 2994056 | 102 | 59071 | 145 | 872667 |
| 17 | 1017509 | 60 | 2275500 | 103 | 1179040 | 146 | 914400 |
| 18 | rs1182 | 61 | 10K-56700 | 104 | 1887519 | 147 | ucla34k_923462 |
| 19 | rs732074 | 62 | rs943851 | 105 | 1179001 | 148 | 1412512 |
| 20 | rs1125962 | 63 | 2282006 | 106 | ucla34k_576465 | 149 | rs968569 |
| 21 | ucla34k_598296 | 64 | 1887786 | 107 | 954052 | 150 | 210086 |
| 22 | 1322671 | 65 | 2076 | 108 | 2492057 | 151 | 783770 |
| 23 | 1570381 | 66 | 928013 | 109 | 2506715 | 152 | 872006 |
| 24 | rs676492 | 67 | 869381 | 110 | 2506696 | 153 | 1537414 |
| 25 | 2286792 | 68 | 3012757 | 111 | 1079783 | 154 | 574840 |
| 26 | 53558 | 69 | 2987378 | 112 | rs77905 | 155 | 1001523 |
| 27 | 1860641 | 70 | 3012717 | 113 | 129891 | 156 | 755722 |
| 28 | 885345 | 71 | 1331631 | 114 | 2027963 | 157 | 1318383 |
| 29 | rs1043368 | 72 | 1412075 | 115 | 628936 | 158 | 730399 |
| 30 | 1557126 | 73 | 1331625 | 116 | rs602990 | 159 | 1009473 |
| 31 | 947507 | 74 | 2149171 | 117 | 2428091 | 160 | 47713 |
| 32 | 914977 | 75 | ucla34k_694625 | 118 | 2428123 | 161 | 2297690 |
| 33 | 2210623 | 76 | 2296868 | 119 | 2519770 | 162 | 2139881 |
| 34 | 1475731 | 77 | rs1185193 | 120 | 2428083 | 163 | 1335099 |
| 35 | 928518 | 78 | 10K-52978 | 121 | 2789861 | 164 | 55096 |
| 36 | 1864709 | 79 | 563521 | 122 | 414848 | 165 | 2501566 |
| 37 | 944605 | 80 | 507998 | 123 | 1536474 | 166 | 2501559 |
| 38 | 2304812 | 81 | 2362369 | 124 | 943435 | 167 | 2183138 |
| 39 | 1866974 | 82 | 577416 | 125 | 943429 | 168 | 1054864 |
| 40 | 2269337 | 83 | 944812 | 126 | 2182640 | 169 | 2275781 |
| 41 | 2583839 | 84 | rs1470190 | 127 | ucla34k_177347 | 170 | 1891629 |
| 42 | 2791743 | 85 | 2247393 | 128 | 16832 | 171 | 1099298 |
| 43 | 2855181 | 86 | 418620 | 129 | ucla34k_642641 | | |

### 5- Génotypage sur les ADN assemblés.

Pour les 459 SNPs retenus lors de l'étape 4, l'étape suivante a été de déterminer leur allélotype, c'est-à-dire la fréquence de chacun des allèles, et ce pour les 4 groupes d'ADN assemblés (poolés) selon la sévérité et la précocité du phénotype (voir la définition des 4 groupes à l'étape 3 et figure 14).
La fréquence allélique des deux allèles a été déterminée pour chacun des SNP dans les 4 groupes. La signification statistique des écarts de fréquences alléliques entre les groupes AI et BI ou AII et BII est estimée par la valeur « p » représentant la significativité. Plus la valeur p est faible, plus l'écart est statistiquement significatif.
Les expériences ont été reproduites 3 fois (3 PCR), chacune des trois PCR étant ensuite testée 5 fois sur le MALDI-TOF afin d'obtenir une valeur moyenne fiable.

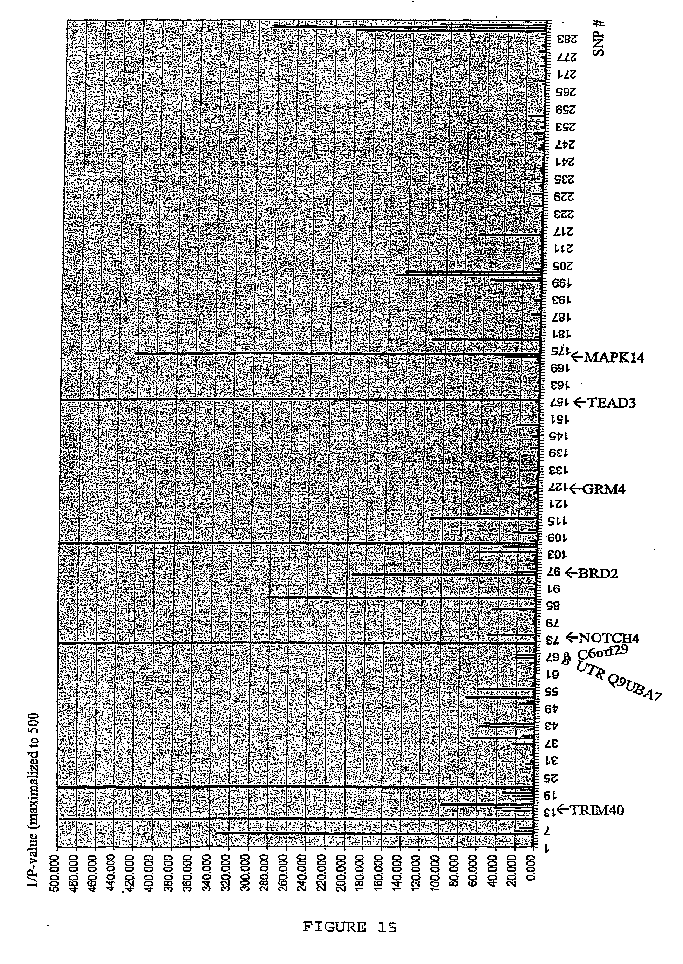

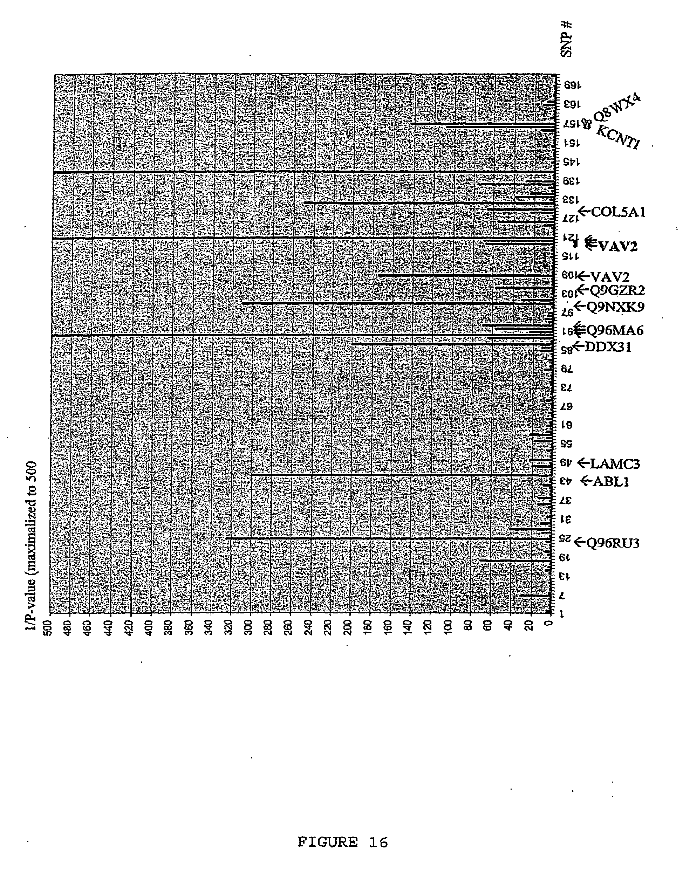

Les figures 15 et 16 illustrent les résultats obtenus sur les régions A et B respectivement pour chaque SNP (numéroté de 1 à 288 le long de la région A et de 1 à 171 le long de la région B). L'ordonnée représente 1/p, mais les valeurs supérieures à 500 (soit p<0,002) sont maximisées à 500.

Le tableau **17** résume les résultats obtenus :

**Tableau 17 : Génotypage sur les pools, nombre de SNPs positifs (total 74) Ces résultats mettent en évidence l'existence de clusters, c'est-à-dire au moins trois SNPs consécutifs (donc physiquement proches les uns des autres dans le génome humain) qui ont tous une significativité p inférieure à 0,05 (appelés « SNPs positifs »). Certains de ces clusters sont illustrés sur les figures 15 et 16.**

| Chromosome 6 | | |
|---|---|---|
| AI-BI<0,05 **ET** AII-BII<0,05 | AI-BI<0,05 | AII-BII<0,05 |
| 11 | 20 | 10 |

| Chromosome 9 | | |
|---|---|---|
| AI-BI<0,05 **ET** AII-BII<0,05 | AI-BI<0,05 | AII-BII<0,05 |
| 2 | 9 | 22 |

Le tableau 18 récapitule les différentes particularités dans la répartition des SNPs dans les régions A et B.

**Tableau 18 : Particularités de la répartition des SNPs positifs dans les régions A e B.**

| Chromosome 6 | |
|---|---|
| Clusters (3 SNPs consécutifs positifs ou plus) | 4 |
| Paires (2 SNPs consécutifs positifs) | 5 |
| 'Double spots' (2 SNPs positifs séparés par un SNP négatif) | 2 |

| Chromosome 9 | |
|---|---|
| Clusters (3 SNPs consécutifs positifs ou plus) | 2 |
| Paires (2 SNPs consécutifs positifs) . | 4 |
| 'Double spots' (2 SNPs positifs séparés par un SNP négatif) | 2 |

Les différents gènes des régions A et B qui sont mis en évidence par des SNPs positifs répartis en clusters, isolés ou répartis en double 'spots' constituent une première série de gènes candidats, y compris les gènes prédits. Leur liste est la suivante:
Région A :
   RNF9, TRIM15, TRIM26, RNF23, FLJ22638, DDR1, HLA-B, HLA-DMB, HLA-DMA, COL11A2, SACM2L, RPS18, B3GALT4, HKE2, RAB2L, TAPBF, ZNF297, DAXX, MAPK14, DOM3Z, MICA, LOC51323, TNFRSF21, MICA, HSPA1B, TNXB, CYP21A2, NOTCH4, PBX2, HLA-DRA, PHF1, ITPR3, MGC14833, BAK1, IHPK3, GRM4, TCP11, TEAD3
Région B :
   DDX31, GTF3C4, C9ORF9, TSC1, ABL1, LOC57109, FREQ, ADAMTS13, LAMC3, SURF5, SURF6, FCN2, FCN1, OLFM1, VAV2, ABO, CELL, SARDH.

Une analyse plus fine a été réalisée qui a permis d'élaborer une nouvelle liste de gènes chevauchés par un SNP positif, grâce à ENSEMBL (*ENSEMBL v.8.30α.1 17 sep 2002*). Cette liste comprend les gènes chevauchés (codants, UnTranslated Région UTR, et intronique) par un SNP positif, à l'exclusion des gènes qui sont proches d'un SNP positif situé dans une région régulatrice.
Codante :
   chromosome 6 : TRIM40, C60RF29, NOTCH4
UTR :
   chromosome 6 : Q9UBA7
Intronique :
   Chromosome 6 : BRD2, GRM4, TEAD3, MAPK14
   Chromosome 9: Q96RU3, ABL1, LAMC3, Q96MA6, Q9NXK9, Q9GZR2, VAV2, COL5A1, KCNT1, Q8WX41

Une nouvelle analyse pour les gènes prédits, grâce à ENSEMBL, a donné les résultats suivants :

| | | | |
|---|---|---|---|
| Chromosome 6 : | ENST00000259854, | ENST00000259855, | ENST00000259941, |
| ENST00000259940, | ENST00000259930, | ENST00000274855, | ENST00000259847, |
| ENST00000293587, | ENST00000299124, | ENST00000259945, | ENST00000259862, |
| ENST00000259876, | ENST00000259875, | ENST00000259895, | ENST00000293682, |
| ENST00000229412, | ENST00000229725, | ENST00000229729, | ENST00000229825, |
| ENST00000244501, | ENST00000293728, | ENST00000244371, | ENST00000293739, |
| ENST00000230240, | ENST00000211372, | ENST00000244475, | ENST00000266008, |
| ENST00000230251, | ENST00000244369, | ENST00000299851, | ENST00000230255, |
| ENST00000229795, | ENST00000229794, | ENST00000296861, | ENST00000274795, |
| ENST00000293645, | ENST00000293707, | ENST00000229780, | ENST00000299791, |
| ENST00000293720, | ENST00000244411, | ENST00000266007, | ENST00000229422. |
| Chromosome 9 : | ENST00000298489, | ENST00000266097, | ENST00000263612, |
| ENST00000245590, | ENST00000298545, | ENST00000298546, | ENST00000298552, |
| ENST00000298554, | ENST00000298555, | ENST00000277434, | ENST00000277433, |
| ENST00000298632, | ENST00000291687, | ENST00000298656, | ENST00000298658, |
| ENST00000298660, | ENST00000277355, | ENST00000298678, | ENST00000298676, |
| ENST00000298656, | ENST00000298658, | ENST00000298660, | ENST00000277355, |
| ENST00000298678, | ENST00000298676, | ENST00000298682, | ENST00000298683, |
| ENST00000291744, | ENST00000291741, | ENST00000223427, | ENST00000198253, |
| ENST00000277527, | ENST00000263604, | ENST00000266109, | ENST00000298467, |
| ENST00000266100, | ENST00000277422, | ENST00000263609. | |

Les tableaux suivants répertorient les gènes prédits des régions A et B dans les clusters, les doubles spots (DS) et les SNP positifs individuels, à partir de la version NCBI Build 28 (Déc. 2001). « CDS » indique la séquence codante et « tx » le transcrit.

| **REGION A** | | | | | | | | |
|---|---|---|---|---|---|---|---|---|
| SNP# 5 à 6 | NOM | chrom | cdsDébut | cdsFin | txDébut | txFin | Brin | Nb EXONS |
| | NOM | chrom | cdsDébut | cdsFin | txDébut | txFin | Brin | Nb EXONS |
| | ENST00000259854 | chr6 | 39694455 | 39909459 | 39694455 | 39909459 | + | 7 |
| | ENST00000259855 | chr6 | 39730113 39909459 | | 39730113 | 39909459 | + | 7 |

| 13 à 21 (cluster grouping) | | | | | | | | |
|---|---|---|---|---|---|---|---|---|
| | NOM | chrom | cdsDébut | cdsFin | txDébut | txFin | Brin | Nb EXONS |
| | ENST00000259941 | chr6 | 40054160 | 40061326 | 40052414 | 40061402 | - | 8 |
| | ENST00000259940 | chr6 | 40054447 | 40061281 | 40053956 | 40061402 | - | 5 |
| | ENST00000259930 | chr6 | 40064144 | 40072806 | 40063675 | 40073156 | + | 7 |
| | ENST00000274855 | chr6 | 40064144 | 40073064 | 40064144 | 40073064 | + | 2 |
| | ENST00000259847 | chr6 | 40086320 | 40099537 | 40085023 | 40105196 | - | 9 |
| | ENST00000293587 | chr6 | 40160063 | 40162772 | 40160063 | 40162772 | + | 6 |
| | ENST00000299124 | chr6 | 40229717 | 40242662 | 40228052 | 40244119 | + | 8 |
| | ENST00000259945 | chr6 | 40229717 | 40242662 | 40228052 | 40244119 | + | 9 |
| | ENST00000259862 | chr6 | 40245577 | 40247213 | 40245564 | 40247263 | + | 5 |

| 18 à 21 DS | | | | | | | | |
|---|---|---|---|---|---|---|---|---|
| | NOM | chrom | cdsDébut | cdsFin | txDébut | txFin | Brin | Nb EXONS |
| | ENST00000299124 | chr6 | 40229717 | 40242662 | 40228052 | 40244119 | + | 8 |
| | ENST00000259945 | chr6 | 40229717 | 40242662 | 40228052 | 40244119 | + | 9 |
| | ENST00000259862 | chr6 | 40245577 | 40247213 | 40245564 | 40247263 | + | 5 |

| 36 à 38 DS | | | | | | | | |
|---|---|---|---|---|---|---|---|---|
| | NOM | chrom | cdsDébut | cdsFin | txDébut | txFin | Brin | Nb EXONS |
| | ENST00000259876 | chr6 | 40789238 | 40799804 | 40784592 | 40800667 | + | 20 |
| | ENST00000259875 | chr6 | 40789238 | 40799804 | 40784592 | 40800667 | + | 19 |
| | ENST00000259895 | chr6 | 40809542 | 40814486 | 40808733 | 40814609 | + | 14 |

| 66 à 67 | | | | | | | | |
|---|---|---|---|---|---|---|---|---|
| | NOM | chrom | cdsDébut | cdsFin | txDébut | txFin | Brin | Nb EXONS |
| | ENST00000293682 | chr6 | 41735883 | 41738214 | 41733465 | 41738312 | + | 4 |
| | ENST00000229412 | chr6 | 41733505 | 41735914 | 41733465 | 41738312 | + | 4 |
| | ENST00000229725 | chr6 | 41758412 | 41761467 | 41757893 | 41761597 | - | 6 |
| | ENST00000229729 | chr6 | 41762320 | 41777667 | 41762320 | 41777667 | - | 21 |

| 95 à 96 | | | | | | | | |
|---|---|---|---|---|---|---|---|---|
| | NOM | chrom | cdsDébut | cdsFin | txDébut | txFin | Brin | Nb EXONS |
| | ENST00000229825 | chr6 | 42776521 | 42781987 | 42775815 | 42782220 | - | 6 |
| | ENST00000244501 | chr6 | 42790440 | 42794203 | 42789785 | 42794258 | - | 5 |
| | ENST00000293728 | chr6 | 42790440 | 42793704 | 42790440 | 42793704 | - | 4 |
| | ENST00000244371 | chr6 | 42814079 | 42821890 | 42809841 | 42822479 | + | 13 |

| 103 à 106 DS | | | | | | | | |
|---|---|---|---|---|---|---|---|---|
| | NOM | chrom | cdsDébut | cdsFin | txDébut | txFin | Brin | Nb EXONS |
| | ENST00000293739 | chr6 | 43091805 | 43112637 | 43091802 | 43112637 | - | 20 |
| | ENST00000230240 | chr6 | 43091805 | 43112637 | 43091802 | 43112637 | - | 18 |
| | ENST00000211372 | chr6 | 43113589 | 43117425 | 43113036 | 43117466 | + | 6 |
| | ENST00000244475 | chr6 | 43118381 | 43119515 | 43118381 | 43119515 | + | 1 |
| | ENST00000266008 | chr6 | 43120240 | 43130132 | 43120066 | 43130176 | - | 15 |
| | ENST00000230251 | chr6 | 43130818 | 43131804 | 43130818 | 43131804 | + | 4 |
| | ENST00000244369 | chr6 | 43133066 | 43139572 | 43132620 | 43139923 | - | 18 |
| | ENST00000299851 | chr6 | 43136980 | 43138945 | 43136980 | 43138945 | - | 6 |
| | ENST00000230255 | chr6 | 43144915 | 43155002 | 43142556 | 43155173 | - | 8 |

| 172 à 173 | | | | | | | | |
|---|---|---|---|---|---|---|---|---|
| | NOM | chrom | cdsDébut | cdsFin | txDébut | txFin | Brin | Nb EXONS |
| | ENST00000229795 | chr6 | 45869083 | 45949370 | 45868721 | 45951684 | + | 12 |
| | ENST00000229794 | chr6 | 45869083 | 45949370 | 45868721 | 45951684 | + | 12 |

| 285 à 286 | | | | | | | | |
|---|---|---|---|---|---|---|---|---|
| | NOM | chrom | cdsDébut | cdsFin | txDébut | txFin | Brin | Nb EXONS |
| | ENST00000296861 | chr6 | 50493533 | 50493905 | 50493248 | 50548202 | - | 5 |
| | ENST00000274795 | chr6 | 50494484 | 50571228 | 50494205 | 50571622 | - | 6 |
| | SNP + individuels | | | | | | | |
| 10 | pas de gènes | | | | | | | |
| 52 | pas de gènes | | | | | | | |
| 55 | | | | | | | | |
| | NOM | chrom | cdsDébut | cdsFin | txDébut | txFin | Brin | Nb EXONS |
| | ENST00000293645 | chr6 | 41309005 | 41405874 | 41309005 | 41405874 | + | 3 |
| 71 | | | | | | | | |
| | NOM | chrom | cdsDébut | cdsFin | txDébut | txFin | Brin | Nb EXONS |
| | ENST00000293707 | chr6 | 41935275 | 41963926 | 41935275 | 41963926 | - | 15 |
| 74 | | | | | | | | |
| | NOM | chrom | cdsDébut | cdsFin | txDébut | txFin | Brin | Nb EXONS |
| | ENST00000229780 | chr6 | 42061138 | 42089652 | 42061115 | 42089727 | - | 31 |
| 83 | pas de gènes | | | | | | | |
| 87 | pas de gènes | | | | | | | |
| 89 | | | | | | | | |
| | NOM | chrom | cdsDébut | cdsFin | txDébut | txFin | Brin | Nb EXONS |
| | ENST00000299791 | chr6 | 42501668 | 42604749 | 42501668 | 42604749 | - | 5 |
| | ENST00000293720 | chr6 | 42501668 | 42658154 | 42501668 | 42658154 | - | 4 |
| 99 | pas de gènes | | | | | | | |
| 110 | pas de gènes | | | | | | | |
| 115 | | | | | | | | |
| | NOM | chrom | cdsDébut | cdsFin | txDébut | txFin | Brin | Nb EXONS |
| | ENST00000244411 | chr6 | 43462531 | 43536706 | 43462495 | 43537491 | + | 58 |
| 126 | | | | | | | | |
| | NOM | chrom | cdsDébut | cdsFin | txDébut | txFin | Brin | Nb EXONS |
| | ENST00000266007 | chr6 | 43863752 | 43974425 | 43862779 | 43974595 | - | 10 |
| 148 | pas de gènes | | | | | | | |
| 157 | | | | | | | | |
| | NOM | chrom | cdsDébut | cdsFin | txDébut | txFin | Brin | Nb EXONS |
| | ENST00000229422 | chr6 | 45315972 | 45327588 | 45314555 | 45337961 | - | 12 |
| 178 | pas de gènes | | | | | | | |
| 199 | pas de gènes | | | | | | | |
| 201 | pas de gènes | | | | | | | |
| 215 | pas de gènes | | | | | | | |
| 226 | pas de gènes | | | | | | | |

| **REGION B** | | | | | | | | |
|---|---|---|---|---|---|---|---|---|
| SNP# | NOM | chrom | cdsDébut | cdsFin | txDébut | txFin Brin | Nb EXONS | |
| 47 à 49 DS | | | | | | | | |
| | NOM | chrom | cdsDébut | cdsFin | txDébut | txFin | Brin | Nb EXONS |
| | ENST00000298489 | chr9 | 125457741 | 125470257 | 125373136 | 125470567 | + | 28 |
| | ENST00000266097 | chr9 | 125373234 | 125470257 | 125373136 | 125470567 | + | 28 |

| 86 à 99 DS chr9:127094511-127505542 | | | | | | | | |
|---|---|---|---|---|---|---|---|---|
| | NOM | chrom | cdsDébut | cdsFin | txDébut | txFin | Brin | Nb EXONS |
| | ENST00000263612 | chr9 | 127045062 | 127120443 | 127044482 | 127120595 | - | 20 |
| | ENST00000245590 | chr9 | 127120792 | 127139136 | 127120534 | 127139622 | + | 5 |
| | ENST00000298545 | chr9 | 127175884 | 127328449 | 127175884 | 127328449 | - | 13 |
| | ENST00000298546 | chr9 | 127334141 | 127338631 | 127328556 | 127340224 | + | 4 |
| | ENST00000298552 | chr9 | 127346431 | 127379066 | 127341543 | 127394815 | - | 23 |
| | ENST00000298554 | chr9 | 127436872 | 127441241 | 127436872 | 127441244 | + | 6 |
| | ENST00000298555 | chr9 | 127469679 | 127471065 | 127469615 | 127471372 | + | 1 |
| | ENST00000277434 | chr9 | 127501047 | 127508171 | 127501047 | 127508171 | + | 8 |
| | ENST00000277433 | chr9 | 127481205 | 127508171 | 127480906 | 127508695 | + | 11 |

| 118 à 120 | | | | | | | | |
|---|---|---|---|---|---|---|---|---|
| | NOM | chrom | cdsDébut | cdsFin | txDébut | txFin Brin | Nb EXONS | |
| | ENST00000298632 | chr9 | 128877580 | 128878579 | 128877580 | 128878579 | - | 1 |
| | ENST00000291687 | chr9 | 128750384 | 128978689 | 128750384 | 128978689 | - | 27 |

| 137 à 138 chr9:130035429-130045373 | | | | | | | | |
|---|---|---|---|---|---|---|---|---|
| | 0 | | | | | | | |
| 128 à 134 DS chr9:129656527-129977399 | | | | | | | | |
| | NOM | chrom | cdsDébut | cdsFin | txDébut | txFin | Brin | Nb EXONS |
| | ENST00000298656 | chr9 | 129757553 | 129770881 | 129757553 | 129770881 | - | 16 |
| | ENST00000298658 | chr9 | 129757607 | 129781523 | 129757607 | 129781523 | - | 13 |
| | ENST00000298660 | chr9 | 129757553 | 129786638 | 129757553 | 129786638 | - | 26 |
| | ENST00000277355 | chr9 | 129607564 | 129789067 | 129607564 | 129789067 | + | 29 |
| | ENST00000298678 | chr9 | 129811215 | 129812613 | 129811213 | 129812613 | + | 2 |
| | ENST00000298676 | chr9 | 129814180 | 129826506 | 129607438 | 129826534 | + | 37 |
| | ENST00000298682 | chr9 | 129864608 | 129868564 | 129864598 | 129870351 | + | 5 |
| | ENST00000298683 | chr9 | 129864608 | 129869270 | 129864598 | 129871307 | + | 7 |
| | ENST00000291744 | chr9 | 129864608 | 129871199 | 129864598 | 129871307 | + | 8 |
| | ENST00000291741 | chr9 | 129864608 | 129871199 | 129864598 | 129871307 | + | 7 |
| | ENST00000223427 | chr9 | 129893584 | 129901655 | 129893369 | 129901747 | - | 9 |
| | ENST00000198253 | chr9 | 129896270 | 129901655 | 129893369 | 129901747 | - | 8 |
| 155 à 156 chr9:130714327-130728681 | | | | | | | | |
| | NOM | chrom | cdsDébut | cdsFin | txDébut | txFin | Brin | Nb EXONS |
| | ENST00000277527 | chr9 | 130609471 | 130715656 | 130609471 | 130715656 | - | 4 |
| | ENST00000263604 | chr9 | 130691065 | 130775279 | 130691064 | 130775281 | + | 29 |
| | SNP + individuels | | | | | | | |
| 6 | pas de gènes | | | | | | | |
| 17 | pas de gènes | | | | | | | |
| 24 | | | | | | | | |
| | NOM | chrom | cdsDébut | cdsFin | txDébut | txFin | Brin | Nb EXONS |
| | ENST00000266109 | chr9 | 124213577 | 124360885 | 124213576 | 124360901 | - | 15 |
| 27 | pas de gènes | | | | | | | |
| 44 | | | | | | | | |
| | NOM | chrom | cdsDébut | cdsFin | txDébut | txFin | Brin | Nb EXONS |
| | ENST00000298467 | chr9 | 125063030 | 125234391 | 125063030 | 125234391 | + | 11 |
| | ENST00000266100 | chr9 | 125184157 | 125234391 | 125183776 | 125236384 | + | 11 |
| 57 | pas de gènes | | | | | | | |
| 100 | pas de gènes | | | | | | | |
| 104 | | | | | | | | |
| | NOM | chrom | cdsDébut | cdsFin | txDébut | txFin | Brin | Nb EXONS |
| | ENST00000277422 | chr9 | 128045772 | 128056657 | 128044878 | 128056857 | - | 8 |
| 108 | pas de gènes | | | | | | | |
| 125 | | | | | | | | |
| | NOM | chrom | cdsDébut | cdsFin | txDébut | txFin | Brin | Nb EXONS |
| | ENST00000263609 | chr9 | 129380168 | 129507477 | 129380168 | 129507477 | + | 9 |
| 141 | pas de gènes | | | | | | | |

### 6- Sélection des SNPs pour le génotypage sur les ADN individuels.

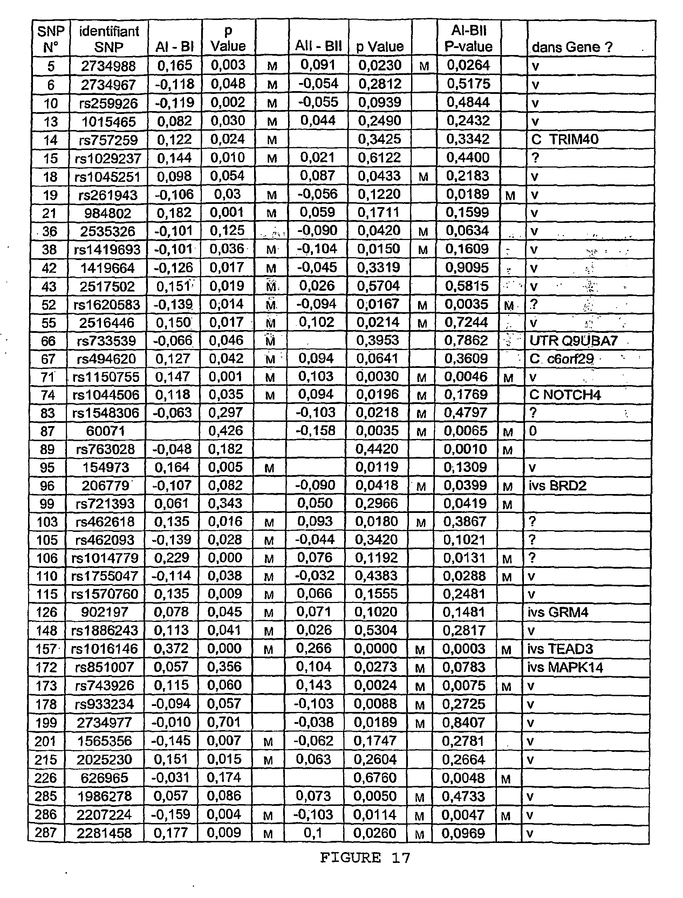

Parmi les 459 SNPs utilisés pour le génotypage sur les ADN assemblés, 76 ont été retenus pour le génotypage des ADN individuels. Les SNPs retenus montrent en effet un écart statistiquement significatif lors du génotypage sur les pools, c'est-à-dire p<0,05 pour AI-BI, AII-BII ou AI-BII. Leur répartition est la suivante :
Région A : 43 SNPs
Région B : 33 SNPs.
La liste des SNPs ainsi choisis et la comparaison A-B sont données dans les figures 17 (région A) et 18 (région B).
Le tableau 19 résume les résultats obtenus.

**Tableau 19 : Choix des SNPs positifs (total 76) suite aux résultats de génotypage sur les pools.**

| Chromosome 6 | | | |
|---|---|---|---|
| AI-BI<0,05 **ET** AII-BII<0,05 | AI-BI<0,05 | AII-BII<0,05 | AI-BII<0,05 |
| 10 | 30 | 20 | 13 |

| Chromosome 9 | | | |
|---|---|---|---|
| AI-BI<0,05 **ET** AII-BII<0,05 | AI-BI<0,05 | AII-BII<0,05 | AI-BII<0,05 |
| 3 | 11 | 25 | 11 |

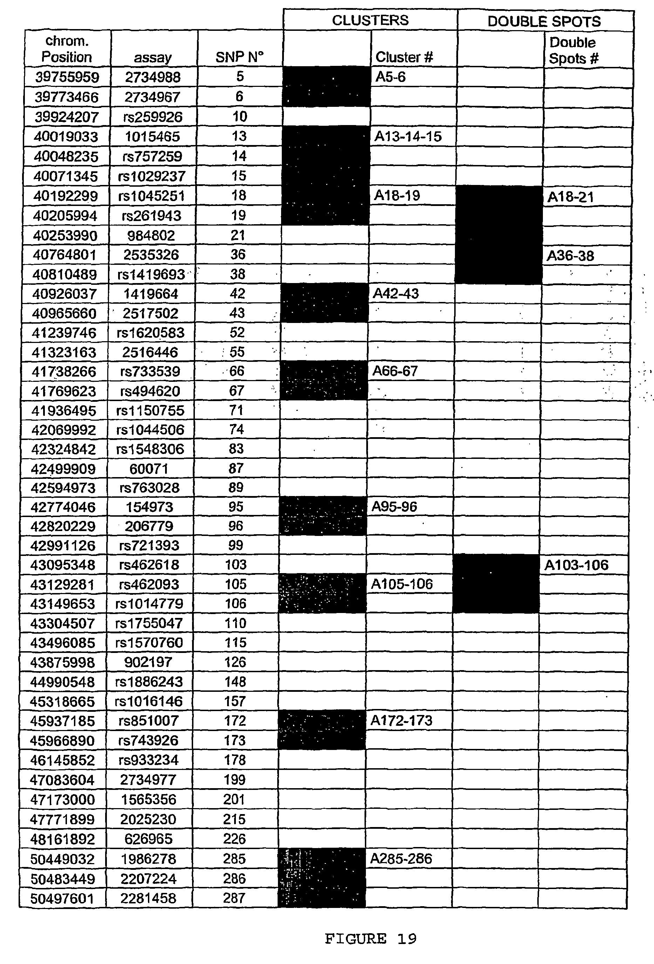

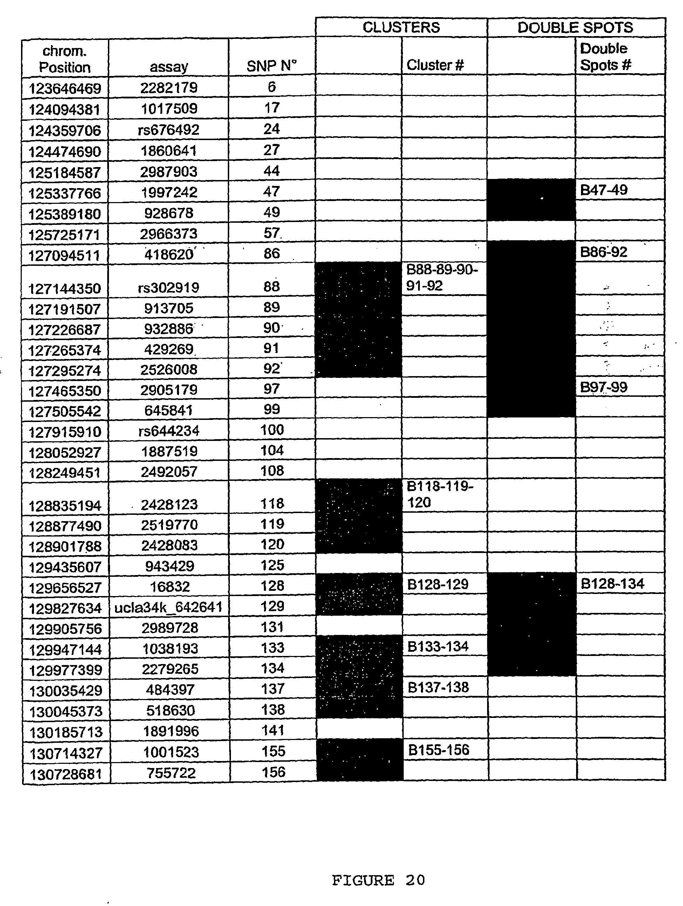

Le choix des 76 SNPs pour le génotypage individuel s'est concentré sur les SNPs présents dans les clusters, ceux formant des paires (2SNPs consécutifs positifs) et ceux formant des 'double spots' (2 SNPs positifs séparés par un SNP négatif). Les figures 19 et 20 illustrent la répartition des 76SNPs choisis.
En effet, il a été observé que l'estimation des fréquences alléliques sur des pools (et non sur des individus) peut conduire à des faux 'positifs' et que cette tendance est majorée lorsque les pools contiennent moins de 200 ADN. De ce fait, les SNPs positifs isolés ont été écartés ainsi que ceux ayant une discordance dans les contrôles (BI et BII).
Les 76 SNPs ont été analysés individuellement sur tous les ADN disponibles (187 individus avec le phénotype CP et 186 individus contrôles sans phénotype CP).

Ce génotypage individuel permet de calculer précisément la fréquence des allèles et des génotypes observés dans les différents groupes. L'ensemble de ces données permet également de comparer la distribution des haplotypes observés au niveau des SNPs positifs organisés en 'clusters'. Par haplotype, on entend la combinaison d'allèles tendant à être transmis ensemble.
L'analyse intégrée de l'ensemble de ces données permet de déterminer les SNPs ou des groupes de SNPs qui montrent une association avec le trait CP, c'est-à-dire un allèle ou un ensemble d'allèles qui, dans une population, sont transmis plus fréquemment avec ce trait.

### 7- Etude du déséquilibre de liaison.

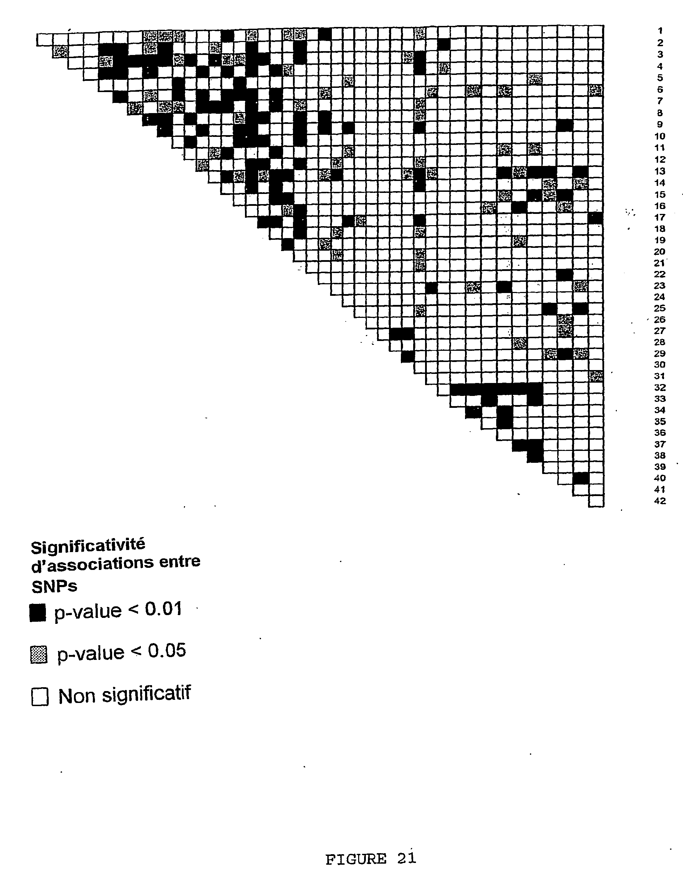

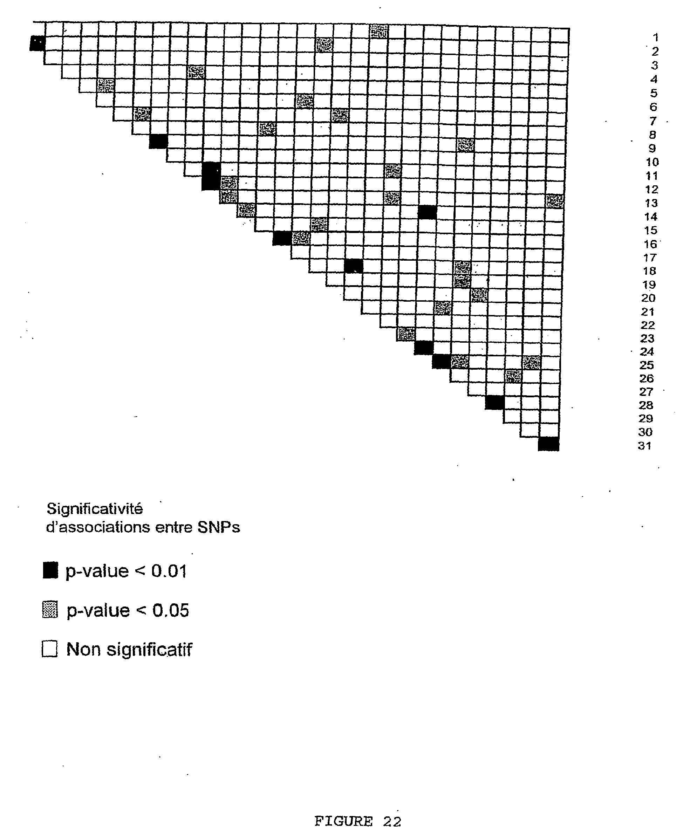

Le déséquilibre de liaison (LD, Linkage Desequilibrium) a été analysé par le programme GenePop, en l'absence de données sur la phase des haplotypes sur les chromosomes analysés.
Le déséquilibre de liaison est une situation où 2 gènes (allèles) ségrègent ensemble à une fréquence plus haute que la fréquence prédite par le produit de leurs fréquences individuelles. Cela signifie que les deux gènes ne sont pas indépendants puisqu'ils ségrègent ensemble plus fréquemment que prévu par les statistiques, il y a donc un déficit d'indépendance entre des allèles situés proche l'un de l'autre sur un même chromosome.
Cette analyse de déséquilibre de liaison a permis de définir des blocs d'ADN qui sont matérialisés par plusieurs marqueurs dont la coségrégation des allèles dévie d'une coségrégation régie par le seul hasard. Cette situation est produite par une absence ou un déficit de recombinaison au sein de ce bloc. La taille des régions présentant un déséquilibre de liaison est variable selon les régions chromosomiques, elle semble s'étendre sur 10kb à 200kb. Les résultats sont présentés dans les figures 21 et 22.

### 8- Comparaison des fréquences alléliques/génotypiques pour chaque SNP.

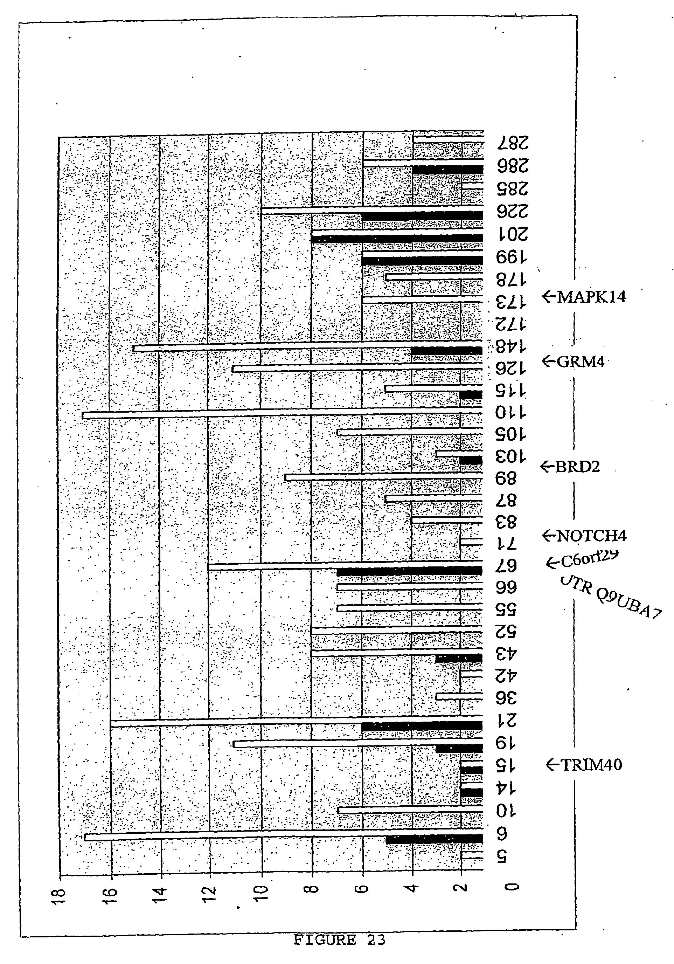

Cette comparaison des fréquences alléliques/génotypiques a été réalisée pour chaque SNP dans les groupes canitie précoce (1 à 5 et p) et dans les groupes de contrôles. Les résultats obtenus sont reproduits dans les tableaux suivants et représentés dans les figures 23 (région A) et 24 (région B).
La colonne « con-con » indique la comparaison entre les différents groupes d'individus contrôles. La colonne « aff » indique les comparaisons pour chaque groupe d'affectés, contre tous les autres groupes d'affectés ou de contrôle.

| **Chromosome 6, région A** | | | | | **Chromosome 9, région B** | | | |
|---|---|---|---|---|---|---|---|---|
| **SNP** | **Con-con** | **aff** | **counts** | | **SNP** | **Con-con** | **aff** | **counts** |
| 5 | 0 | 2 | 2 | | 6 | 0 | 5 | 5 |
| 6 | 5 | 17 | 22 | | 24 | 2 | 0 | 2 |
| 10 | 0 | 7 | 7 | | 27 | 0 | 21 | 21 |
| 14 | 2 | 2 | 4 | | 44 | 0 | 2 | 2 |
| 15 | 2 | 2 | 4 | | 49 | 0 | 4 | 4 |
| 19 | 3 | 11 | 14 | | 57 | 3 | 2 | 5 |
| 21 | 6 | 16 | 22 | | 86 | 2 | 4 | 6 |
| 36 | 0 | 3 | 3 | | 88 | 0 | 7 | 7 |
| 42 | 1 | 2 | 3 | | 90 | 0 | 6 | 6 |
| 43 | 3 | 8 | 11 | | 91 | 0 | 1 | 1 |
| 52 | 1 | 8 | 9 | | 92 | 0 | 5 | 5 |
| 55 | 0 | 7 | 7 | | 97 | 2 | 3 | 5 |
| 66 | 1 | 7 | 8 | | 99 | 0 | 6 | 6 |
| 67 | 7 | 12 | 19 | | 100 | 0 | 4 | 4 |
| 71 | 1 | 2 | 3 | | 104 | 0 | 1 | 1 |
| 83 | 0 | 4 | 4 | | 118 | 0 | 4 | 4 |
| 87 | 0 | 5 | 5 | | 120 | 0 | 6 | 6 |
| 89 | 0 | 9 | 9 | | 125 | 0 | 2 | 2 |
| 103 | 2 | 3 | 5 | | 128 | 0 | 2 | 2 |
| 105 | 0 | 7 | 7 | | 129 | 0 | 3 | 3 |
| 110 | 1 | 17 | 18 | | 131 | 2 | 5 | 7 |
| 115 | 2 | 5 | 7 | | 133 | 4 | 4 | 8 |
| 126 | 0 | 11 | 11 | | 134 | 2 | 6 | 8 |
| 148 | 4 | 15 | 19 | | 137 | 0 | 10 | 10 |
| 172 | 0 | 1 | 1 | | 138 | 0 | 1 | 1 |
| 173 | 0 | 6 | 6 | | 141 | 0 | 3 | 3 |
| 178 | 0 | 5 | 5 | | 155 | 0 | 5 | 5 |
| 199 | 6 | 6 | 12 | | | | | |
| 201 | 8 | 8 | 16 | | | | | |
| 226 | 6 | 10 | 16 | | | | | |
| 285 | 0 | 2 | 2 | | | | | |
| 286 | 4 | 6 | 10 | | | | | |
| 287 | 0 | 4 | 4 | | | | | |

### 9- Conclusions.

Les principales conclusions qui peuvent être tirées des résultats sont les suivantes :
Tout d'abord, il y a une grande similitude entre les observations faites sur l'analyse des pools et les génotypages individuels.
Les grands « clusters » sont confirmés.
Le chromosome 9 révèle un intervalle, en déséquilibre de liaison (cluster majeur) qui est fortement associé au trait canitie précoce (SNP 418620 à SNP 2526008, position 126'544'533 nt à position 126'745'296 nt, soit une taille de 201 kb). Ce cluster comprend les gènes DDX31, GTF3C4 et Q96MA6.
Les gènes ou gènes prédits identifiés dans les intervalles associés à un haplotype positif ou un cluster de SNPs positifs sont les suivants :
Région A
   **Haplotype 5-6**
      HLAG : Début (position sur chrom.): 39730109 Fin (position sur chrom.): 39733287
      NT_007592.445: Début (position sur chrom.): 39750711 Fin (position sur chrom.): 39778231
      NT_007592.446 :Début (position sur chrom.): 39787841 Fin (position sur chrom.): 39809968
   **Haplotypes 42-43**
      NT_007592.506: Début (position sur chrom.): 40890755 Fin (position sur chrom.): 40945799
      NT_007592.507): Début (position sur chrom.): 40954831 Fin (position sur chrom.): 40968983
      NT_007592.508): Début (position sur chrom.): 40977433 Fin (position sur chrom.): 41013059
   **Haplotypes 66-67**
      HSPA1B : Début (position sur chrom.): 41726349 Fin (position sur chrom.): 41728808
      G8 :Début (position sur chrom.): 41733475 Fin (position sur chrom.): 41738312
      NEU1 neuraminidase precursor : Début (position sur chrom.): 41757894 Fin (position sur chrom.): 41761597
      NG22 : Début (position sur chrom.): 41761889 Fin (position sur chrom.): 41777684
      BAT8 ankyrin repeat-containing protein ; Mouse Ortholog: Bat8 : Début (position sur chrom.): 41778446 Fin (position sur chrom.): 41791599
   **Haplotypes 95-96**
      HLA-DMB : Début (position sur chrom.): 27217082 Fin (position sur chrom.): 27223420
      HLA-DMA : Début (position sur chrom.): 27231061 Fin (position sur chrom.): 27235519
      BRD2 : Début (position sur chrom.): 27251103 Fin (position sur chrom.): 27263747
   **Haplotypes 89 (87)**
      HLA-DQA1 : Début (position sur chrom.): 42482839 Fin (position sur chrom.): 42489050
      HLA-DQA2 : Début (position sur chrom.): 42582711 Fin (position sur chrom.): 42587664
      NT_007592.588 : Début (position sur chrom.): 42498795 Fin (position sur chrom.): 42505711
   **Haplotypes 126**
      GRM4 glutamate receptor, metabotropic 4 : Début (position sur chrom.): 43862780 Fin (position sur chrom.): 43974595
   **Haplotypes 21 (18-21)**
      RNF23 : Début (position sur chrom.): 40229287 Fin (position sur chrom.): 40243110 hypothetical protein FLJ22638 :Début (position sur chrom.): 40245566 Fin (position sur chrom.): 40247261
      or (NT_007592.459) : Début (position sur chrom.): 40245578 Fin (position sur chrom.): 40369534
      NT_007592.457): Début (position sur chrom.): 40160064 Fin (position sur chrom.): 40218336
Region B
   **Haplotype 27**
      FREQ: PubMed on Product: frequenin homolog/ Mouse Ortholog: Freq
      Début (position sur chrom.): 124490317 Fin (position sur chrom.): 124554366 NT_030046.18 :Début (position sur chrom.): 124458070 Fin (position sur chrom.): 124489558
      NT_030046.17 : Début (position sur chrom.): 124371672 Fin (position sur chrom.): 124452860
   **Haplotype 97-100**
      GTF3C5: PubMed on Product: general transcription factor IIIC, polypeptide 5
      Début (position sur chrom.): 127480920 Fin (position sur chrom.): 127508694
      CEL : PubMed on Product: carboxyl ester lipase (bile salt-stimulated)
      Début (position sur chrom.): 127512178 Fin (position sur chrom.): 127522054
      CELL: PubMed on Product: carboxyl ester lipase-like (bile salt-stimulated)
      Début (position sur chrom.): 127532733 Fin (position sur chrom.): 127537549
      FS: PubMed on Product: Forssman synthetase
      Début (position sur chrom.): 127603661 Fin (position sur chrom.): 127614093
      ABO blood group (transferase A, alpha): Début (position sur chrom.): 127907180 Fin (position sur chrom.): 127924298
   **Haplotype 86-92**
      BARHL1
      DDX31
      GTF3C4
      Q96MA6 (Adenylate cyclase)

### Nouvelle analyse de la région correspondant à l'haplotype 86-92, avec de nouveaux SNPs 'Single Nucleotide Polymorphism'

Cette région du chromosome 9 a fait l'objet d'une nouvelle analyse avec une batterie de nouveaux SNPs permettant de courvrir la région de manière plus dense (1 SNP tous les 2 à 3kb sur cette région de 120 kb).
Le génotypage individuel effectué avec ces SNPs permet de mettre en évidence 2 gènes très fortement positifs sur les 4 : DDX31 et GTF3C4.

### EXEMPLE 3 : Analyse des régions d'intérêt sur les chromosomes 3, 5 et 11 avec des SNP 'Single Nucleotide Polymorphism'

Les mêmes expériences d'allélotypage sur pools ont été réalisées sur les régions d'intérêt des chromosomes 3, 5 et 11 telles que définies dans l'exemple 1, en choisissant les SNPs dans les régions géniques ou voisines.
Cette analyse a permis de mettre en évidence les gènes suivants sur cette région :
Les coordonnées sont données en fonction du build 30 (juin 2002)

**Chromosome 3 (région C) :**
   C1 (41527287-41677819):
      - Protéine hypothétique KIAA1042
      - CCK: gastrin/cholecystokinin type b receptor (cck-b receptor)
   C2: (52846896-52913941) :
      - CACNA1D: voltage-dependent 1-type calcium channel alpha-1d subunit C3 : (55638663-56042041) :

      - ARHGEF3 rho guanine nucleotide exchange factor 3; rhogef protein; 59.8 kda protein; exchange factor found in platelets and leukemic and neuronal tissues, xpln.
      - Protéine hypothétique AL133097
**Chromosome 5 (région D) :**
   D1 (136479801-137007868):
      - KLHL3 : kelch-like protein 3
      - HNRPA0 : heterogeneous nuclear ribonucleoprotein a0 (hnrnp a0).
   **D2 (137542040-137771805)**
      - CDC25C: map/microtubule affinity-regulating kinase 3 (ec 2.7.1.27)
      - EGR1 : early growth response protein 1 (egr-1) (krox-24 protein)
      - C5orf6 : prédit
      - C5orf7 : prédit
      - LOC51308 : prédit
      - ETF1 : eukaryotic peptide chain release factor subunit 1 (erfl)
      - HSPA9B : stress-70 protein, mitochondrial precursor
   **D3 (139931847-140118601)**
      - PCDHA1 à PCDHA13 :protocadherin alpha 1 precursor à protocadherin alpha 1 precursor
   D4 (149518721-149586774)
      - GSF1R : Macrophage Colony Stimulating Factor I Receptor Precursor
      - RPL7: 60s ribosomal protein 17
      - PDGFRB : beta platelet-derived growth factor receptor precursor (ec 2.7.1.112)
   **D5 (149793126-149995886)**
      - TCOF1 : Treacle Protein (Treacher Collins Syndrome Protein).
      - AL133039 : prédit
      - CD74 : h1a class ii histocompatibility antigen, gamma chain
      - RPS14 : 40s ribosomal protein s14
      - NDST1 : Heparan Sulfate N-Deacetylase/N-Sulfotransferase (Ec 2.8.2.8)
   **D6 (151235618-151373121)**
      - G3BP : ras-gtpase-activating protein binding protein 2
      - GLRA1 : glycine receptor alpha-1 chain precursor
   **D7 (153463449-153854650)**
      - C5orf3: prédit
      - MFAP3 : microfibril-associated glycoprotein 3 precursor
      - GALNT10 : putative udp-galnac:polypeptide n-acetylgalactosaminyltransferase
      - FLJ11715 : prédit
**Chromosome 11 (région E) :**
   E1 (108893187-108944206)
      - GUCY1A2 : guanylate cyclase soluble, alpha-2 chain (ec 4.6.1.2)
   **E2 (110056711-110546142)**
      - CUL5 : vasopressin-activated calcium-mobilizing receptor (vacm-1) (cullin homolog 5)
      - ACAT1 : acetyl-coa acetyltransferase, mitochondrial precursor (ec 2.3.1.9)
      - NPAT :nuclear protein, ataxia-telangiectasia locus; e14 gene;
      - ATM : serine-protein kinase atm (ec 2.7.1.37) (ataxia telangiectasia mutated
      - AF035326 : prédit
      - AF035327 : prédit
      - AF035328 : prédit
      - BC029536 :prédit
   E3 (115527211-115745012)
      - FLJ20535
      - DRD2 : d(2) dopamine receptor
      - ENS303941 : prédit
   E4 (117397672-117752160) :
      IGSF4 : immunoglobulin superfamily, member 4; nectin-like protein 2
   **E5 (118532530-118685957)**
      Pas de gène connu
   **E6 (119417270-119469358)**
      - LOC51092: prédit
      - BC010946 : prédit
      - TAGLN : transgelin (smooth muscle protein 22-alpha) (sm22-alpha) (ws3-10) (22 kda actin-binding protein).
      - PCSK7 : proprotein convertase subtilisin/kexin type 7 precursor (ec 3.4.21.-)
      - ENS300650 : prédit

### EXEMPLE 4

### Exemples de compositions

- lotion capillaire

| | |
|---|---|
| fragment d'ADN issu de l'un des gènes divulgués appartenant à la zone chromosomique comprise entre les marqueurs D6S1629 et D6S257 | 0,5 g |
| Propylène glycol | 20 g |
| Ethanol 95° | 30 g |
| Eau qsp | 100 g |

Cette lotion est appliquée quotidiennement sur les zones à traiter et de préférence sur l'ensemble du cuir chevelu pendant au moins 10 jours et préférentiellement 1 à 2 mois.
On constate alors une diminution de l'apparition des cheveux blancs ou gris et une repigmentation des cheveux gris.
- shampooing traitant

| | |
|---|---|
| fragment d'ADN issu de l'un des gènes divulgués appartenant à la zone chromosomique comprise entre le marqueur D9S290 et le télomère du bras long | 1,5 g |
| Polyglycéryl 3-hydroxylarylether | 26 g |
| Hydroxy propyl cellulose vendue sous la dénomination de Klucell G par la société Hercules | 2 g |
| Conservateurs | qps |
| Ethanol 95° | 50 g |
| Eau qsp | 100 g |

Ce shampooing est utilisé à chaque lavage avec un temps de pose d'environ d'une minute. Un usage prolongé, de l'ordre de deux mois, conduit à la repigmentation progressive des cheveux gris.
Ce shampooing peut également être utilisé à titre préventif afin de retardé le blanchissement des cheveux.
- Gel traitant

| | |
|---|---|
| fragment d'ADN issu de l'un des gènes divulgués appartenant à la zone chromosomique comprise entre les marqueurs D6S1629 et D6S257 | 0,75 g |
| Huiles essentielles d'Eucalyptus | 1 g |
| Econozole | 0,2 g |
| Lauryl polyglyceryl 6 cetearyl glycoether | 1,9 g |
| Conservateurs | qs |
| Carbopol 934P vendu par la société BF Goodrich Corporation | 0,3 g |
| Agent de neutralisation | qs pH 7 |
| Eau qsp | 100 g |

Ce gel est appliqué sur les zones à traiter deux fois par jour (matin et soir) avec un massage terminal. Après trois mois d'application, on observe une repigmentation des poils ou cheveux de la zone traitée.

### Références bibliographiques

• E. Lander and L. Kruglyak. Genetic dissection of complex traits: guidelines for interpreting and reporting linkage results. Nat.Genet. 11 (3):241-247, 1995.

## Revendications

1. Procédé de criblage de molécules capables de moduler la fonction d'un fragment polynucléotidique, ledit fragment comprenant au moins 18 nucléotides consécutifs dont la séquence correspond à tout ou partie d'un gène du chromosome 9 humain choisi parmi les gènes DDX31 et GTF3C4, comprenant les étapes de
- mise en présence de la molécule à tester et du fragment polynucléotidique; et
- détection de la variation d'un paramètre lié à la fonction dudit fragment,
permettant l'identification d'un agent pour une utilisation à des fins cosmétiques ou thérapeutiques, dans le domaine de la pigmentation.

2. Procédé de criblage selon la revendication 1, où l'étape de détection consiste en la détection d'une fixation de ladite molécule sur le fragment polynucléotidique.

3. Procédé de criblage selon la revendication 2, où ladite détection d'une fixation est mise en évidence par une technique de détection de ligands.

4. Procédé de criblage selon l'une des revendications 1 à 3, permettant d'identifier un inhibiteur de la fonction du gène DDX31 ou du gène GTF3C4.

5. Procédé de criblage selon la revendication 1, permettant d'identifier un agent favorisant ou inhibant la transcription du gène DDX31 ou du gène GTF3C4.

6. Procédé de criblage de molécules capables de moduler la fonction du produit d'expression d'un fragment polynucléotidique, ledit fragment comprenant au moins 18 nucléotides consécutifs dont la séquence correspond à tout ou partie d'un gène du chromosome 9 humain choisi parmi les gènes DDX31 et GTF3C4, comprenant les étapes de
- mise en présence de la molécule à tester et du produit d'expression; et
- détection de la variation d'un paramètre lié à la fonction dudit produit d'expression,
permettant l'identification d'un agent pour une utilisation à des fins cosmétiques ou thérapeutiques, dans le domaine de la pigmentation.

7. Procédé de criblage selon la revendication 6, où l'étape de détection consiste en la détection d'une fixation de ladite molécule sur le produit d'expression.

8. Procédé de criblage selon la revendication 7, où ladite détection d'une fixation est mise en évidence par une technique de détection de ligands.

9. Procédé de criblage selon l'une des revendications 6 à 8, permettant d'identifier un inhibiteur des fonctions d'un produit d'expression du gène DDX31 ou du gène GTF3C4.

10. Procédé de criblage selon l'une des revendications 6 à 9 où ledit produit d'expression est un ARN issu de la transcription du gène DDX31 ou du gène GTF3C4.

11. Procédé de criblage selon l'une des revendications 6 à 9 où ledit produit d'expression est un polypeptide issu de la traduction du gène DDX31 ou du gène GTF3C4.

12. Procédé de criblage selon l'une des revendications 6 à 11 où ladite molécule est une molécule d'ARN antisens.

13. Procédé de criblage selon l'une des revendications 6 à 11 où ladite molécule est un anticorps.

14. Utilisation d'au moins un des marqueurs choisis parmi les SNP 418620, rs302919, 913705, 932886, 429269 et 2526008 pour la détermination des gènes impliqués dans la pigmentation de la peau ou des phanères.

## Claims

1. A method for screening molecules that can modulate the function of a polynucleotide fragment, said fragment comprising at least 18 consecutive nucleotides the sequence of which corresponds to all or part of a gene on human chromosome 9, said gene being chosen amongst the DDX31 gene and the GTF3C4 gene, comprising the steps of:
- bringing the molecule to be tested into the presence of the polynucleotide fragment; and
- detecting a variation in a parameter linked to the function of said fragment,
for the identification of an agent for cosmetic or therapeutic purposes, in the field of pigmentation.

2. The method of claim 1, wherein the detection step is the detection of any binding of said molecule to the polynucleotide fragment.

3. The method of claim 2, wherein said detection of any binding is demonstrated by a ligand binding detection method.

4. The method according to any one of claims 1 to 3 for the detection of an inhibitor for the function of the DDX31 gene or the GTF3C4 gene.

5. The method according to claim 1, for the detection of an agent promoting or inhibiting the transcription of the DDX31 gene or the GTF3C4 gene.

6. A method for screening molecules that can modulate the function of the expression product of a polynucleotide fragment, said fragment comprising at least 18 consecutive nucleotides the sequence of which corresponds to all or part of a gene on human chromosome 9, said gene being chosen amongst the DDX31 gene and the GTF3C4 gene, comprising the steps of:
- bringing the molecule to be tested into the presence of the expression product; and
- detecting a variation in a parameter linked to the function of said expression product,
for cosmetic or therapeutic purposes, in the field of pigmentation.

7. The method of claim 6, wherein the detection step is the detection of any binding of said molecule to the expression product.

8. The method of claim 7, wherein said detection of any binding is demonstrated by a ligand binding detection method.

9. The method according to any one of claims 6 to 9 for the detection of an inhibitor for the functions of an expression product of the DDX31 gene or the GTF3C4 gene.

10. The method according to any one of claims 6 to 9, wherein said expression product is an RNA molecule derived from transcription of the DDX31 gene or of the GTF3C4 gene.

11. The method according to any one of claims 6 to 9, wherein said expression product is a polypeptide derived from translation of the DDX31 gene or of the GTF3C4 gene.

12. The method according to any one of claims 6 to 11, wherein said molecule is an antisense RNA molecule.

13. The method according to any one of claims 6 to 11, wherein said molecule is an antibody.

14. Use of at least one marker selected from SNP 418620, rs302919, 913705, 932886, 429269 and 2526008, for the determination of genes involved in the pigmentation of the skin or phanera.

## Patentansprüche

1. Verfahren zum Screenen von Molekülen, die in der Lage sind, die Funktion eines Polynukleotidfragments zu modulieren, wobei dieses Fragment mindestens 18 aufeinanderfolgende Nukleotide umfasst, deren Sequenz ganz oder teilweise einem Gen des menschlichen Chromosoms 9 entspricht, das ausgewählt ist aus den Genen DDX31 und GTF3C4, umfassend die Schritte
- Zusammengeben des zu testenden Moleküls und des Polynukleotidfragments;
- Nachweis der Veränderung eines Parameters, der mit der Funktion dieses Fragments zusammenhängt,
wodurch ermöglicht wird, einen Wirkstoff für eine Verwendung zu kosmetischen oder therapeutischen Zwecken auf dem Gebiet der Pigmentierung zu identifizieren.

2. Screenverfahren gemäß Anspruch 1, in dem der Nachweisschritt aus dem Nachweis einer Anlagerung dieses Moleküls an das Polynukleotidfragment besteht.

3. Screenverfahren gemäß Anspruch 2, in dem dieser Nachweis einer Anlagerung durch eine Methode zum Nachweis von Liganden festgestellt wird.

4. Screenverfahren gemäß einem der Ansprüche 1 bis 3, das ermöglicht, einen Inhibitor der Funktion des Gens DDX31 oder des Gens GTF3C4 zu identifizieren.

5. Screenverfahren gemäß Anspruch 1, das ermöglicht, einen Wirkstoff zu identifizieren, der die Transkription des Gens DDX31 oder des Gens GTF3C4 fördert oder inhibiert.

6. Verfahren zum Screenen von Molekülen, die in der Lage sind, die Funktion des exprimierten Produkts eines Polynukleotidfragments zu modulieren, wobei dieses Fragment mindestens 18 aufeinanderfolgende Nukleotide umfasst, deren Sequenz ganz oder teilweise einem Gen des menschlichen Chromosoms 9 entspricht, das ausgewählt ist aus den Genen DDX31 und GTF3C4, umfassend die Schritte
- Zusammengeben des zu testenden Moleküls und des exprimierten Produkts; und
- Nachweis der Veränderung eines Parameters, der mit der Funktion dieses exprimierten Produkts zusammenhängt,
wodurch ermöglicht wird, einen Wirkstoff für eine Verwendung zu kosmetischen oder therapeutischen Zwecken auf dem Gebiet der Pigmentierung zu identifizieren.

7. Screenverfahren gemäß Anspruch 6, in dem der Nachweisschritt aus dem Nachweis einer Anlagerung dieses Moleküls an das exprimierte Produkt besteht.

8. Screenverfahren gemäß Anspruch 7, in dem dieser Nachweis einer Anlagerung durch eine Methode zum Nachweis von Liganden festgestellt wird.

9. Screenverfahren gemäß einem der Ansprüche 6 bis 8, das ermöglicht, einen Inhibitor der Funktionen eines exprimierten Produkts des Gens DDX31 oder des Gens GTF3C4 zu identifizieren.

10. Screenverfahren gemäß einem der Ansprüche 6 bis 9, in dem dieses exprimierte Produkt eine RNA ist, die aus der Transkription des Gens DDX31 oder des Gens GTF3C4 hervorgegangen ist.

11. Screenverfahren gemäß einem der Ansprüche 6 bis 9, in dem dieses exprimierte Produkt ein Polypeptid ist, das aus der Translation des Gens DDX31 oder des Gens GTF3C4 hervorgegangen ist.

12. Screenverfahren gemäß einem der Ansprüche 6 bis 11, in dem dieses Molekül ein Antisense-RNA-Molekül ist.

13. Screenverfahren gemäß einem der Ansprüche 6 bis 11, in dem dieses Molekül ein Antikörper ist.

14. Verwendung von mindestens einem der Marker, die ausgewählt sind aus den SNP 418620, rs302919, 913705, 932886, 429269 und 2526008, zur Bestimmung von Genen, die an der Pigmentierung der Haut oder von exodermalen Auswüchsen beteiligt sind.
